(19) Europäisches Patentamt
European Patent Office
Office européen des brevets

(11) EP 1 587 810 B1

(12) **FASCICULE DE BREVET EUROPEEN**

(45) Date de publication et mention de la délivrance du brevet:
**08.11.2006 Bulletin 2006/45**

(21) Numéro de dépôt: **03809363.9**

(22) Date de dépôt: **17.10.2003**

(51) Int Cl.:
***C07D 498/14*** (2006.01) ***C07D 498/22*** (2006.01)
***C07D 491/14*** (2006.01) ***C07D 487/04*** (2006.01)
***A61P 35/00*** (2006.01) ***A61K 31/5365*** (2006.01)

(86) Numéro de dépôt international:
**PCT/FR2003/003069**

(87) Numéro de publication internationale:
**WO 2004/037831 (06.05.2004 Gazette 2004/19)**

(54) **NOUVEAUX DERIVES DE 1,4 BENZODIOXINO 2,3-e ISOINDOLE SUBSTITUES, LEUR PROCEDE DE PREPARATION ET LES COMPOSITIONS PHARMACEUTIQUES QUI LES CONTIENNENT**

SUBSTITUIERTE ¬1,4|BENZODIOXIN¬2,3-E|ISOINDOL-DERIVATEN, IHR VERFAHREN ZUR HERSTELLUNG UND IHRE PHARMAZEUTISCHE ZUSAMMENSETZUNGEN

NOVEL SUBSTITUTED 1,4|BENZODIOXINO 2,3-E|ISOINDOLE DERIVATIVES, METHOD FOR PREPARING SAME AND PHARMACEUTICAL COMPOSITIONS CONTAINING SAME

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **18.10.2002 FR 0212965**

(43) Date de publication de la demande:
**26.10.2005 Bulletin 2005/43**

(73) Titulaire: **Les Laboratoires Servier
92415 Courbevoie Cédex (FR)**

(72) Inventeurs:
- **COUDERT, Gérard
F-45560 Saint Denis en Val (FR)**
- **AYERBE, Nathalie
F-34250 Palavas Les Flots (FR)**
- **LEPIFRE, Franck
F-45160 Olivet (FR)**
- **ROUTIER, Sylvain
F-45510 Tigy (FR)**
- **CAIGNARD, Daniel-Henri
F-78230 Lepecq (FR)**
- **RENARD, Pierre
F-78150 Le Chesnay (FR)**
- **HICKMAN, John
F-75017 Paris (FR)**
- **PIERRE, Alain
F-78580 Les Alluets Le Roi (FR)**
- **LEONCE, Stéphane
F-78000 Versailles (FR)**

(56) Documents cités:
**EP-A- 0 841 337**
**WO-A-98/09967**
**US-A- 5 705 511**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 587 810 B1

## Description

La présente invention concerne les nouveaux dérivés [1,4]benzodioxino[2,3-e]isoindole substitués, leur procédé de préparation et les compositions pharmaceutiques qui les contiennent. Les composés de la présente invention trouvent une utilisation intéressante grâce à leur activité antitumorale.

La demande de brevet WO 00/18407 décrit des dérivés de pyrrolocarbazole utiles dans la prévention et le traitement de la surdité et de la sensation de perte d'équilibre. Les demandes des brevets US 5.705.511 et WO 96/11933 présentent des dérivés de cyclopenta[g]pyrrolo[3,4-e]indole fusionnés par la partie indole et la partie cyclopentène des dérivés, à un système cyclique aromatique ou non aromatique, et comportant éventuellement des hétéroatomes. Ces composés possèdent des activités pharmacologiques les rendant notamment utiles dans le traitement des cellules cancéreuses. La demande de brevet WO 01/85686 décrit des dérivés aryles de pyrrolocarbazole utiles dans le traitement des cancers. La demande de brevet EP 0841337 revendique des dérivés 7,12-dioxobenzo[a] anthracéniques substitués et décrit leurs propriétés anticancéreuses.

Les composés de la présente invention trouvent leur originalité à la fois dans leur structure et dans leur utilisation en tant qu'agents antitumoraux.

Plus spécifiquement, la présente invention concerne les composés de formule (I) :

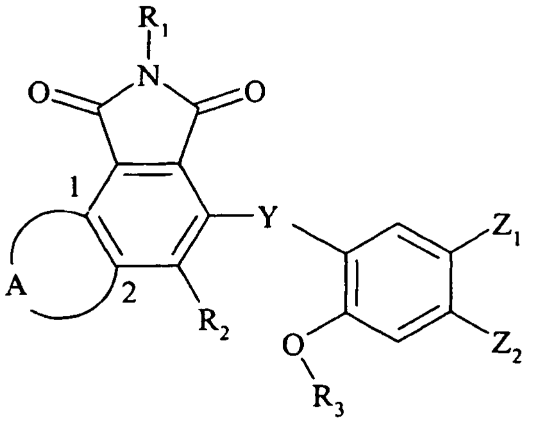

(I)

dans laquelle :

- A représente avec les atomes de carbone auxquel il est lié, un groupement de formule (a) ou (b) :

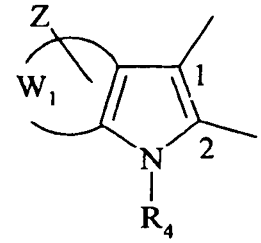

(a)

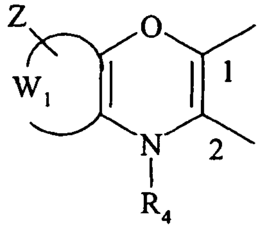

(b)

dans lesquels :

✧ $W_1$ représente avec les atomes de carbone auxquels il est lié, un groupement phényle ou un groupement pyridinyle,

Z représente un groupement choisi parmi atome d'hydrogène, halogène, groupement alkyle ($C_1$-$C_6$) linéaire ou ramifié, nitro, cyano, hydroxy, alkoxy ($C_1$-$C_6$) linéaire ou ramifié, aryle, arylalkyle ($C_1$-$C_6$) linéaire ou ramifié, aryloxy, arylalkoxy ($C_1$-$C_6$) linéaire ou ramifié, $NR_5R_6$, dans lequel $R_5$ et $R_6$, identiques ou différents, indépendamment l'un de l'autre, représentent chacun un groupement choisi parmi atome d'hydrogène, groupement alkyle ($C_1$-$C_6$) linéaire ou ramifié,

✧ $R_4$ représente un groupement choisi parmi atome d'hydrogène, groupement alkyle ($C_1$-$C_6$) linéaire ou ramifié, aryle, arylalkyle ($C_1$-$C_6$) linéaire ou ramifié, ou un groupement -C(O)-OR'$_5$, dans lequel R'$_5$ représente un groupement choisi parmi groupement alkyle ($C_1$-$C_6$) linéaire ou ramifié, aryle, ou aryalkyle ($C_1$-$C_6$) linéaire ou ramifié,

- Y représente un groupement choisi parmi atome d'oxygène ou groupement méthylène,
- $R_2$ représente un atome d'hydrogène, et dans ce cas :

  $R_3$ représente un groupement choisi parmi atome d'hydrogène, groupement alkyle ($C_1$-$C_6$) linéaire ou ramifiée, aryle, arylalkyle ($C_1$-$C_6$) linéaire ou ramifié, ou $SO_2CF_3$,

- ou bien $R_2$ et $R_3$ forment une liaison,
- $R_1$ représente un groupement choisi parmi atome d'hydrogène, groupement alkyle ($C_1$-$C_6$) linéaire ou ramifié, aryle, arylalkyle ($C_1$-$C_6$) linéaire ou ramifié, ou une chaîne alkylène ($C_1$-$C_6$) linéaire ou ramifiée, substituée par un ou plusieurs groupements, identiques ou différents, choisis parmi -OR"$_5$, -NR"$_5$R"$_6$, dans lesquels R"$_5$ et R"$_6$ ont les mêmes définitions que $R_5$ et $R_6$ tels que définis précédemment,
- $\mathbf{Z_1}$ et $\mathbf{Z_2}$ représentent chacun un atome d'hydrogène ou,
  $\mathbf{Z_1}$ et $\mathbf{Z_2}$ forment ensemble, avec les atomes de carbone qui les portent, un groupement phényle,

étant entendu que, lorsque Z représente un atome d'hydrogène alors $R_1$ est différent de atome d'hydrogène,
leurs énantiomères, diastéréoisomères, N-oxyde, ainsi que leurs sels d'addition à un acide ou à une base pharmaceutiquement acceptable,
étant entendu que par aryle, on comprend un groupement phényle, naphtyle, dihydronaphtyle, tétrahydronaphtyle, indényle ou indanyle, chacun de ces groupements étant éventuellement substitué par un ou plusieurs groupements, identiques ou différents, choisis parmi halogène, alkyle ($C_1$-$C_6$) linéaire ou ramifié, triahalogénoalkyle ($C_1$-$C_6$) linéaire ou ramifié, hydroxy, alkoxy ($C_1$-$C_6$) linéaire ou ramifié, et amino éventuellement substitué par un ou deux groupements alkyle ($C_1$-$C_6$) linéaire ou ramifié.

Parmi les acides pharmaceutiquement acceptables, on peut citer à titre non limitatif, les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer à titre non limitatif, l'hydroxyde de sodium, l'hydroxyde de potassium, la triéthylamine, etc...

Les composés préférés de l'invention sont les composés de formule (I) répondant plus particulièrement à la formule (IA) :

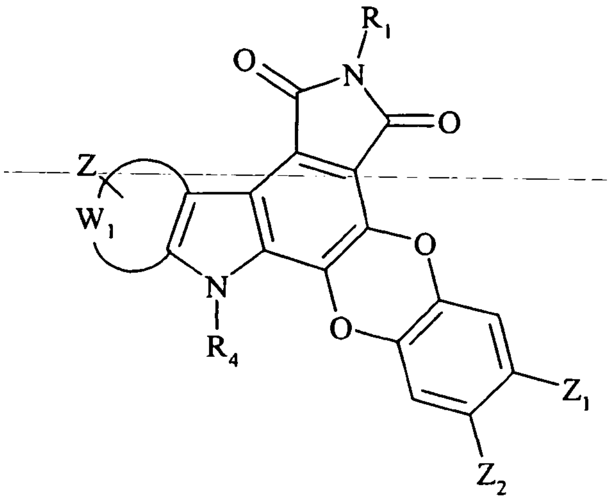

(IA)

dans laquelle $R_1$, $R_4$, $W_1$, Z, $Z_1$ et $Z_2$ sont tels que définis dans la formule (I).

Selon une deuxième variante avantageuse, les composés préférés de l'invention sont les composés de formule (I) répondant plus particulièrement à la formule (IB) :

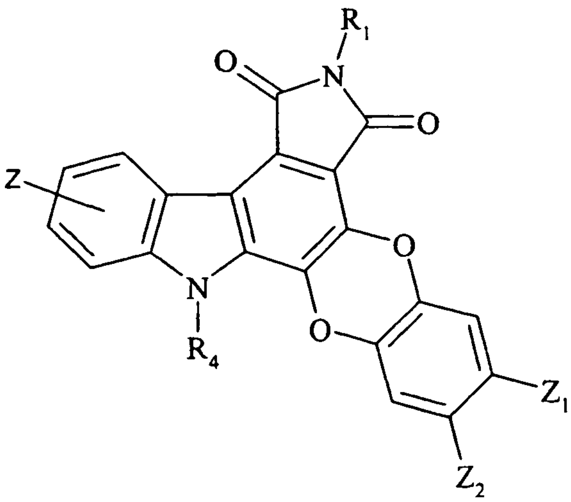

(IB)

dans laquelle $R_1$, $R_4$, Z, $Z_1$ et $Z_2$ sont tels que définis dans la formule (1).

Selon une troisième variante avantageuse, les composés préférés de l'invention sont les composés de formule (I) répondant plus particulièrement à la formule (IC) :

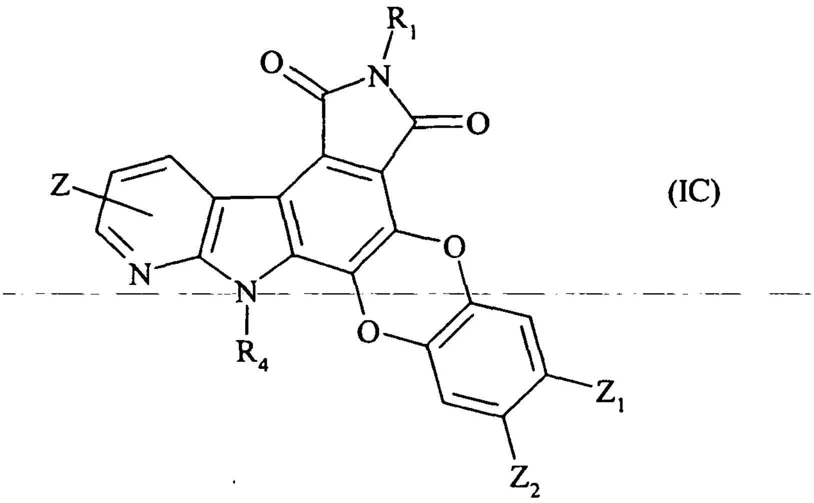

(IC)

dans laquelle $R_1$, $R_4$, Z, $Z_1$ et $Z_2$ sont tels que définis dans la formule (I).

Selon une quatrième variante avantageuse, les composés préférés de l'invention sont les composés de formule (I) répondant plus particulièrement à la formule (ID) :

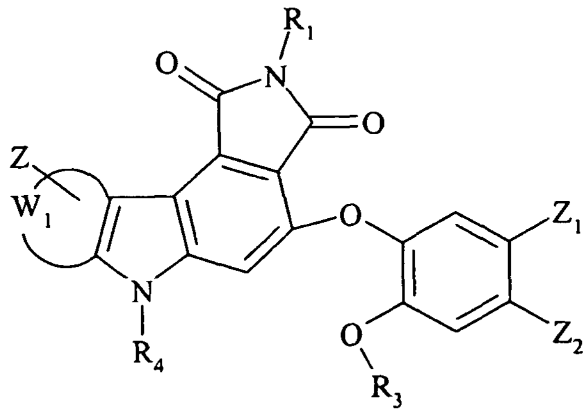

(ID)

dans laquelle $R_1$, $R_3$, $R_4$, $W_1$, Z, $Z_1$ et $Z_2$ sont tels que définis dans la formule (I).

Selon une cinquième variante avantageuse, les composés préférés de l'invention sont les composés de formule (I) répondant plus particulièrement à la formule (IE) :

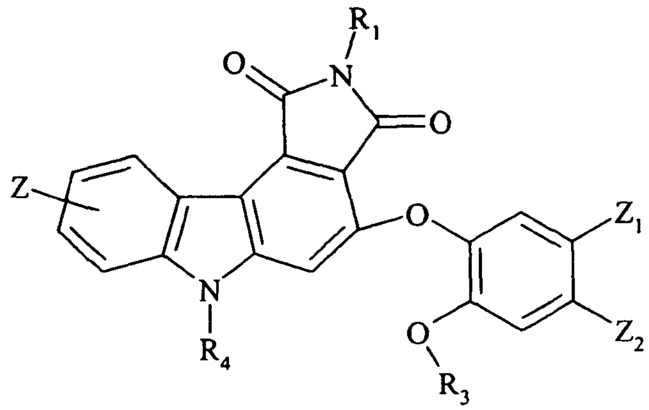

(IE)

dans laquelle $R_1$, $R_3$, $R_4$, Z, $Z_1$ et $Z_2$ sont tels que définis dans la formule (I).

Selon une sixième variante avantageuse, les composés préférés de l'invention sont les composés de formule (I) répondant plus particulièrement à la formule (IF) :

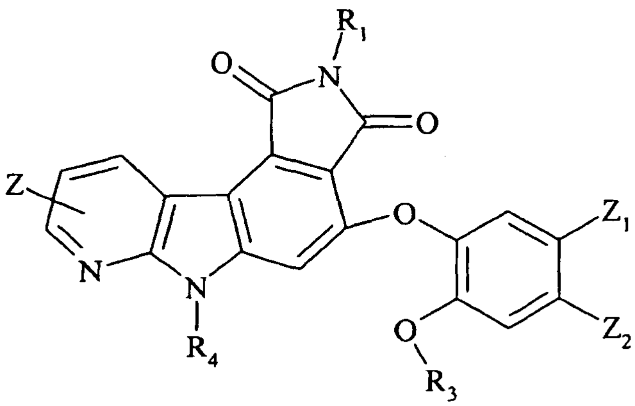

(IF)

dans laquelle $R_1$, $R_3$, $R_4$, Z, $Z_1$ et $Z_2$ sont tels que définis dans la formule (I).

Selon une septième variante avantageuse, les composés préférés de l'invention sont les composés de formule (I) répondant plus particulièrement à la formule (IG) :

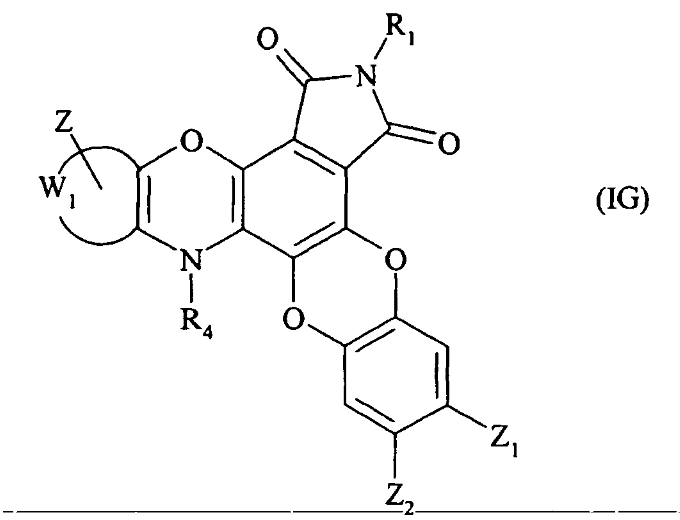

(IG)

dans laquelle $R_1$, $R_4$, $W_1$, Z, $Z_1$ et $Z_2$ sont tels que définis dans la formule (I).

Selon une huitième variante avantageuse, les composés préférés de l'invention sont les composés de formule (I) répondant plus particulièrement à la formule (IH) :

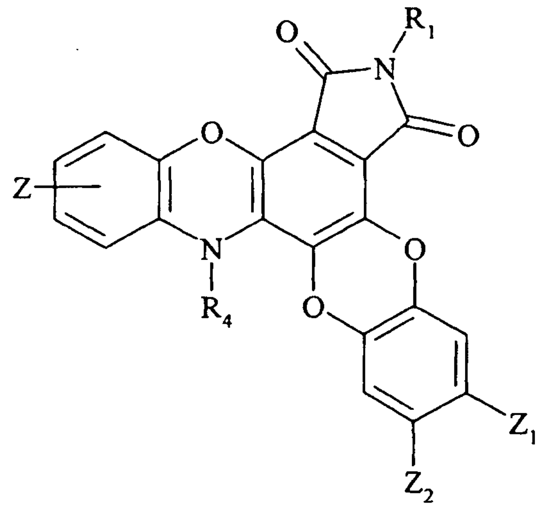

(IH)

dans laquelle $R_1$, $R_4$, Z, $Z_1$ et $Z_2$ sont tels que définis dans la formule (I).

Selon une neuvième variante avantageuse, les composés préférés de l'invention sont les composés de formule (I) répondant plus particulièrement à la formule (IJ) :

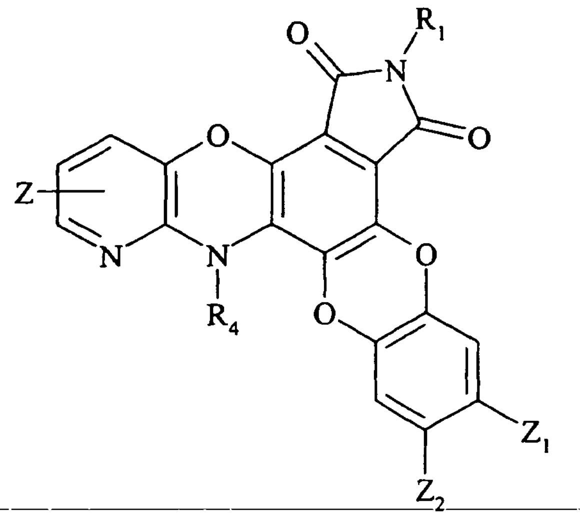

(IJ)

dans laquelle $R_1$, $R_4$, Z, $Z_1$ et $Z_2$ sont tels que définis dans la formule (I).

D'une façon intéressante, le groupement Z préféré selon l'invention est l'atome d'hydrogène, halogène et le groupement hydroxy.

D'une façon avantageuse, le groupement A, avec les atomes de carbone auxquels ils sont liés, préférés selon l'invention sont les groupements de formule :

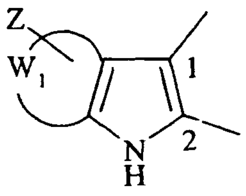 ou 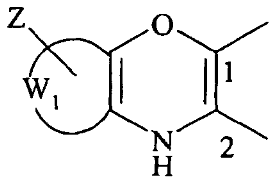 .

D'une façon particulièrement intéressante, le groupement $R_3$ préféré selon l'invention est l'atome d'hydrogène et le groupement alkyle ($C_1$-$C_6$) linéaire ou ramifié.

D'une façon particulièrement avantageuse, le groupement $R_1$ préféré selon l'invention est l'atome d'hydrogène, le groupement alkyle ($C_1$-$C_6$) linéaire ou ramifié et une chaîne alkylène ($C_1$-$C_6$) linéaire ou ramifiée substituée par un ou plusieurs groupements, identiques ou différents, choisis parmi -$NR_5R_6$, dans lequel $R_5$ et $R_6$ sont tels que définis dans la formule (I).

D'une autre façon intéressante les groupements $Z_1$ et $Z_2$ préférés selon l'invention sont l'atome d'hydrogène.

Les composés préférés selon l'invention sont le :

- 7-méthyl[1,4]benzodioxino[2,3-a]pyrrolo[3,4-c]carbazole-6,8-dione,
- 10-fluoro-7-méthyl[1,4]benzodioxino[2,3-a]pyrrolo[3,4-c]carbazole-6,8-dione,
- 11-fluoro-7-méthyl[1,4]benzodioxino[2,3-a]pyrrolo[3,4-c]carbazole-6,8-dione,
- 7-[2-(diméthylamino)éthyl]- 1 0-fluoro[1,4]benzodioxino[2,3-a]pyrrolo[3,4-c] carbazole-6,8-dione,
- 10-hydroxy[1,4]benzodioxino[2,3-a]pyrrolo[3,4-c]carbazole-6,8-dione,
- 11 -hydroxy[1,4]benzodioxino[2,3-a]pyrrolo[3,4-c]carbazole-6,8-dione,
- 7-[2-(diméthytamino)éthyl][1,4]benzodioxino[2,3-a]pyrrolo[3,4-c]carbazole-6,8-dione,
- 7-[2-(diméthylamino)éthyl]-10-hydroxy[1,4]benzodioxino[2,3-a]pyrrolo[3,4-c] carbazole-6,8-dione,
- 7-[2-(diméthylamino)éthyl]-11-hydroxy[1,4]benzodioxino[2,3-a]pyrrolo[3,4-c] carbazole-6,8-dione,
- 7-[2-(diméthylamino)éthyl][1,4]benzodioxino[2,3-e]pyrido[2',3':5,6][1,4]oxazino [3,2-g]isoindole-6,8-dione.

Les énantiomères, diastéréoisomères, N-oxydes, ainsi que leurs sels d'addition à un acide ou à une base pharmaceutiquement acceptable des composés préférés font partie intégrante de l'invention.

L'invention s'étend également au procédé de préparation des composés de formule (I), caractérisé en ce que l'on utilise comme produit de départ un composé de formule (II) :

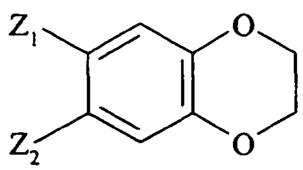 (II)

dans laquelle $Z_1$ et $Z_2$ sont tels que définis dans la formule (I), composé de formule (II) que l'on fait réagir avec du N-bromosuccinimide en présence de péroxyde de benzoyle pour conduire au composé de formule (III) :

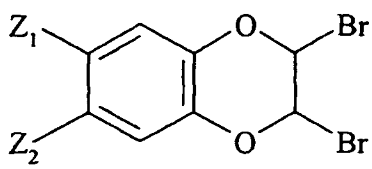 (III)

dans laquelle $Z_1$ et $Z_2$ sont tels que définis précédemment,

composé de formule (III) qui est mis à réagir avec de l'iodure de sodium pour conduire au composé de formule (IV) :

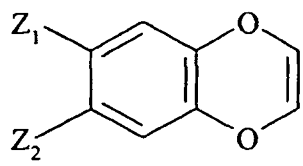

(IV)

dans laquelle $Z_1$ et $Z_2$ sont tels que définis précédemment,
composé de formule (IV) qui est mis à réagir avec du n-butyllithium puis du chlorure de triméthylétain pour conduire au composé de formule (V) :

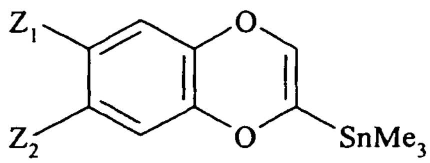

(V)

dans laquelle $Z_1$ et $Z_2$ sont tels que définis précédemment,
composé de formule (V) qui est :

- soit traité, en présence de tétrakis(triphénylphosphine)palladium (0), par un composé de formule (VI) :

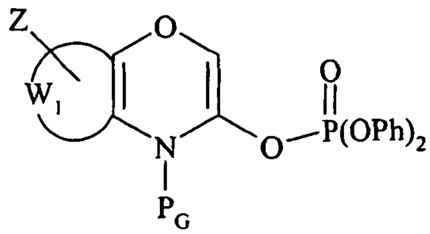

(VI)

dans laquelle $P_G$ représente un groupement protecteur des amines bien connu en synthèse organique et $W_1$ et Z sont tels que définis dans la formule (I) pour conduire au composé de formule (VII) :

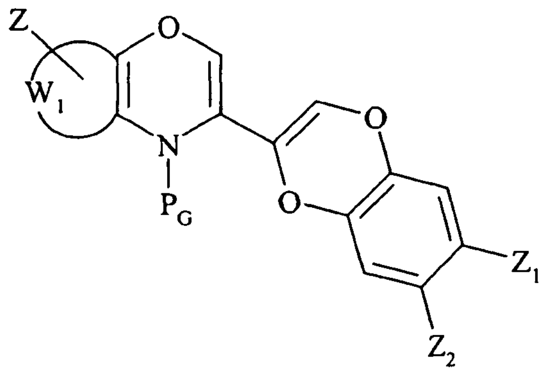

(VII)

dans laquelle $P_G$, Z, $Z_1$, $Z_2$ et $W_1$ sont tels que définis précédemment, composé de formule (VII) qui est traité par un composé de formule (VIII) :

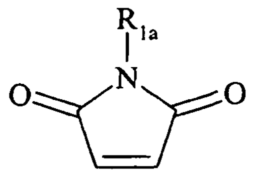

(VIII)

dans laquelle $R_{1a}$, représente un atome d'hydrogène ou un groupement méthyle, pour conduire au composé de formule (IX) :

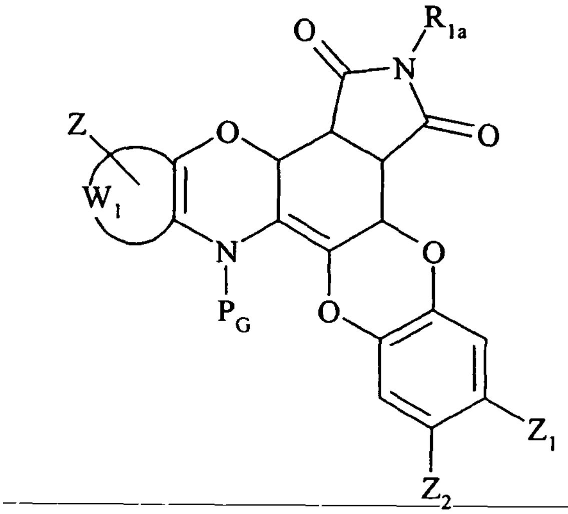

(IX)

dans laquelle $P_G$, $R_{1a}$, Z, $Z_1$, $Z_2$ et $W_1$ sont tels que définis précédemment, composé de formule (IX) qui est mis en présence de N-bromosuccinimide et de péroxyde de benzoyle, pour conduire au composé de formule (I/a), cas particulier des composés de formule (I) :

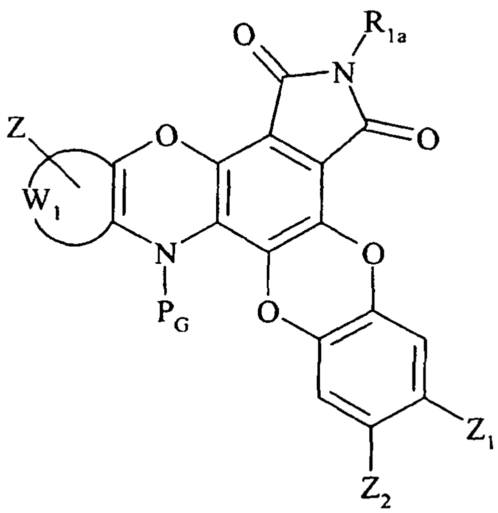

(I/a)

dans laquelle $P_G$, $R_{1a}$, Z, $Z_1$, $Z_2$ et $W_1$ sont tels que définis précédemment, composé de formule (I/a) qui est éventuellement traité par un composé de formule (X) :

$R_{1b}$-$NH_2$ (X)

dans laquelle $R_{1b}$, différent de atome d'hydrogène et de groupement méthyle, a la même définition que $R_1$ dans la formule (I), pour conduire au composé de formule (I/b), cas particulier des composés de formule (I) :

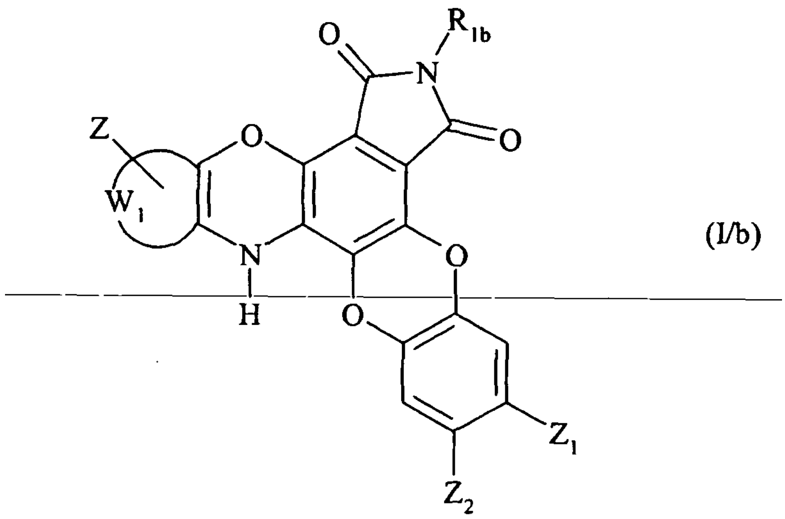

(I/b)

dans laquelle $R_{1b}$, Z, $Z_1$, $Z_2$ et $W_1$ sont tels que définis précédemment, les composés des formules (I/a) et (I/b) forment les composés de formule (1/c) :

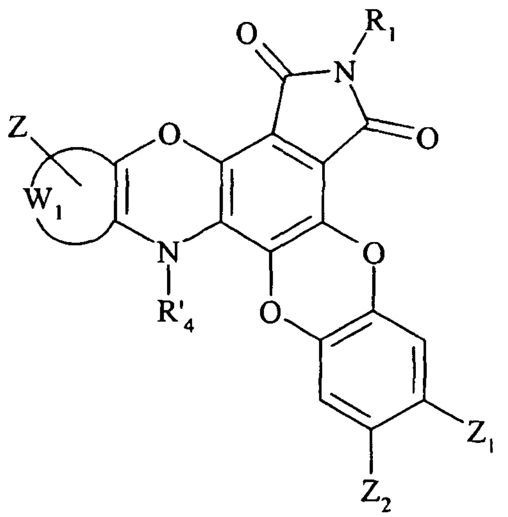

(I/c)

dans laquelle $R'_4$ représente un atome d'hydrogène ou un groupement $P_G$ et $R_1$, Z, $Z_1$, $Z_2$ et $W_1$ sont tels que définis précédemment,

- soit traité, en présence de chlorure de bistriphénylphosphinepalladium (II), par un composé de formule (XI) :

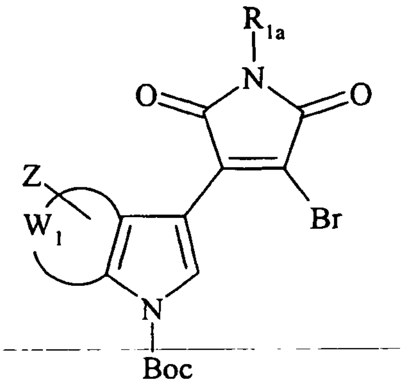

(XI)

dans laquelle Boc représente un groupement tert-butoxycarbonyle et $R_{1a}$, $W_1$ et Z sont tels que définis précédemment, pour conduire au composé de formule (XII) :

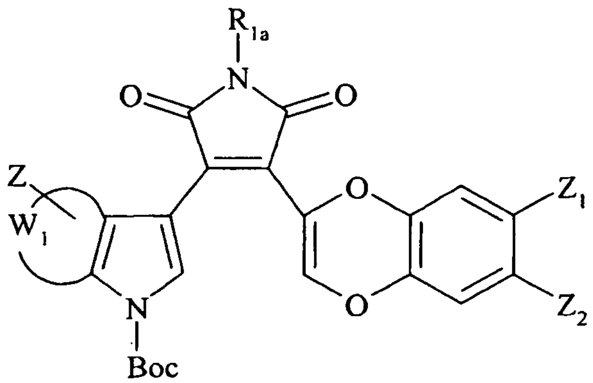

(XII)

dans laquelle Boc, $R_{1a}$, Z, $Z_1$, $Z_2$ et $W_1$ sont tels que définis précédemment, composé de formule (XII) qui est :

♦ soit irradié par une lampe UV, en présence d'iode, dans un solvant apolaire et aprotique, pour conduire aux composés de formules (I/d) et (I/e), cas particulier des composés de formule (I) :

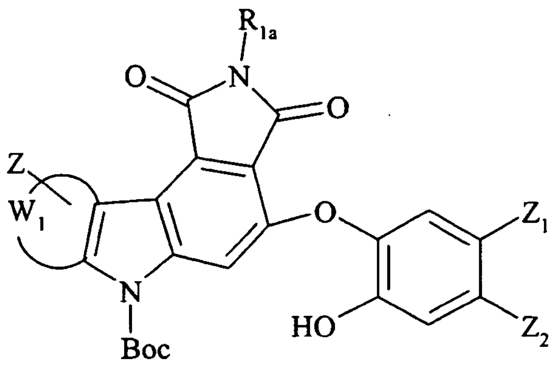

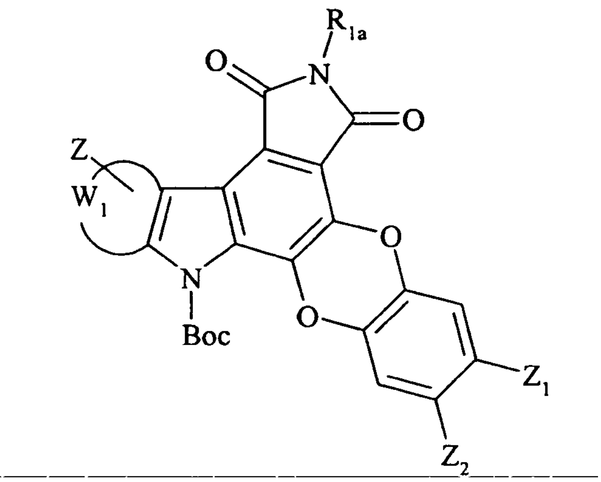

(I/d) (I/e)

dans lesquelles Boc, $R_{1a}$, Z, $Z_1$, $Z_2$ et $W_1$ sont tels que définis précédemment, composés de formule (I/d) :

- dont on déprotège éventuellement la fonction amine selon des méthodes classiques de la synthèse organique pour conduire au composé de formule (I/f), cas particulier des composés de formule (I) :

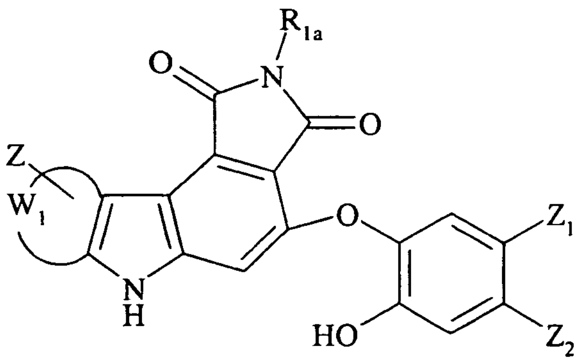

(I/f)

dans laquelle $R_{1a}$, Z, $Z_1$, $Z_2$ et $W_1$ sont tels que définis précédemment,

- soit soumis éventuellement à l'action d'un composé de formule (XIII) :

$R_{3a}$-G (XIII)

dans laquelle $R_{3a}$, différent de atome d'hydrogène, à la même définition que $R_3$ dans la formule (I) et G est tel que défini précédemment, pour conduire au composé de formule (I/g), cas particulier des composés de formule (I) :

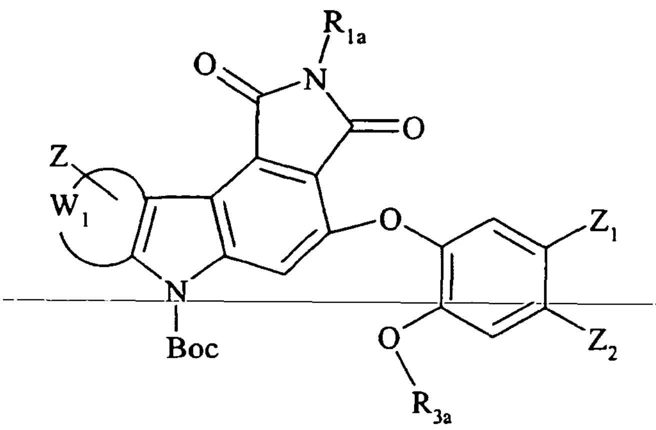

(I/g)

dans laquelle Boc, $R_{1a}$, $R_{3a}$, Z, $Z_1$, $Z_2$ et $W_1$ sont tels que définis précédemment, les composés de formule (I/d), (I/e) et (I/g) forment le composé de formule (I/h) :

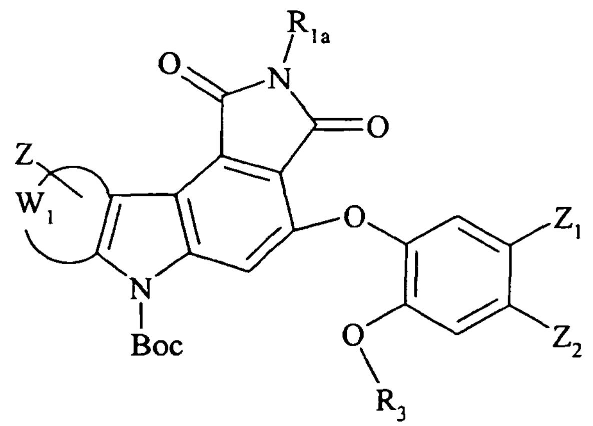

(I/h)

dans laquelle Boc, $R_{1a}$, $R_3$, Z, $Z_1$, $Z_2$ et $W_1$ sont tels que définis précédemment, composés de formule (I/h) qui est éventuellement soumis aux mêmes conditions de réaction que le composé de formule (1/a), pour conduire au composé de formule (I/i), cas particulier des composés de formule (I) :

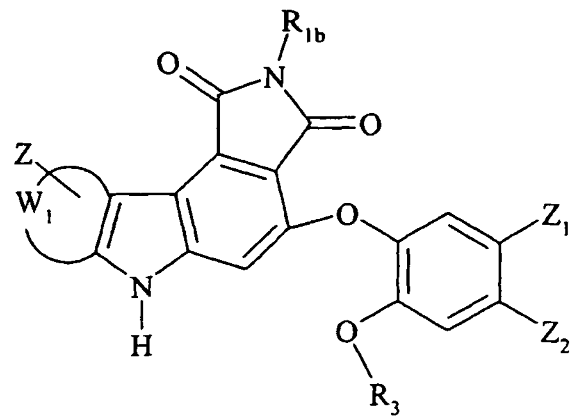

(I/i)

dans laquelle $R_{1b}$, $R_3$, Z, $Z_1$, $Z_2$ et $W_1$ sont tels que définis précédemment,

♦ soit soumis à l'action d'acide chlorhydrique pour conduire au composé de formule (XIV):

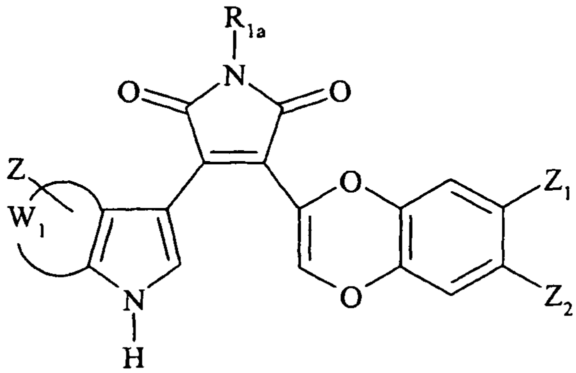

(XIV)

dans laquelle $R_{1a}$, Z, $Z_1$, $Z_2$ et $W_1$ sont tels que définis précédemment,
composé de formule (XIV) qui est soumis à l'action d'un composé de formule (XV) :

$R_{4a}$-G (XV)

dans laquelle G représente un groupement partant et $R_{4a}$, différent de atome d'hydrogène, a la même définition que $R_4$ dans la formule (I), pour conduire au composé de formule (XVI) :

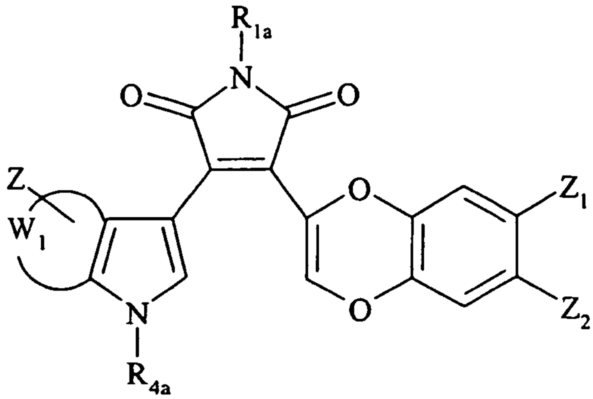

(XVI)

dans laquelle $R_{1a}$, $R_{4a}$, Z, $Z_1$, $Z_2$ et $W_1$ sont tels que définis précédemment,
composé de formule (XVI) qui est soumis aux mêmes conditions de réaction que le composé de formule (XII) pour conduire aux composés de formules (I/j) et (I/k) cas particulier des composés de formule (I) :

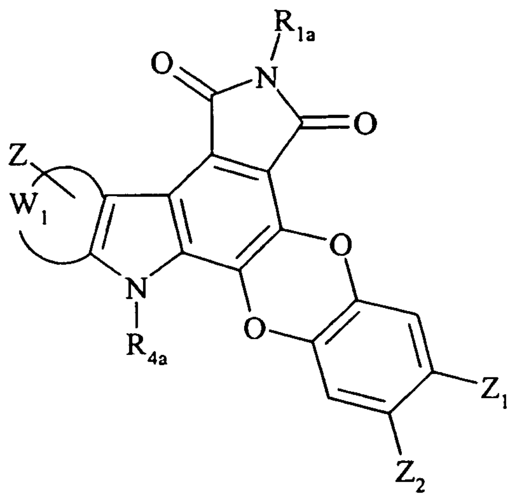

(I/j)

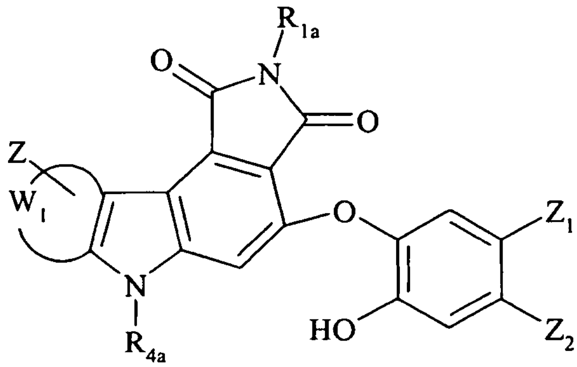

(I/k)

dans lesquelles $R_{1a}$, $R_{4a}$, Z, $Z_1$, $Z_2$ et $W_1$ sont tels que définis précédemment,
composé de formule (I/k) qui est éventuellement soumis à l'action d'un composé de formule (XIII) tel que défini précédemment pour conduire au composé de formule (I/l) :

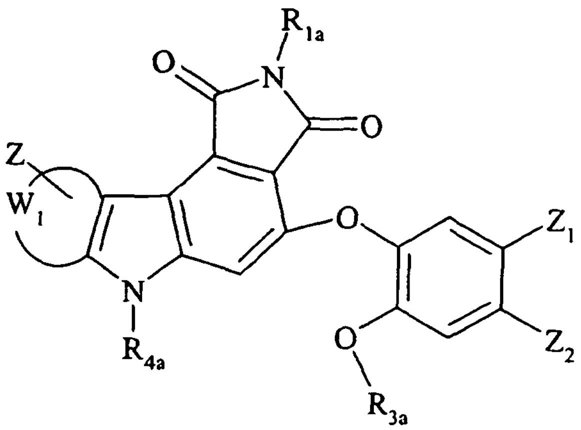

(I/l)

dans laquelle $R_{1a}$, $R_{3a}$, $R_{4a}$, Z, $Z_1$, $Z_2$ et $W_1$ sont tels que définis précédemment, les composés de formule (I/j), (I/k) et (I/l) forment les composés de formule (I/m) :

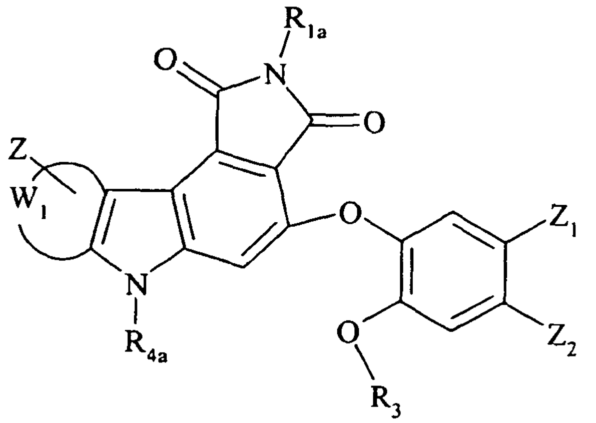

(I/m)

dans laquelle $R_{1a}$, $R_3$, $R_{4a}$, Z, $Z_1$, $Z_2$ et $W_1$ sont tels que définis précédemment,
composé de formule (I/m) qui est éventuellement soumis aux mêmes conditions de réaction que le composé de formule (I/h) pour conduire au composé de formule (I/n) :

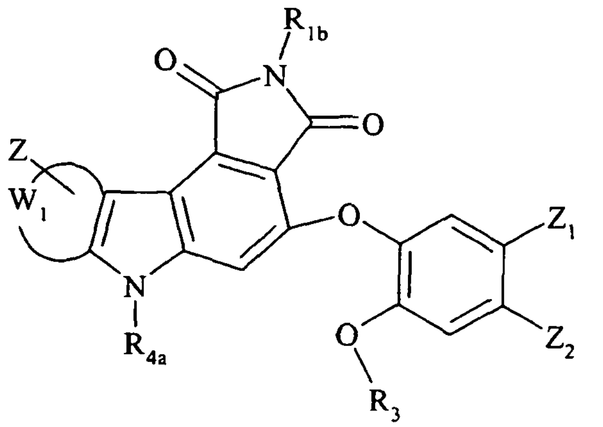

(I/n)

dans laquelle $R_{1b}$, $R_3$, $R_{4a}$, Z, $Z_1$, $Z_2$ et $W_1$ sont tels que définis précédemment,
les composés de formule (I/e), (I/h) et (I/i), (I/m) et (I/n) forment les composés de formule (I/o) :

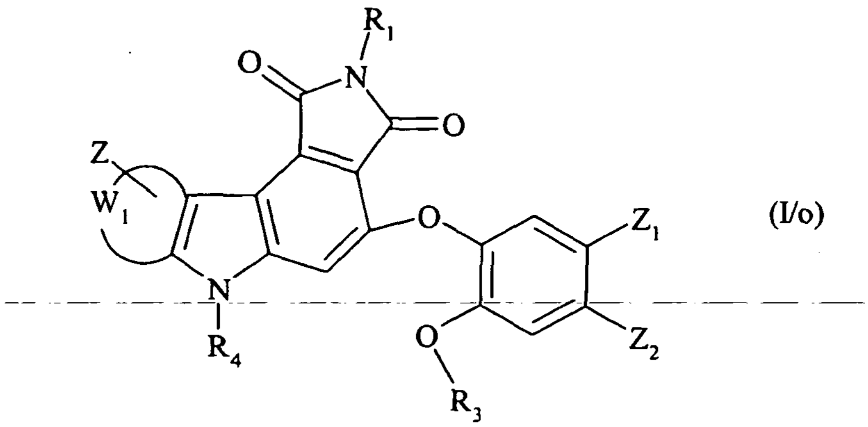

(I/o)

dans laquelle $R_1$, $R_3$, $R_4$, Z, $Z_1$, $Z_2$ et $W_1$ sont tels que définis précédemment,

les composés de formule (I/a) à (I/o) forment l'ensemble des composés de formule (I), que l'on purifie, le cas échéant, selon des techniques classiques de purification, qui peuvent, si on le désire, être séparés en leurs différents isomères, selon une technique classique de séparation, et que l'on transforme, si on le souhaite, en leurs N-oxydes et, le cas échéant, en leurs sels d'addition à un acide ou à une base pharmaceutiquement acceptable.

Les composés de formule (VI) peuvent avantageusement être obtenus à partir d'un composé de formule (A) :

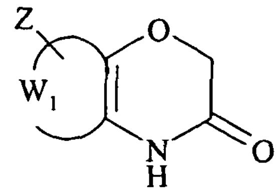

(A)

dans laquelle Z et $W_1$ sont tels que définis précédemment,
composé de formule (A) dont on protège la fonction amine avec un groupement protecteur $P_G$ bien connu de l'homme de l'art pour conduire au composé de formule (B) :

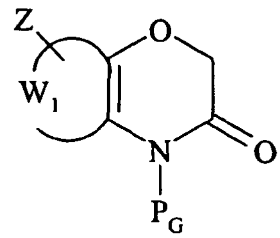

(B)

dans laquelle $P_G$ représente un groupement tert-butoxycarbonyle ou phénoxycarbonyle et $W_1$ et Z sont tels que définis précédemment,
composé de formule (B) qui est traité par du diisopropylamidure de lithium suivi de chlorophosphate de diphényle pour conduire au composé de formule (VI).

Les composés de formule (XI) peuvent avantageusement être obtenus à partir d'un composé de formule (C) :

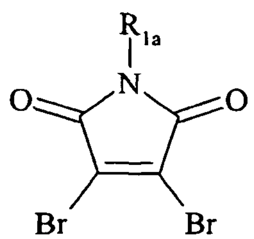

(C)

dans laquelle $R_{1a}$ est tel que défini précédemment,
composé de formule (C) qui est traité, en présence de bis(triméthylsilyl)amidure de lithium, par un composé de formule (D) :

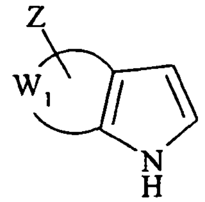

(D)

dans laquelle $W_1$ et Z sont tels que définis précédemment, pour conduire à un composé de formule (E) :

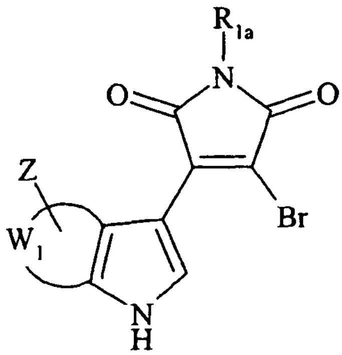

(E)

dans laquelle $R_{1a}$, Z et $W_1$ sont tels que définis précédemment,
composé de formule (E), qui est mis à réagir avec du *di*-tert-butyl-dicarbonate en présence de 4-diméthylaminopyridine pour conduire au composé de formule (XI).

Les composés de formule (II), (VIII), (X), (XIV), (XVI), (A), (C) et (D) sont soit des composés commerciaux, soit obtenus selon des méthodes classiques de la synthèse organique facilement accessibles à l'homme du métier.

Les composés de formule (I) possèdent d'intéressantes propriétés pharmacologiques. Ils ont une excellente cytotoxicité *in vitro* non seulement sur des lignées leucémiques mais également sur des lignées de tumeurs solides ; ils ont également une action sur le cycle cellulaire et sont actifs *in vivo,* sur un modèle leucémique. Ces propriétés permettent leur utilisation en thérapeutique en tant qu'agents antitumoraux.

Parmi les types de cancer qui peuvent être traités par les composés de la présente invention, on peut citer à titre non limitatif les adénocaminomes et carcinomes, sarcomes, gliomes et leucémies

La présente invention a également pour objet les compositions pharmaceutiques contenant les produits de formule (I), ses énantiomères, diastéréoisomères, N-oxydes ou un de leurs sels d'addition à une base ou un acide pharmaceutiquement acceptables, seuls ou en combinaison avec un ou plusieurs excipients ou véhicules inertes, non toxiques. Parmi les compositions pharmaceutiques selon l'invention, il sera cité plus particulièrement celles qui conviennent pour l'administration orale, parentérale, nasale, rectale, perlinguale, oculaire ou respiratoire, et notamment les comprimés simples ou dragéifiés, les comprimés sublinguaux, les sachets, les paquets, les gélules, les glossettes, les tablettes, les suppositoires, les crèmes, les pommades, les gels dermiques, les préparations injectables ou buvables, les aérosols, les gouttes oculaires ou nasales.

De part les propriétés pharmacologiques caractéristiques des composés de formule (I), les compositions pharmaceutiques contenant comme principe actif lesdits composés de formule (I), sont donc particulièrement utiles pour le traitement des cancers.

La posologie utile varie selon l'âge et le poids du patient, la voie d'administration, la nature de l'indication thérapeutique et des traitements éventuellement associés et s'échelonne entre 0,1 et 400 mg par jour, en une ou plusieurs administrations.

Les exemples suivants illustrent l'invention et ne la limitent en aucune façon. Les produits de départ utilisés sont des produits connus ou préparés selon des modes opératoires connus.

Les structures des composés décrits dans les exemples ont été déterminées selon les techniques spectrophotométriques usuelles (infrarouge, résonance magnétique nucléaire, spectrométrie de masse, ...).

**PREPARATION A : Benzo[1,4]dioxine-2-yl(triméthyl)stannane**

***Stade A : 2,3-Dibromo-2,3-dihydro-1,4-benzodioxine***

Sous atmosphère inerte, 73,45 mmol de 1,4-benzodioxane, 150 ml de tétrachlorure de carbone, puis 163,32 mmol de N-bromosuccinimide recristallisé et 88,1 μmol de péroxyde de benzoyle sont mélangés. Le milieu réactionnel est porté à reflux à l'aide d'une lampe (60 W) pendant 6 heures. Le succinimide précipité est éliminé par filtration et le filtrat est concentré permettant d'isoler le produit attendu.

***Stade B : Benzo[1,4]dioxine***

73,45 mmol du composé obtenu au stade A précédent sont dissous dans 125 ml d'acétone, puis la solution est agitée pendant 2 heures à reflux, en présence d'iodure de sodium (359,9 mmol). Après évaporation du solvant, le résidu est mis en solution dans un mélange eau/acétate d'éthyle (100 ml/200 ml). La phase organique est ensuite lavée par une solution aqueuse de thiosulfate de sodium à 20%, puis la phase aqueuse est extraite deux fois à l'acétate d'éthyle. Les phases organiques sont réunies, séchées sur sulfate de magnésium puis concentrées à sec. Une purification par chromatographie sur gel de silice (éther de pétrole/acétate d'éthyle:95/5), permet d'obtenir le produit attendu.
IR(film NaCl) : $\nu_{(C=C\ \text{éther d'énol})} = 1665\ cm^{-1}$ ; $\nu_{(C=C\ Ar)} = 1590\ cm^{-1}$.

***Stade C: Benzo[1,4]dioxine-2-yl(triméthyl)stannane***

14,91 mmol de 1,4-benzodioxine sont dissous dans 18 ml de tétrahydrofurane anhydre sous atmosphère anhydre, et la température de la solution est abaissée à -78°C. 23,86 mmol d'une solution 1,5M de n-butyllithium dans l'hexane sont ajoutés goutte à goutte au milieu réactionnel qui est agité à -78°C pendant 2h15. 37,28 mmol de chlorure de triméthylétain en solution dans 10 ml de tétrahydrofurane anhydre sont additionnés goutte à goutte et le mélange réactionnel est agité à -78°C pendant 45 minutes, puis remonté à température ambiante pendant 15 heures. Le milieu réactionnel est hydrolysé avec une solution aqueuse à 15% en fluorure de potassium, et agité pendant 45 minutes. Les sels d'étain précipités sont éliminés par filtration, et la phase aqueuse extraite 3 fois à l'acétate d'éthyle. Les phases organiques sont réunies et concentrées. Une purification par chromatographie flash sur gel de silice (éther de pétrole/acétate d'éthyle : 95/5), permet d'obtenir le produit attendu.
IR(film NaCl) : $\nu_{(C=C\ \text{éther d'énol})} = 1639\ cm^{-1}$; $\nu_{(C=C\ Ar)} = 1592\ cm^{-1}$.

**PREPARATION B: Naphto[2,3-b][1,4]dioxine-2-yl(tributyl)stannane**

***Stade A: 2,3-Dihydronaphto[2,3-b][1,4]dioxine-2-carboxylate d'éthyle***

Sous atmosphère inerte, 47,03 mmol de carbonate de potassium sec sont additionnés à une solution de 62,43 mmol de 2,3 dihydroxynaphtalène dans 150 ml d'acétone anhydre. Le milieu réactionnel est porté à reflux, après addition de 17,2 mmol de 2,3-dibromopropanoate d'éthyle. 15 minutes plus tard, 47,03 mmol de carbonate de potassium sec, ainsi que 17,2 mmol de 2,3-dibromopropanoate d'éthyle sont à nouveau ajoutés au milieu réactionnel. Cette opération est encore répétée 2 fois, toutes les 15 minutes. Le reflux est maintenu pendant 18 heures. Le milieu réactionnel est ensuite filtré, et le résidu lavé avec de l'acétone. Le filtrat est concentré, puis repris avec de l'acétate d'éthyle, et lavé avec 100 ml d'eau. La phase aqueuse est extraite trois fois avec de l'acétate d'éthyle, puis les phases organiques sont réunies, séchées sur du sulfate de magnésium filtrées, et évaporées sous pression réduite. Une purification par chromatographie flash sur gel de silice (éther de pétrole/acétate d'éthyle : 9/1), permet d'isoler le produit attendu.
IR(film NaCl) : $\nu_{C=O} = 1759\ cm^{-1}$.
Spectre de masse : 259 $[M + 1]^+$.

***Stade B : Naphto[2,3-b][1,4]dioxine-2-carboxylate d'éthyle***

Sous atmosphère inerte, une solution de 25,94 mmol du composé obtenu au stade A précédent dans 120 ml de tétrachlorure de carbone, est portée à reflux à l'aide d'une lampe (60 W), en présence de 57,03 mmol de N-bromosuccinimide recristallisé, et d'une quantité catalytique de péroxyde de benzoyle. L'agitation est maintenue à reflux pendant 2h30. Après refroidissement, le succinimide libéré est filtré, et le filtrat est concentré à sec sous vide. L'ester dibromé obtenu, est dissous dans 100 ml d'acétone, puis 129,62 mmol d'iodure de sodium sont ajoutés. L'agitation est maintenue à température ambiante pendant 4 heures. Après évaporation du solvant, le résidu est repris dans un mélange eau/acétate d'éthyle, et lavé avec une solution aqueuse de thiosulfate de sodium 1M. La phase organique est séchée sur du sulfate de magnésium, filtrée, et évaporée. Une purification par chromatographie flash sur gel de silice (éther de

pétrole/acétate d'éthyle : 9/1), permet d'isoler le produit attendu.
Point de fusion : 98°C.
IR(KBR) : $\nu_{C=O}$ = 1724 $cm^{-1}$ ; $\nu_{C=C}$ = 1682 $cm^{-1}$.
Spectre de masse : 257 $[M + 1]^+$.

***Stade C**: Acide naphto[2,3-b][1,4]dioxine-2-carboxylique*

Une solution de 24,58 mmol du composé obtenu au stade B précédent dans 25 ml de méthanol, est portée à reflux pendant une heure, en présence de 20 ml d'une solution aqueuse d'hydroxyde de lithium 1M. Après refroidissement, et évaporation du méthanol, le mélange réactionnel est acidifié avec une solution aqueuse d'acide chlorhydrique 1M, jusqu'à obtention d'un pH = 1. Le précipité formé est filtré, permettant d'isoler le produit attendu.
Point de fusion : > 360°C.
IR(KBR): $\nu_{C=O}$ = 1678 $cm^{-1}$ ; $\nu_{C=C}$ = 1661 $cm^{-1}$ ; $\nu_{OH}$ = 3450 $cm^{-1}$.
Spectre de masse : 229 $[M + 1]^+$.

***Stade D**: Naphto[2,3-b][1,4]dioxine*

Une solution de 0,66 mmol du composé obtenu au stade C précédent dans 1 ml de quinoléine, est portée à 220°C pendant 3 heures, en présence d'une quantité catalytique de poudre de cuivre. Après refroidissement, le résidu est repris dans de l'acétate d'éthyle, et lavé avec une solution aqueuse d'acide chlorhydrique 1M. La phase organique est séchée sur sulfate de magnésium filtrée, et concentrée. Une purification par chromatographie flash sur gel de silice (éther de pétrole/acétate d'éthyle : 9/1), permet d'obtenir le produit attendu.
Point de fusion : 96-98°C.
IR(KBR) : $\nu_{C=C}$ = 1665 $cm^{-1}$ ; $\nu_{C-O}$= 1296 $cm^{-1}$ .

***Stade E** : Naphto[2,3-b][1,4]dioxine-2-yl(tributyl)stannane*

Le produit attendu est obtenu selon le procédé décrit au stade C de la préparation A à partir du composé du stade D précédent et du chlorure de tributylétain.
IR (film NaCl) : $\nu_{C-O}$ = 1166, 1247 $cm^{-1}$.
Spectre de masse : 473 $[M + 1]^+$.

**PREPARATION C : 3-[(Diphénoxyphosphoryl)oxy]-4*H*-1,4-benzoxazine-4-carboxylate de phényle**

***Stade A** : 2,3-Dihydro-4H-1,4-benzoxazine-3-one-4-carboxylate de phényle*

Sous atmosphère anhydre, une solution de 10 mmol de *2H*-1,4-benzoxazine-3-one dans 50 ml de tétrahydrofurane est refroidie à -78°C. A cette température, 11 mmol d'une solution de *n*-butyllithium à 1,6M dans l'hexane sont ajoutés goutte à goutte. Après 30 minutes de temps de contact à -78°C, 11 mmol de chloroformiate de phényle sont ajoutés goutte à goutte et l'agitation est maintenue pendant 2 heures supplémentaires. Après retour à température ambiante, la solution est hydrolysée puis extraite à l'acétate d'éthyle. La phase organique est séchée sur sulfate de magnésium, filtrée et évaporée. Après purification par chromatographie sur gel de silice (éther de pétrole/acétate d'éthyle : 8/2), le produit attendu est isolé.
IR (film NaCl) : $\nu_{C=O}$ = 1739, 1796 $cm^{-1}$.
Spectre de masse : 270 (M + 1).

***Stade B** : 3-[(Diphénoxyphosphoryl)oxy]-4H-1,4-benzoxazine-4-carboxylate de phényle*

Sous atmosphère anhydre, une solution de 10 mmol du produit obtenu au stade A précédent dans 50 ml de THF anhydre est refroidie à -78°C. A cette température 12 mmol de LDA 2M (dans une solution heptane /THF) sont ajoutés goutte à goutte. Après 2 heures d'agitation 12 mmol de chlorophosphate de diphényle sont ajoutés goutte à goutte au mélange réactionnel qui est maintenue pendant 2 heures supplémentaires à -78°C.
Après retour à température ambiante, la solution est hydrolysée puis extraite à l'acétate d'éthyle. La phase organique est séchée sur sulfate de magnésium, filtrée et concentrée. Après purification du résidu par chromatographie sur gel de silice (éther de pétrole/acétate d'éthyle : 9/1), le produit attendu est isolé.
IR(film NaCl) : $\nu_{C=O}$ = 1748 $cm^{-1}$ ; $\nu_{P=O}$= 1315 $cm^{-1}$,
Spectre de masse : 502 (M + 1).

**PREPARATION D : 3-[(Diphénoxyphosphoryl)oxy]-2,3-dihydro-*4H*-pyrido [3,2-b][1,4]oxazine-4-carboxylate de phényle**

***Stade A : 1,3-Dihydro-4H-pyrido[3,1-b][1,4]oxazine-4-carboxylate de phényle***

Le produit attendu est obtenu selon le procédé décrit au stade A de la préparation C en utilisant le chloroformiate de phényle comme substrat.
Point de fusion : 97°C.
IR(KBr): $\nu_{C=O}$= 1717 $cm^{-1}$; 1803 $cm^{-1}$.
Spectre de masse : m/z 271 (M + 1).

***Stade B : 3-[(Diphénoxyphosphoryl)oxy]-2,3-dihydro-4H-pyrido[3,2-b][1,4]oxazine-4-carboxylate de phényle***

Le produit attendu est obtenu selon le procédé décrit au stade B de la préparation C à partir du composé du stade précédent.
Point de fusion : 82°C.
IR(KBr) : $\nu_{C=O}$ = 1749 $cm^{-1}$ ; $\nu_{P=O}$ 1294 $cm^{-1}$.
Spectre de masse : m/z 503 (M + 1).

**PREPARATION E : 3-Bromo-4-(1*H*-1-carboxylate de tert-butyle-indol-3-yl)-1-méthyl-1*H*-pyrrole-2,5-dione**

***Stade A : 3-Bromo-4-(1H-indol-3-yl)-1-méthyl-1H-pyrrole-2,5-dione***

11,16 mmol d'indole sont mis en solution dans 29 ml de tétrahydrofurane anhydre sous atmosphère inerte. La température du milieu réactionnel est abaissée à -15°C, puis 17,85 mmol d'une solution 1M de bis(triméthylsilyl)lithium dans de l'hexane sont additionnées goutte à goutte. Après agitation à -15°C pendant 1h10, 7,44 mmol de 2,3-dibromo-*N*-méthylmaléimide en solution dans 8 ml de tétrahydrofurane anhydre sont ajoutées au milieu réactionnel, qui est ensuite agité pendant 20 minutes à -15°C, et 15 minutes de - 15°C à température ambiante. Le mélange réactionnel est hydrolysé avec quelques ml d'une solution aqueuse 0,3N d'acide chlorhydrique jusqu'à obtenir un pH d'environ 7. Puis quelques ml d'acétate d'éthyle sont additionnés, et la phase aqueuse est extraite quatre fois avec de l'acétate d'éthyle. Les phases organiques sont réunies, lavées avec une solution aqueuse saturée de chlorure de sodium puis évaporées. Le résidu est lavé, filtré et rincé avec du méthanol permettant d'isoler le produit attendu.
Point de fusion : 167°C (décomposition).
IR(KBr) : $\nu_{C=O}$ = 1715, 1767 $cm^{-1}$; $\nu_{NH}$ = 3304 $cm^{-1}$.
Spectre de masse : 305 $[M + 1]^+$.

***Stade B : 3-Bromo-4-(1H-1-carboxylate de tert-butyle-indol-3-yl)-1-méthyl-1H-pyrrole-2,5-dione***

8,19 mmol du composé obtenu au stade A précédent sont mis en solution à 0°C dans 37,5 ml de tétrahydrofurane anhydre, en présence de 28,68 mmol de di-*tert*-butyldicarbonate, et de 614,5 µmol de 4-diméthylaminopyridine. Le milieu réactionnel est ramené à température ambiante, et agité pendant 2h30. Après évaporation du solvant, le résidu obtenu est repris dans du méthanol, filtré, rincé avec du méthanol, puis séché sous vide, permettant d'isoler le produit attendu.
Point de fusion : 142°C.
IR(KBr) : $\nu_{C=O}$ = 1762 $cm^{-1}$.
Spectre de masse : 405 $[M + 1]^+$.

**PREPARATION F : 3-Bromo-4-(1*H*-indol-3-yl)-1*H*-pyrrole-2,5-dione**

15,69 mmol d'indole sont mis en solution dans 25 ml de tétrahydrofurane anhydre sous atmosphère inerte. 15,69 mmol d'une solution de 3M de bromure d'éthylmagnésium dans de l'éther diéthylique sont additionnées, et le mélange réactionnel est chauffé à 60°C pendant 1h30. Une fois refroidi, ce mélange est additionné goutte à goutte à une solution de 3,92 mmol 2,3-dibromomaléimide dans 6 ml de tétrahydrofurane anhydre, puis le milieu réactionnel est agité à température ambiante pendant une heure. Le mélange réactionnel est hydrolysé avec 20 ml d'une solution aqueuse 0,3N d'acide chlorhydrique jusqu'à obtenir un pH de 8,5, et quelques ml d'acétate d'éthyle sont additionnés. La phase aqueuse est extraite trois fois avec de l'acétate d'éthyle, puis les phases organiques sont réunies, lavées avec une solution aqueuse saturée de chlorure de sodium puis évaporées à sec sous vide. Le résidu est lavé, filtré et rincé avec du méthanol, permettant d'isoler le produit attendu.

Point de fusion : > 300°C.
IR(KBr) : $\nu_{C=O}$ = 1772 $cm^{-1}$ **;** $\nu_{NH}$= 3343, 3699 $cm^{-1}$.
Spectre de masse : 291 $[M + 1]^+$.

**PREPARATION G : 3-Bromo-4-(5-fluoro-1*H*-indol-3-yl)-1-méthyl-1*H*-pyrrole-2,5-dione**

Le produit attendu est obtenu selon le procédé décrit au stade A de la préparation E avec du 5-fluoroindole.
Point de fusion : > 300°C.
IR(KBr) : $\nu_{C=O}$ = 1705 $cm^{-1}$ ; $\nu_{NH}$ = 3358 $cm^{-1}$ .
Spectre de masse : 323 $[M + 1]^+$.

**PREPARATION H : 3-Bromo-4-(6-fluoro-1*H*-indol-3-yl)-1-méthyl-1*H*-pyrrole-2,5-dione**

Le produit attendu est obtenu selon le procédé décrit au stade A de la préparation E avec du 6-fluoroindole.
Point de fusion : 201°C.
IR(KBr) : $\nu_{C=O}$ = 1701, 1767 $cm^{-1}$ ;$\nu_{NH}$ = 3312 $cm^{-1}$.
Spectre de masse : 323$[M+1]^+$.

**PREPARATION I : 3-Bromo-4-(5-fluoro-1*H*-indol-3-yl)-1*H*-pyrrole-2,5-dione**

Le produit attendu est obtenu selon le procédé décrit dans la préparation F avec du 5-fluoroindole.
Point de fusion : 219°C (décomposition).
IR(KBr) : $\nu_{C=O}$ = 1723, 1769 $cm^{-1}$; $\nu_{NH}$ = 3358 $cm^{-1}$.
Spectre de masse : 309,5 $[M + 1]^+$.

**PREPARATION J : 3-Bromo-4-(6-fluoro-1*H*-indol-3-yl)-1*H*-pyrrole-2,5-dione**

Le produit attendu est obtenu selon le procédé décrit dans la préparation F avec du 6-fluoroindole.
Point de fusion : 199°C (décomposition).
IR(KBr) : $\nu_{C=O}$ = 1723, 1778 $cm^{-1}$ ; $\nu_{NH}$ = 3329 $cm^{-1}$.
Spectre de masse : 309 $[M + 1]^+$.

**PREPARATION K : 5-(Benzyloxy)-3-(4-bromo-1-méthyl-2,5-dioxo-2,5-dibydro-1*H*-pyrrol-3-yl)-1*H*-indole-1-carboxylate de tert-butyle**

***Stade A: 3-[5-(Benzyloxy)-1H-indol-3-yl]-4-bromo-1-méthyl-1H-pyrrole-2,5-dione***

Le produit attendu est obtenu selon le procédé décrit au stade A de la préparation E avec du 5-benzyloxyindole.
Point de fusion : 150°C (décomposition).
IR(KBr) : $\nu_{C=O}$ = 1698 $cm^{-1}$ ; $\nu_{NH}$ = 3315 $cm^{-1}$.
Spectre de masse : 411 $[M + 1]^+$.

***Stade B : 5-(Benzyloxy)-3-(4-bromo-1-méthyl-2,5-dioxo-2,5-dihydro-1H-pyrrol-3 yl)-1H-indole-1-carboxylate de tert-butyle***

Le produit attendu est obtenu selon le procédé décrit au stade B de la préparation E à partir du composé du stade A précédent.
Point de fusion : 155°C.
IR(KBr) : $\nu_{C=O}$ = 1709, 1738, 1773 $cm^{-1}$.
Spectre de masse : 511 $[M + 1]^+$.

**PREPARATION L : 6-(Benzyloxy)-3-(4-bromo-1-méthyl-2,5-dioxo-2,5-dihydro-1*H*-pyrrol-3-yl)-1*H*-indole-1-carboxylate de tert-butyle**

***Stade A: 3-[6-(Benzyloxy)-1H-indol-3-yl]-4-bromo-1-méthyl-1H-pyrrole-2,5-dione***

Le produit attendu est obtenu selon le procédé décrit au stade A de la préparation E avec du 6-benzyloxyindole.
Point de fusion : 138°C (décomposition).

IR(KBr) : $\nu_{C=O}$ = 1705, 1762 $cm^{-1}$ ; $\nu_{NH}$ = 3314 $cm^{-1}$.
Spectre de masse : 411 $[M + 1]^+$.

***Stade B** : 6-(Benzyloxy)-3-(4-bromo-1-méthyl-2,5-dioxo-2,5-dihydro-1H-pyrrol-3-yl)-1H-indole-1-carboxylate de tert-butyle*

Le produit attendu est obtenu selon le procédé décrit au stade B de la préparation E à partir du composé du stade A précédent.
Point de fusion : 158°C (décomposition).
IR(KBr): $\nu_{V=O}$ = 1715, 1737, 1745 $cm^{-1}$.
Spectre de masse :511 $[M+1]^+$.

**PREPARATION M : 5-(Benzyloxy)-3-[4-bromo-1-(tert-butoxycarbonyl)-2,5-dioxo-2,5-dihydro-1*H*-pyrrol-3-yl]-1*H*-indole-1-carboxylate de tert-butyle**

***Stade A** : 3-[5-(Benzyloxy)-1H-indol-3 yl]-4-bromo-1H-pyrrole-2,5-dione*

Le produit attendu est obtenu selon le procédé décrit dans la préparation F avec du 5-benzyloxyindole.
Point de fusion : 154°C (décomposition).
IR(KBr) : $\nu_{C=O}$ = 1697 $cm^{-1}$ **;** $\nu_{NH}$= 3333 $cm^{-1}$.
Spectre de masse : 397 $[M + 1]^+$.

***Stade B** : 5-(Benzyloxy)-3-[4-bromo-1-(tert-butoxycarbonyl)-2,5-dioxo-2,5-dihydro-1H-pyrrol-3-yl]-1H-indole-1-carboxylate de tert-butyle*

Le produit attendu est obtenu selon le procédé décrit au stade B de la préparation E à partir du composé du stade A précédent.
Point de fusion : 134°C.
IR(KBr): $\nu_{C=O}$ = 1743, 1768, 1801 $cm^{-1}$.
Spectre de masse : 495 $[M - Boc]^+$.

**PREPARATION N : 6-(Benzyloxy)-3-[4-bromo-1-(tert-butoxycarbonyl)-2,5-dioxo-2,5-dihydro-1*H*-pyrrol-3-yl]-1*H*-indole-1-carboxylate de tert-butyle**

***Stade A:** 3-[6-(Benzyloxy)-1H-indol-3-yl]-4-bromo-1H-pyrrole-2,5-dione*

Le produit attendu est obtenu selon le procédé décrit dans la préparation F avec du 6-benzyloxyindole.
Point de fusion : 166°C (décomposition).
IR(KBr) : $\nu_{C=O}$ = 1697, 1762 $cm^{-1}$ ; $\nu_{NH}$ = 3353 $cm^{-1}$.
Spectre de masse : 397 $[M + 1]^+$.

***Stade B** : 6-(Benzyloxy)-3-[4-bromo-]-(tert-butoxycarbonyl)-2,5-dioxo-2,5-dihydro-1H-pyrrol-3-yl]-1H-indole-1-carboxylate de tert-butyle*

Le produit attendu est obtenu selon le procédé décrit au stade B de la préparation E à partir du composé du stade A précédent.
Point de fusion : 120°C.
IR(KBr) : $\nu_{C=O}$= 1719, 1744, 1764, 1807 $cm^{-1}$.

**PREPARATION O : 7-(Benzyhydryloxy)-3-(4-bromo-1-méthyl-2,5-dioxo-2,5-dibydro-1*H*-pyrrol-3-yl)-1*H*-indole-1-carboxylate de tert-butyle**

***Stade A** : 1-(Benzhydryloxy)-2-nitrobenzène*

A une solution d'ortho-nitrophénol sec (79 mmol) dans 200 ml d'acétone, sont additionnées 127 mmol de carbonate de potassium, ainsi que 79 mmol de bromure de diphénylméthane. Le mélange réactionnel est porté à reflux pendant 6 heures, puis est abaissé à température ambiante et agité encore une nuit. Le milieu réactionnel est filtré et rincé à l'acétone. Le filtrat est évaporé, puis repris avec de l'éther diéthylique et hydrolysé. La phase aqueuse est extraite

avec de l'éther diéthylique, puis la phase organique est séchée sur du sulfate de magnésium, filtrée et concentrée. Le résidu obtenu est repris avec de l'éther de pétrole, filtré, rincé avec de l'éther de pétrole, puis séché sous vide, permettant d'isoler le produit attendu.
Point de fusion : 98°C.
Spectre de masse : 323 $[M + NH_4^+]^+$.

***Stade B* : 7-(Benzhydryloxy)-1H-indole**

11,95 mmol du composé obtenu au stade A précédent sont dissous, sous atmosphère inerte, dans 80 ml de tétrahydrofurane anhydre. La température du milieu réactionnel est alors abaissée à -40°C, puis, 41,84 mmol d'une solution 1M de bromure de vinylmagnésium dans le tétrahydrofurane sont additionnés goutte à goutte, à la solution, qui est ensuite agitée pendant 2h50, de -40°C, à 0°C. Le mélange réactionnel est hydrolysé à 0°C, avec 100 ml d'une solution aqueuse saturée de chlorure d'ammonium, et la phase aqueuse est extraite trois fois avec de l'acétate d'éthyle. Les phases organiques sont réunies, séchées sur du sulfate de magnésium, filtrées, et évaporées. Une purification par chromatographie flash sur gel de silice (éther de pétrole/acétate d'éthyle : 9/1), permet d'isoler le produit attendu.
Point de fusion : 114°C (décomposition).
IR(KBR) : $\nu_{NH}$ = 3425 $cm^{-1}$.
Spectre de masse : 300 $[M + 1]^+$.

***Stade C:* 3-[7-(Benzhydryloxy)-1H-indol-3yl]-4-bromo-1-méthyl-1H-pyrrole-2,5-dione**

Le produit attendu est obtenu selon le procédé décrit au stade A de la préparation E avec des composés du stade B précédent.
Point de fusion : 205°C.
IR(KBr) : $\nu_{C=O}$ = 1699, 1763 $cm^{-1}$ ; $\nu_{NH}$ = 3345 $cm^{-1}$.
Spectre de masse : 487 $[M + 1]^+$.

***Stade D* 7-(Benzyhydryloxy)-3-(4-bromo-1-méthyl-2,5-dioxo-2,5-dihydro-1H-pyrrol-3-yl)-1H indole-1-carboxylate de tert-butyle**

Le produit attendu est obtenu selon le procédé décrit au stade B de la préparation E à partir du composé du stade C précédent.
Point de fusion : 148°C (décomposition).
IR(KBr) : $\nu_{C=O}$ = 1709, 1759 $cm^{-1}$.
Spectre de masse : 587 $[M + 1]^+$.

**EXEMPLE 1 : 7-Méthyl-6,8-dioxo-7,8-dihydro[1,4]benzodioxino[2,3-a]pyrrolo [3,4-c]phénoxazine-15(6*H*)carboxylate de phényle**

***Stade A:* 3-(1,4-Benzodioxin-2-yl)-4H-1,4-benzoxazine-4-carboxylate de phényle**

Sous atmosphère inerte, un mélange de 1 mmol du composé de la préparation C, 2 mmol du composé de la préparation A, 3 mmol de chlorure de lithium et 5% de tétrakis (triphénylphosphine)palladium (0) dans 5 ml de tétrahydrofurane est chauffé à reflux. Après refroidissement et concentration, le résidu est repris dans l'acétate d'éthyle, lavé avec une solution saturée de chlorure de sodium. La phase organique est séchée sur sulfate de magnésium, filtrée et concentrée. Une purification par chromatographie sur gel de silice (éther de pétrole/acétate d'éthyle : 6/4), permet d'isoler le produit attendu.
Point de fusion : 75°C (gomme).
IR(KBr) : $\nu_{C=O}$ = 1739 $cm^{-1}$.
Spectre de masse : 386 $[M + 1]^+$.

***Stade B:* 7-Méthyl-6,8-dioxo-5b,6,7,8,8a,8b-hexahydro[1,4]benzodioxino[2,3-a] pyrrolo[3,4-c]phénoxazine-15 (5aH)-carboxylate de phényle**

Dans un système clos, 1 mmol du composé obtenu au stade A précédent et 3 mmol de *N*-méthylmaléimide sont agités à 95°C pendant 2 heures en présence de quelques gouttes de toluène. Une purification par chromatographie su gel de silice (éther de pétrole/acétate d'éthyle : 3/7), permet d'obtenir le produit attendu.
Point de fusion : > 250°C.

IR(KBr): $\nu_{C=O}$ = 1709, 1769 $cm^{-1}$.
Spectre de masse : 497 $[M + 1]^+$.

***Stade C:* 7-Méthyl-6,8-dioxo-7,8-dihydro[1,4]benzodioxino[2,3-a]pyrrolo[3,4-c] phénoxazine-15(6H)-carboxylate de phényle**

Sous atmosphère inerte, 1 mmol du composé obtenu au stade B précédent et 3 mmol de N-bromosuccinimide recristallisé sont chauffés dans du tétrachlorure de carbone distillé à reflux pendant 7 minutes à l'aide d'une lampe (60 W) en présence d'une quantité catalytique de péroxyde de benzoyle. Après refroidissement, la solution est filtrée et concentrée. Une purification par chromatographie sur gel de silice (éther de pétrole/acétate d'éthyle : 1/1), permet d'obtenir le produit attendu.
Point de fusion : 130°C (gomme).
IR(KBr) : $\nu_{C=O}$ = 1714, 1745 $cm^{-1}$ .
Spectre de masse : 493 $[M + 1]^+$.

**EXEMPLE 2 : 7-[2-(Diméthylamino)éthyl][1,4]benzodioxino[2,3-a]pyrrolo[3,4-c] phénoxazine-6,8(7*H*,15*H*)-dione**

Sous atmosphère inerte, 0,1 mmol du composé de l'exemple 1 dans 2 ml de *N,N*-diméthyléthylène diamine sont chauffés à 100°C pendant 6 heures. Après refroidissement, la solution est concentrée. Une purification par chromatographie sur gel de silice (dichlorométhane/méthanol : 8/2), permet d'obtenir le produit attendu.
Point de fusion : 150°C.
IR(KBr): $\nu_{C=O}$ = 1701, 1751 $cm^{-1}$; $\nu_{NH}$= 3423 $cm^{-1}$.
Spectre de masse : 430 $[M + 1]^+$.

**EXEMPLE 3 : 7-Méthyl-6,8-dioxo-7,8-dihydro[1,4]benzodioxino[2,3-e]pyrido [2',3':5,6][1,4]oxazino[3,2-g]isoindole-15(6*H*)-carboxylate de phényle**

***Stade A : 3-(1,4-Benzodioxin-2 yl)-4H pyrido[3,2-b][1,4]oxazine-4-carboxylate de phényle***

Le produit attendu est obtenu selon le procédé décrit au stade A de l'exemple 1 à partir du composé de la préparation D.
Point de fusion : 142°C.
IR(KBr) : $\nu_{C=O}$ = 1741 $cm^{-1}$.
Spectre de masse : 387 $[M + 1]^+$.

***Stade B: 7-Méthyl-6,8-dioxo-5b,6,7,8,8a,8b-hexahydro[1,4]benzodioxino[2,3-e] pyrido[2',3':5,6][1,4]oxazino [3,2-g]isoindole-15(5aH)-carboxylate de phényle***

Le produit attendu est obtenu selon le procédé décrit au stade B de l'exemple 1 à partir du composé au stade A précédent.
Point de fusion : 245°C.
IR(KBr) : $\nu_{C=O}$ = 1709, 1769 $cm^{-1}$.
Spectre de masse : 498 $[M + 1]^+$.

***Stade C: 7-Méthyl-6,8-dioxo-7,8-dihydro[1,4]benzodioxino[2,3-e]pyrido [2',3':5,6][1,4]oxazino[3,2-g]isoindole-15(6H)-carboxylate de phényle***

Le produit attendu est obtenu selon le procédé décrit au stade C de l'exemple 1 à partir du composé obtenu au stade B précédent.
Point de fusion : > 250°C.
IR(KBr) : $\nu_{C=O}$ = 1711, 1760 $cm^{-1}$.
Spectre de masse : 494 $[M + 1]^+$.

**EXEMPLE 4 : 7-[2-(Diméthylamino)éthyl][1,4]benzodioxino[2,3-e]pyrido [2',3':5,6] [1,4]oxazino[3,2-g]isoindole-6,8(7H,15H)-dione**

Le produit attendu est obtenu selon le procédé de l'exemple 2 à partir du composé de l'exemple 3.

Point de fusion : 150°C (dégradation).
IR(KBr) : $\nu_{C=O}$ = 1703, 1759 $cm^{-1}$ ; $\nu_{NH}$ = 3433 $cm^{-1}$.
Spectre de masse : 431 $[M + 1]^+$.

**EXEMPLE 5 : 7-Méthyl-6,8-dioxo-7,8-dihydro[1,4]benzodioxino[2,3-a]pyrrolo [3,4-c]carbazole-13(6*H*)-carboxylate de tert-butyle**

***Stade A : 3-[4-(1,4-Benzodioxin-2-yl)-1-méthyl-2,5-dioxo-2,5-dihydro-1H-pyrrol-3 yl]-1H-indole-1-carboxylate de tert-butyle***

A une solution de 592,23 µmol du composé de la préparation A dans 10 ml de 1,4-dioxane sont additionnés, 493,52 µmol du composé de la préparation E, 49,35 µmol d'iodure de cuivre, et 49,35 µmol de tétrakistriphénylphosphine de palladium. Le milieu réactionnel est chauffé à 100°C, sous atmosphère inerte, pendant 3h35. La solution est ensuite filtrée pour éliminer les restes de palladium, et le filtrat est évaporé. Une purification par chromatographie flash sur gel de silice (éther de pétrole/acétate d'éthyle/triéthylamine : 8/1, 9/0,1), permet d'obtenir le produit attendu.
Point de fusion : 140°C.
IR KBr : $\nu_{C=O}$ =1704, 1740 $cm^{-1}$ .
Spectre de masse : 459 $[M + 1]^+$.

***Stade B: 7-Méthyl-6,8-dioxo-7,8-dihydro[1,4]benzodioxino[2,3-a]pyrrolo[3,4-c] carbazole-13(6H)-carboxylate de tert-butyle***

218,1 µmol du composé obtenu au stade A précédent et 5,91 mmol d'iode bisublimé, sont mis en solution dans 500 ml de toluène, à l'intérieur de la cuve de l'appareil à irradier. Après 30 minutes d'irradiation, sous agitation, le milieu réactionnel est refroidi, puis dilué avec de l'acétate d'éthyle. Un lavage avec 80 ml d'une solution aqueuse à 20 % de thiosulfate de sodium est effectué, jusqu'à décoloration de la phase organique. La phase aqueuse est extraite trois fois avec de l'acétate d'éthyle, puis les phases organiques sont réunies, et directement concentrées sous vide. Un résidu mi-huileux, mi-solide marron est recueilli. De la même manière, et avec les mêmes quantités, la procédure est répétée encore cinq fois. Les bruts réactionnels des six essais sont réunis. Un lavage avec de l'acétate d'éthyle est effectué sur les six bruts réactionnels, qui sont ensuite filtrés. Le filtrat est évaporé, puis repris dans du méthanol, filtré, rincé avec du méthanol, puis séché sous vide permettant d'isoler le produit attendu.
Point de fusion : 189°C (décomposition).
Spectre de masse : 457 $[M + 1]^+$.

**EXEMPLE 6 : 7-Méthyl[1,4]benzodioxino[2,3-a]pyrrolo[3,4-c]carbazole-6,8 (7*H*,13*H*)-dione**

***Stade A : 3-(1,4-Benzodioxin-2-yl)-4-(1H-indol-3yl)-1-méthyl-1H-pyrrole-2,5-dione***

331,54 µmol du composé du stade A de l'exemple 5 sont mis en solution dans 9 ml d'acide formique. Le mélange réactionnel est agité sous atmosphère inerte et à température ambiante pendant 3h30. Le solvant est éliminé sous pression réduite, puis le résidu est lavé, filtré, et rincé avec du méthanol, puis séché sous vide, permettant d'isoler le produit attendu.
Point de fusion : 219°C.
IR(KBr): $\nu_{C=O}$ = 1697 $cm^{-1}$.
Spectre de masse : 359,5 $[M + 1]^+$.

***Stade B : 7-Méthyl[1,4]benzodioxino[2,3-a]pyrrolo[3,4-c]carbazole-6,8(7H,13H)-dione***

Dans un réacteur à irradiation 418,5 µmol du composé obtenu au stade A précédent sont mis en solution dans 50 ml de toluène, puis 5,91 mmol d'iode bisublimé sont introduits dans le milieu réactionnel. L'irradiation est réalisée à 500 W pendant 1h10. Le mélange réactionnel est ensuite dilué avec de l'acétate d'éthyle, puis lavé avec 80 ml d'une solution aqueuse de thiosulfate de sodium à 20 %, jusqu'à décoloration de la phase organique. La phase aqueuse est extraite trois fois avec de l'acétate d'éthyle, puis les phases organiques sont réunies, et directement concentrées. Le résidu solide obtenu, est lavé dans un premier temps avec de l'éther diéthylique, puis filtré. Dans un second temps, il est lavé avec de l'acétate d'éthyle, puis du tétrahydrofurane, et filtré. Une purification par chromatographie flash sur gel de silice (éther de pétrole/acétate d'éthyle : 6/4), permet d'obtenir le produit attendu.
Point de fusion : > 355°C.
IR(KBr) : $\nu_{C=O}$ = 1692, 1703 $cm^{-1}$ **;** $\nu_{NH}$ = 3343 $cm^{-1}$ .

Spectre de masse : 357 $[M + 1]^+$.

**EXEMPLE 7 : 7-[2-(Diméthylamino)éthyl][1,4]benzodioxino[2,3-a]pyrrolo[3,4-c] carbazole-6,8(7*H*,13*H*)-dione**

238,5 μmol du composé de l'exemple 6 sont mis en solution dans 4 ml de *N,N*-diméthyléthylènediamine. Le milieu réactionnel est porté à reflux pendant 16 heures, puis il est refroidi, et concentré. Le résidu obtenu est lavé, filtré et rincé avec du méthanol, avant d'être séché sous vide, permettant d'isoler le produit attendu.
Point de fusion : 298°C (décomposition).
IR(KBr) : $\nu_{C=O}$ = 1703, 1753 $cm^{-1}$; $\nu_{NH}$ = 3441 $cm^{-1}$.
Spectre de masse : 414 $[M + 1]^+$.

**EXEMPLE 8 : 7,13-Diméthyl[1,4]benzodioxino[2,3-a]pyrrolo[3,4-c]carbazole-6,8 (7H,13H)-dione**

***Stade A: 3-(1,4-Benzodioxin-2-yl)-1-méthyl-4-(1-méthyl-1H-indol-3-yl)-1H-pyrrole-2,5-dione***

69,76 μmol du composé obtenu au stade A de l'exemple 6, 111,62 μmol de soude, 7,46 μmol de chlorure de benzyltriéthylammonium, et 418,58 mmol d'iodure de méthyle, sont mis en solution dans 0,5 ml de dichlorométhane. Le milieu réactionnel est agité à température ambiante pendant 1h45, puis le solvant est éliminé sous vide. Une purification par chromatographie flash sur gel de silice (éther de pétrole/acétate d'éthyle : 7/3 à acétate d'éthyle), permet d'obtenir le produit attendu.
Point de fusion : 204°C (décomposition).
IR(KBr) : $\nu_{C=O}$ = 1697 $cm^{-1}$.
Spectre de masse : 373 $[M + 1]^+$.

***Stade B : 7,13-Diméthyl[1,4]benzodioxino[2,3-a]pyrrolo[3,4-c]carbazole-6,8 (7H,13H)-dione***

Le produit attendu est obtenu selon le procédé décrit au stade B de l'exemple 5 à partir des composés obtenu au stade A précédent.

**EXEMPLE 9 : 13-Ethyl-7-méthyl[1,4]benzodioxino[2,3-a]pyrrolo[3,4-c]carbazole-6,8(7*H*,13*H*)-dione**

***Stade A: 3-(1,4-Benzodioxin-2-yl)-4-(1-éthyl-1H-indol-3-yl)-1-méthyl-1H-pyrrole-2,5-dione***

Le produit attendu est obtenu selon le procédé décrit dans le stade A de l'exemple 8 à partir du composé obtenu au stade A de l'exemple 6 en remplaçant l'iodure de méthyle par de l'iodure d'éthyle.

***Stade B: 13-Ethyl-7-méthyl[1,4]benzodioxino[2,3-a]pyrrolo[3,4-c]carbazole-6,8(7H,13H)dione***

Le produit attendu est obtenu selon le procédé décrit au stade B de l'exemple 6 à partir du composé obtenu au stade A précédent.

**EXEMPLE 10 : Naphto[2',3':5,6][1,4]dioxino[2,3-a]pyrrolo[3,4-c]carbazole-5,7 (6*H*,16*H*)-dione**

***Stade A : 3-(1H-Indol-3-yl)-4-naphto[2,3-b][1,4]dioxin-2-yl-1H-pyrrole-2,5-dione***

Le produit attendu est obtenu selon le procédé décrit au stade A de l'exemple 5 à partir des composés des préparations B et F.
Point de fusion 261°C.
IR(KBr) : $\nu_{C=O}$ = 1759 $cm^{-1}$ ; $\nu_{NH}$ = 3171, 3398 $cm^{-1}$.
Spectre de masse : 395 $[M + 1]^+$.

***Stade B: Naphto[2',3':5,6][1,4]dioxino[2,3-a]pyrrolo[3,4-c]carbazole-5,7 (6H,16H)-dione***

Le produit attendu est obtenu selon le procédé décrit au stade B de l'exemple 6 à partir du composé obtenu au stade précédent.

**<u>EXEMPLE 11</u> : 10-Fluoro-7-méthyl[1,4]benzodioxino[2,3-a]pyrrolo[3,4-c] carbozole-6,8(7*H*,13*H*)-dione**

***<u>Stade A:</u> 3-(1,4-Benzodioxin-2 yl)-4-(5-fluoro-1H-indol-3-yl)-1-méthyl-1H-pyrrole-2,5-dione***

Le produit attendu est obtenu selon le procédé décrit au stade A de l'exemple 5 à partir du composé de la préparation G.
<u>Point de fusion</u> : 229°C.
<u>IR(KBr)</u> : $\nu_{C=O}$ = 1698 $cm^{-1}$ ; $\nu_{NH}$ = 3380 $cm^{-1}$.
<u>Spectre de masse :</u> 377 $[M + 1]^+$.

***<u>Stade B:</u> 10-Fluoro-7-méthyl[1,4]benzodioxino[2,3-a]pyrrolo[3,4-c]carbazole-6,8(7H,13H)-dione***

Le produit attendu est obtenu selon le procédé décrit au stade B de l'exemple 6 à partir du composé obtenu au stade A précédent.
<u>Point de fusion:</u> > 355°C.
<u>IR(KBr)</u> : $\nu_{C=O}$ = 1699, 1753 $cm^{-1}$ ; $\nu_{NH}$ = 3339 $cm^{-1}$.
<u>Spectre de masse :</u> 375 $[M + 1]^+$.

**<u>EXEMPLE 12 :</u> 7-[2-(Diméthylamino)éthyl]-10-fluoro[1,4]benzodioxino[2,3-a] pyrrolo[3,4-c]carbazole-6,8(7*H*, 13*H*)-dione**

Le produit attendu est obtenu selon le procédé décrit dans l'exemple 7 à partir du composé de l'exemple 11.
<u>Point de fusion</u> : 337°C (décomposition).
<u>IR(KBr)</u> : $\nu_{C=O}$= 1701, 1755 $cm^{-1}$ ; $\nu_{NH}$ = 3447 $cm^{-1}$.
<u>Spectre de masse :</u> 432 $[M + 1]^+$.

**<u>EXEMPLE 13 :</u> 11 -Fluoro-7-méthyl[1,4]benzodioxino[2,3-a]pyrrolo[3,4-e]carbazole -6,8(7*H*,13*H*)-dione**

***<u>Stade A</u> : 3-(1,4-Benzodioxin-2-yl)-4-(6-fluoro-1H-indol-3-yl)-1-méthyl-1H-pyrrole-2,5-dione***

Le produit attendu est obtenu selon le procédé décrit au stade A de l'exempe 5 à partir du composé de la préparation H.
<u>Point de fusion :</u> 214°C.
<u>IR(KBr)</u> : $\nu_{C=O}$ = 1693 $cm^{-1}$ ; $\nu_{NH}$ = 3322 $cm^{-1}$.
<u>Spectre de masse :</u> 377 $[M +1]^+$.

***<u>Stade B</u> : 11-Fluoro-7-méthyl[1,4]benzodioxino[2,3-a]pyrrolo[3,4-c]carbazole-6,8 (7H,13H)-dione***

Le produit attendu est obtenu selon le procédé décrit au stade B de l'exemple 6 à partir du composé obtenu au stade A précédent.
<u>Point de fusion</u> : > 360°C.
<u>IR(KBr)</u> : $\nu_{C=O}$ = 1697, 1752 $cm^{-1}$ ; $\nu_{NH}$ = 3349 $cm^{-1}$ .
<u>Spectre de masse :</u> 375 $[M + 1]^+$.

**<u>EXEMPLE 14 :</u> 7-[2-(Diméthylamino)éthyl]-11-fluoro[1,4]benzodioxino[2,3-a] pyrrolo[3,4-c]carbazole-6,8(7*H*, 13*H*)-dione**

Le produit attendu est obtenu selon le procédé décrit dans l'exemple 7 à partir du composé de l'exemple 13.
<u>Point de fusion :</u> 301°C (décomposition).
<u>IR(KBr)</u> : $\nu_{C=O}$ = 1703, 1752 $cm^{-1}$ ; $\nu_{NH}$ = 3439 $cm^{-1}$.
<u>Spectre de masse</u> : $432[M+1]^+$.

**<u>EXEMPLE 15 :</u> 10-Fluoro[1,4]benzodioxino[2,3-a]pyrrolo[3,4-c]carbazole-6,8 (7*H*,13*H*)-dione**

***<u>Stade A</u> : 3-(1,4-Benzodioxin-2 yl)-4-(5-fluoro-1H-indol-3-yl)-1H-pyrrole-2,5-dione***

Le produit attendu est obtenu selon le procédé décrit au stade A de l'exemple 5 à partir du composé de la préparation I.

Point de fusion : 241°C (décomposition).
IR(KBr) : $\nu_{C=O}$ = 1693 $cm^{-1}$ ; $\nu_{NH}$ = 3177, 3335 $cm^{-1}$.
Spectre de masse : 363 $[M + 1]^+$.

***Stade B:* 10-Fluoro[1,4]benzodioxino[2,3-a]pyrrolo[3,4-c]carbazole-6,8(7H,13H)-dione**

Le produit attendu est obtenu selon le procédé décrit au stade B de l'exemple 6 à partir du composé obtenu au stade A précédent.
Point de fusion : > 360°C.
IR(KBr) : $\nu_{C=O}$= 1707 $cm^{-1}$ ; $\nu_{NH}$ = 3258, 3379 $cm^{-1}$.
Spectre de masse : 361 $[M + 1]^+$.

**EXEMPLE 16 : 11-Fluoro[1,4]benzodioxino[2,3-a]pyrrolo[3,4-c]carbazole-6,8 (7*H*,13*H*)-dione**

***Stade A :* 3-(1,4-Benzodioxin-2-yl)-4-(6-fluoro-1H-indol-3-yl)-1H-pyrrole-2,5-dione**

Le produit attendu est obtenu selon le procédé décrit au stade A de l'exemple 5 à partir du composé de la préparation J.
Point de fusion : 203°C.
IR(KBr): $\nu_{C=O}$ = 1697, 1762 $cm^{-1}$ ; $\nu_{NH}$ = 3179, 3344 $cm^{-1}$.
Spectre de masse : 363 $[M + 1]^+$.

***Stade B*: *11-Fluoro[1,4]benzodioxino[2,3-a]pyrrolo[3,4-c]carbazole-6,8(7H,13H)-dione***

Le produit attendu est obtenu selon le procédé décrit au stade B de l'exemple 6 à partir du composé obtenu au stade A précédent.
Point de fusion : > 360°C.
IR(KBr): $\nu_{C=O}$ = 1710 $cm^{-1}$ ; $\nu_{NH}$ = 3190, 3455 $cm^{-1}$.
Spectre de masse : 361 $[M + 1]^+$.

**EXEMPLE 17** : **10-(Benzyloxy)-7-méthyl-6,8-dioxo-7,8-dihydro[1,4]benzodioxino [2,3-a]pyrrolo[3,4-c]carbazole-13(6*H*)-carboxylate de tert-butyle**

***Stade A:* 3-[4-(1,4-Benzodioxin-2-yl)-1-méthyl-2,5-dioxo-2,5-dihydro-1H-pyrrol-3-yl]-5-(benzyloxy)-1H-indole-1-carboxylate de tert-butyle**

Le produit attendu est obtenu selon le procédé décrit au stade A de l'exemple 5 à partir du composé de la préparation K.
Point de fusion : 93°C.
IR(KBr) : $\nu_{C=O}$= 1703, 1706, 1737, 1741 $cm^{-1}$.
Spectre de masse : 565,5 $[M + 1]^+$.

***Stade B*: *10-(Benzyloxy)-7-méthyl-6,8-dioxo-7,8-dihydro(1,4)benzodioxino [2,3-a]pyrrolo[3,4-c]carbazole-13 (6H)-carboxylate de tert-butyle***

177,1 µmol du composé obtenu au stade A précédent, et 5,91 mmol d'iode bisublimé, sont mis en solution dans 500 ml de toluène, à l'intérieur de la cuve de l'appareil à irradier. Après 40 minutes d'irradiation à 500 W, sous agitation, le milieu réactionnel est refroidi, puis dilué avec de l'acétate d'éthyle. Un lavage avec 80 ml d'une solution aqueuse à 20% de thiosulfate de sodium est effectué, jusqu'à décoloration de la phase organique. La phase aqueuse est extraite trois fois avec de l'acétate d'éthyle, puis les phases organiques sont réunies, et directement concentrées sous vide. De la même manière, et dans les mêmes quantités, la procédure est répétée encore deux fois. Les bruts réactionnels des trois essais sont réunis, et le solide marron obtenu est lavé, filtré et rincé avec du méthanol, avant d'être séché sous vide, permettant d'isoler le produit attendu.
Point de fusion : 302°C (décomposition).
IR(KBr) : $\nu_{C=O}$ = 1705, 1731, 1760 $cm^{-1}$.
Spectre de masse : 463 $[M\text{-}Boc + 1]^+$.

**EXEMPLE 18 : 10-Hydroxy-7-méthyl[1,4]benzodioxino[2,3-a]pyrrolo[3,4-c] carbazole-6,8(7*H*,13*H*)-dione**

44,4 μmol du composé de l'exemple 17 sont dissous, sous atmosphère inerte, dans 3,5 ml de dichlorométhane anhydre. Une solution 1M de tribromure de bore dans le dichlorométhane (89 μmol) est ensuite additionnée à la solution refroidie à 0°C, goutte à goutte, et sous une vive agitation. Le milieu réactionnel est agité à 0°C pendant 15 minutes, puis 1h15 en, le laissant revenir à température ambiante. Après hydrolyse avec de l'eau distillée, le milieu réactionnel est dilué avec de l'acétate d'éthyle, et la phase aqueuse extraite trois fois avec de l'acétate d'éthyle. Les phases organiques sont réunies, et évaporées à sec. Le résidu obtenu est lavé, filtré, et rincé avec du méthanol, puis séché sous vide, permettant d'isoler le produit attendu.
Point de fusion : > 350°C.
IR (KBr) : $\nu_{C=O}$ =1691, 1749cm$^{-1}$ ; $\nu_{OH,NH}$= 3345 cm$^{-1}$.
Spectre de masse : 373 [M + 1]$^+$.

**EXEMPLE 19 : 10-(Benzyloxy)-7-[2-(diméthylamino)éthyl][1,4]benzodioxino[2,3-a] pyrrolo[3,4-c]carbazole-6,8 (7*H*,13*H*)-dione**

Le produit attendu est obtenu selon le procédé décrit l'exemple 7 à partir du composé de l'exemple 17.
Point de fusion : 254°C (décomposition).
IR(KBr): $\nu_{C=O}$ = 1701, 1751 cm$^{-1}$ ; $\nu_{NH}$ = 3433 cm$^{-1}$.
Spectre de masse : 520,5 [M + 1]$^+$.

**EXEMPLE 20 : 7-[2-(Diméthylamino)éthyl]-10-hydroxy[1,4]benzodioxino[2,3-a] pyrrolo[3,4-c]carbazole-6,8(7*H*, 13*H*)-dione**

Le produit attendu est obtenu selon le procédé décrit l'exemple 18 à partir du composé de l'exemple 19.
Point de fusion : > 360°C.
IR(KBr) : $\nu_{C=O}$ = 1697 cm$^{-1}$; $\nu_{OH,NH}$ = 3447 cm$^{-1}$.
Spectre de masse : 430 [M + 1]$^+$.

**EXEMPLE 21 : 11-(Benzyloxy)-7-méthyl-6,8-dioxo-7,8-dihydro[1,4]benzodioxino [2,3-a]pyrrolo[3,4-c]carbazole-13(6*H*)-carboxylate de tert-butyle**

***Stade A: 3-[4-(1,4-Benzodioxin-2-yl)-1-méthyl-2,5-dioxo-2,5-dihydro-1H-pyrrol-3-yl]-6-(benzyloxy)-1H-indole-1-carboxylate de tert-butyle***

Le produit attendu est obtenu selon le procédé décrit au stade A de l'exemple 5 à partir du composé de la préparation L.
Point de fusion : 88°C.
IR(KBr) : $\nu_{C=O}$ = 1703, 1737 cm$^{-1}$.
Spectre de masse : 565 [M + 1]$^+$.

***Stade B: 11-(Benzyloxy)-7-méthyl-6,8-dioxo-7,8-dihydro[1,4]benzodioxino[2,3-a] pyrrolo[3,4-c]carbazole-13 (6H)-carboxylate de tert-butyle***

Le produit attendu est obtenu selon le procédé décrit au stade B de l'exemple 17 à partir du composé obtenu au stade A précédent.
Point de fusion : 204°C.
IR(KBr) : $\nu_{C=O}$ = 1703, 1747 cm$^{-1}$.
Spectre de masse : 463 [M-Boc + 1]$^+$.

**EXEMPLE 22 : 11-Hydroxy-7-méthyl[1,4]benzodioxino[2,3-a]pyrrolo[3,4-c] carbazole-6,8(7H,13H)-dione**

Le produit attendu est obtenu selon le procédé décrit au stade B de l'exemple 18 à partir du composé de l'exemple 21.
Point de fusion : 360°C.
IR(KBr) : $\nu_{C=O}$ = 1684, 1696, 1737 cm$^{-1}$ ; $\nu_{OH,NH}$ = 3317, 3442 cm$^{-1}$.
Spectre de masse : 373 [M + 1]$^+$.

**<u>EXEMPLE 23</u> : 11-(Benzyloxy)-7-[2-(diméthylamino)ethyl][1,4]benzodioxino[2,3-a] pyrrolo[3,4-c]carbazole-6,8 (7*H*,13*H*)-dione**

Le produit attendu est obtenu selon le procédé décrit dans l'exemple 7 à partir du composé de l'exemple 21. <u>Point de fusion :</u> 262°C (décomposition). <u>IR(KBr)</u> : $\nu_{C=O}$ = 1701, 1750 $cm^{-1}$; $\nu_{OH,NH}$= 3432 $cm^{-1}$. <u>Spectre de masse :</u> 520,5 $[M + 1]^+$.

**<u>EXEMPLE 24 :</u> 7-[2-(Diméthylamino)éthyl]-11-hydroxy[1,4]benzodioxino[2,3-a] pyrrolo[3,4-c]carbazole-6,8(7*H*, 13*H*)-dione**

Le produit attendu est obtenu selon le procédé décrit dans l'exemple 18 à partir du composé de l'exemple 23. <u>Point de fusion :</u> > 360°C. <u>IR(KHr)</u> : $\nu_{C=O}$ = 1686 $cm^{-1}$ ; $\nu_{OH,NH}$ = 3405 $cm^{-1}$. <u>Spectre de masse :</u> 430 $[M + 1]^+$.

**<u>EXEMPLE 25 :</u> 10-(Benzyloxy)-6,8-dioxo-7,8-dihydro[1,4]benzodioxino[2,3-a] pyrrolo[3,4-c]carbazole-13 (6*H*)-carboxylate de tert-butyle**

***<u>Stade A</u> : 3-[4-(1,4-Benzodioxin-2-yl)-1-(tert-butoxycarbonyl)-2,5-dioxo-2,5-dihydro-1H-pyrrol-3 yl]-5-(benzyloxy)-1H-indole-1-carboxylate de tert-butyle***

Le produit attendu est obtenu selon le procédé décrit au stade A de l'exemple 5 à partir du composé de la préparation M. <u>Point de fusion :</u> 76°C. <u>IR(KBr)</u> : $\nu_{C=O}$ = 1741, 1763, 1797 $cm^{-1}$. <u>Spectre de masse :</u> 551,5 $[M\text{-}Boc + 1]^+$.

***<u>Stade B</u> : 10-(Benzyloxy)-6,8-dioxo-7,8-dihydro[1,4]benzodioxino[2,3-a] pyrrolo[3,4-c]carbazole-13(6H)-carboxylate de tert-butyle***

Le produit attendu est obtenu selon le procédé décrit au stade B de l'exemple 6 à partir du composé obtenu au stade A précédent. <u>Point de fusion :</u> > 304°C. <u>IR(KBr)</u> : $\nu_{C=O}$ = 1719, 1731 $cm^{-1}$; $\nu_{NH}$ = 3198 $cm^{-1}$. <u>Spectre de masse</u> : 549,5 $[M+1]^+$.

**<u>EXEMPLE 26</u> : 10-Hydroxy[1,4]benzodioxino[2,3-a]pyrrolo[3,4-c]carbazole-6,8 (7*H*,13*H*)-dione**

Le produit attendu est obtenu selon le procédé décrit dans l'exemple 8 à partir du composé de l'exemple 25. <u>Point de fusion :</u> > 360°C. <u>IR(KBr)</u> : $\nu_{C=O}$ = 1712, 1750 $cm^{-1}$; $\nu_{NH,OH}$ = 3061, 3218, 3442 $cm^{-1}$. <u>Spectre de masse :</u> 359 $[M + 1]^+$.

**<u>EXEMPLE 27 :</u> 11-(Benzyloxy)-6,8-dioxo-7,8-dihydro[1,4]benzodioxino[2,3-a] pyrrolo[3,4-c]carbazole-13 (6*H*)-carboxylate de tert-butyle**

***<u>Stade A:</u> 3-[4-(1,4-Benzodioxin-2 yl)-1-(tert-butoxycarbonyl)-2,5-dioxo-2,5-dihydro-1H-pyrrol-3-yl]-6-(benzyloxy)-1H-indole-1-carboxylate de tert-butyle***

Le produit attendu est obtenu selon le procédé décrit dans le stade A de l'exemple 5 à partir du composé de la préparation N. <u>Point de fusion</u> : 81°C. <u>IR(KBr)</u> : $\nu_{C=O}$= 1716, 1739, 1762 $cm^{-1}$. <u>Spectre de masse :</u> 551,5 $[M\text{-}Boc + 1]^+$.

***Stade B*: 11-(Benzyloxy)-6,8-dioxo-7,8-dihydro[1,4]benzodioxino[2,3-a]pyrrolo[3,4-c] carbazole-13(6H)-carboxylate de tert-butyle**

Le produit attendu est obtenu selon le procédé décrit au stade B de l'exemple 6 à partir du composé obtenu au stade A précédent.
Point de fusion : 199°C.
IR (KBr) : $\nu_{C=O}$ = 1731, 1761 $cm^{-1}$; $\nu_{NH}$ = 3384 $cm^{-1}$.
Spectre de masse : 549,5 $[M + 1]^+$.

**EXEMPLE 28 : 11-Hydroxy[1,4]benzodioxino[2,3-a]pyrrolo[3,4-c]carbazole-6,8(7*H*,13*H*)-dione**

Le produit attendu est obtenu selon le procédé décrit dans l'exemple 8 à partir du composé de l'exemple 27.
Point de fusion : > 340°C.
IR(KBr): $\nu_{C=O}$ = 1711, 1745 $cm^{-1}$; $\nu_{OH,NH}$ = 3054, 3197, 3427 $cm^{-1}$.
Spectre de masse : 359 $[M + 1]^+$.

**EXEMPLE 29 : 6-Méthyl-5,7-dioxo-6,7-dihydronaphtho[2',3':5,6][1,4]dioxino [2,3-a] pyrrolo[3,4-c]carbazole-16 (5*H*)-carboxylate de tert-butyle**

***Stade A* : 3-(1-Méthyl-4-naphtho[2,3-b][1,4]dioxin-2-yl-2,5-dioxo-2,5-dihydro-1H-pyrrol-3 yl)-1H-indole-1-carboxylate de tert-butyle**

Le produit attendu est obtenu selon le procédé décrit au stade A de l'exemple 5 à partir du composé de la préparation B.
Point de fusion : 158°C.
IR(KBr) : $\nu_{C=O}$= 1702, 1736 $cm^{-1}$.
Spectre de masse : 409 $[M\text{-}Boc + 1]^+$.

***Stade B* : 6-Méthyl-5,7-dioxo-6,7-dihydronaphtho[2',3':5,6][1,4]dioxino[2,3-a] pyrrolo[3,4-c]carbazole-16 (5H)-carboxylate de tert-butyle**

Le produit attendu est obtenu selon le procédé décrit au stade B de l'exemple 5 à partir du composé obtenu au stade A précédent.

**EXEMPLE 30 : 8-(Benzyloxy)-4-(2hydroxyphénoxy)-1,3-dioxo-2,3-dihydropyrrolo [3,4-c]carbazole-6(1*H*)-carboxylate de tert-butyle**

Le produit attendu est obtenu lors de la purification du composé du stade B de l'exemple 27.
Point de fusion : 198°C.
IR(KBr) :$\nu_{C=O}$ = 1725, 1757 $cm^{-1}$ ; $\nu_{OH,NH}$ = 3277 $cm^{-1}$.
Spectre de masse : 551,5 $[M + 1]^+$.

**EXEMPLE 31 : 9-(Benzyloxy)-4-(2-hydroxyphénoxy)-2-méthyl-1,3-dioxo-2,3-dihydropyrrolo[3,4-c]carbazole-6 (1*H*)-carboxylate de tert-butyle**

Dans un réacteur à irradiation, 177,1 μmol du composé obtenu au stade A de l'exemple 17 sont mis en solution dans 500 ml de toluène, puis 5,91 mmol d'iode bisublimé sont introduits dans le milieu réactionnel. L'irradiation est réalisée à 500 W pendant 40 minutes. Le mélange réactionnel est ensuite dilué avec de l'acétate d'éthyle, puis lavé avec 80 ml d'une solution aqueuse de thiosulfate de sodium à 20 %, jusqu'à décoloration de la phase organique. La phase aqueuse est extraite trois fois à l'acétate d'éthyle, puis les phases organiques sont réunies, et directement concentrées à sec sous vide. De la même manière, et dans les mêmes quantités, la réaction est encore exécutée trois autres fois. Les bruts réactionnels des quatre essais sont réunis. Le résidu obtenu est lavé, filtré et rincé avec du méthanol puis évaporé. Une purification par chromatographie flash sur gel de silice (éther de pétrole/acétate d'éthyle : 4/6), permet d'isoler le produit attendu.
Point de fusion : 170°C.
IR(KBr) : $\nu_{C=O}$ = 1700, 1727, 1759 $cm^{-1}$ ; $\nu_{OH}$= 3425 $cm^{-1}$.
Spectre de masse : 565,0 $[M + 1]^+$.

**EXEMPLE 32 : 9-Hydroxy-4-(2-hydroxyphénoxy)-2-méthylpyrrolo[3,4-c] carbazole-1,3(2*H*,6*H*)-dione**

Le produit attendu est obtenu selon le procédé de l'exemple 18 à partir du composé de l'exemple 31.
Point de fusion : 277°C (décomposition).
IR(KBr) : $\nu_{C=O}$ = 1698, 1757 $cm^{-1}$; $V_{OH,NH}$ = 3366 $cm^{-1}$ .
Spectre de masse : 375 $[M + 1]^+$.

**EXEMPLE 33 : 4-(2-Hydroxyphénoxy)-2-méthyl-1,3-dioxo-2,3-dihydropyrrolo [3,4-c]carbazole-6(1*H*)-carboxylate de tert-butyle**

A une solution de 740,2 μmol du composé de la préparation A dans 12,5 ml de 1,4-dioxane sont additionnés, 616,9 μmol du composé de la préparation E, 61,7 μmol d'iodure de cuivre, et 61,7 μmol de tétrakis triphénylphosphine de palladium. Le milieu réactionnel est chauffé à 100°C, sous atmosphère inerte, pendant 21 heures. La solution est ensuite filtrée pour éliminer les restes de palladium, et le filtrat est évaporé à sec. Une purification par chromatographie flash sur gel de silice (éther de pétrole/acétate d'éthyle : 7/3), permet d'obtenir le produit attendu.
Point de fusion : 182°C (décomposition).
IR(KBr) : $\nu_{C=O}$ = 1697, 1730, 1757 $cm^{-1}$ ; $\nu_{OH}$= 3432 $cm^{-1}$.
Spectre de masse : 459 $[M + 1]^+$.

**EXEMPLE 34 : 4-(2-Méthoxyphénoxy)-2-méthyl-l,3-dioxo-2,3-dihydropyrrolo [3,4-c]carbazole-6(1*H*)-carboxylate de tert-butyle**

A une solution de 152,68 μmol du composé de l'exemple 33 dans 5 ml d'acétone, sont additionnés 458,05 μmol de carbonate de potassium sec, et 763,41 μmol d'iodure de méthyle. Le milieu réactionnel est porté à reflux et agité pendant 2h25. Le solvant est ensuite éliminé sous vide, et le résidu recueilli est lavé, filtré, et rincé avec du méthanol, avant d'être séché sous vide, permettant d'isoler le produit attendu.
Point de fusion: > 333°C.
IR(KBr) : $\nu_{C=O}$ = 1705, 1724 $cm^{-1}$.
Spectre de masse : 473,5 $[M + 1]^+$.

**EXEMPLE 35 : 4-(2-Méthoxyphénoxy)-2-méthylpyrrolo[3,4-c]carbazole-1,3(2*H*,6*H*)-dione**

84,6 μmol du composé de l'exemple 34 sont mis en solution dans 2 ml d'acide trifluoroacétique, puis le mélange réactionnel est agité à température ambiante pendant 3 heures. L'acide est éliminé sous vide, puis le résidu est lavé, filtré et rincé avec du méthanol, avant d'être séché sous vide, permettant d'isoler le produit attendu.
Point de fusion : 228°C.
IR(KBr): $\nu_{C=O}$= 1698, 1753 $cm^{-1}$ ; $\nu_{NH}$= 3341, 3643 $cm^{-1}$.
Spectre de masse : 373 $[M + 1]^+$.

**EXEMPLE 36 : 4-(2-Hydroxyphénoxy)-2-méthylpyrrolo[3,4-c]carbazole-1,3 (2*H*,6*H*)-dione**

261,7 μmol du composé de l'exemple 33 sont mis en solution dans 7 ml d'acide formique, puis le mélange réactionnel est agité à température ambiante pendant 18 heures. L'acide est éliminé sous vide, puis le résidu est lavé, filtré et rincé avec du méthanol, avant d'être séché sous vide, permettant d'isoler le produit attendu.
Point de fusion : 258°C.
IR(KBr) : $\nu_{C=O}$ = 1695, 1757 $cm^{-1}$ ; $\nu_{OH,NH}$ = 3243, 3525 $cm^{-1}$.
Spectre de masse : 359 $[M + 1]^+$.

**EXEMPLE 37 : 2-Méthyl-1,3-dioxo-4-(2-{[(trifluorométhyl)sulfonyl]oxy}phénoxy)-2,3-dihydropyrrolo[3,4-c]carbazole-6(1*H*)-carboxylate de tert-butyle**

124,3 μmol du composé de l'exemple 33 sont dissous, sous atmosphère anhydre, dans 1 ml de dichlorométhane sec. La température de la solution est abaissée à 0°C, puis 161,5 μmol de triéthylamine, et 161,5 μmol d'anhydride triflique, sont successivement additionnés goutte à goutte au milieu réactionnel, qui est agité de 0°C à température ambiante, pendant 1h20. Après hydrolyse avec de l'eau distillée, le mélange réactionnel est dilué avec du dichlorométhane, puis la phase aqueuse extraite trois fois avec le même solvant. Les phases organiques sont réunies, séchées sur du sulfate de magnésium, filtrées, et concentrées. Une purification par chromatographie sur gel de silice (éther de pétrole/acétate d'éthyle : 7/3), permet d'isoler le produit attendu.

Point de fusion : 182°C.
IR(KBr): $\nu_{C=O}$ = 1710, 1737, 1766 $cm^{-1}$.
Spectre de masse : 591 $[M + 1]^+$.

**EXEMPLE 38 : Trifluoro-méthanesulfonate-2-1(2-méthyl-1,3-dioxo-1,2,3,6-tétrahydropyrrolo[3,4-c]carbazol-4-yl)oxy]phényle**

Sous atmosphère inerte, 67,7 μmol du composé de l'exemple 37 sont dissous dans 1 ml de diméthylformamide distillé, en présence de 6,8 μmol de chlorure de bistriphénylphosphine de palladium, 203,1 μmol de chlorure de lithium sec, et de 81,2 μmol de 1,8-diazabicyclo[5.4.0]undéc-7-ène. Après 4h20 d'agitation à 140°C, le milieu réactionnel est refroidi, puis le diméthylformamide éliminé. Une purification par chromatographie sur gel de silice (éther de pétrole/acétate d'éthyle : 6/4), permet d'isoler le produit attendu.
Point de fusion : 214°C.
IR(KBr): $\nu_{C=O}$ = 1700-1708, 1757 $cm^{-1}$; $\nu_{NH}$= 3430 $cm^{-1}$.
Spectre de masse : 491 $[M + 1]^+$.

**EXEMPLE 39 : 8-(Benzyloxy)-4-(2-hydroxyphénoxy)-2-méthyl-1,3-dioxo-2,3-dihydropyrrolo[3,4-c]carbazole-6(1*H*)-carboxylate de tert-butyle**

Le produit attendu est obtenu lors de la purification du composé du stade B de l'exemple 21.
Point de fusion : 191°C.
IR(KBr) : $\nu_{C=O}$ = 1701, 1734 $cm^{-1}$ ;$\nu_{OH}$= 3425 $cm^{-1}$.
Spectre de masse : 565 $[M + 1]^+$.

**EXEMPLE 40 : 8-(Beazyloxy)-2-méthyl-1,3-dioxo-4-(2-{[(trifluorométhyl) sulfonyl]oxy}phénoxy)-2,3-dihydropyrrolo[3,4-c]carbazole-6(1*H*)-carboxylate de tert-butyle**

Le produit attendu est obtenu selon le procédé de l'exemple 37 à partir du composé de l'exemple 39.
Point de fusion : 186°C.
IR(KBr) : $\nu_{C=O}$ = 1703, 1714, 1724 $cm^{-1}$.
Spectre de masse : 697 $[M + 1]^+$.

**EXEMPLE 41 : 8-Hydroxy-4-(2-hydroxyphénoxy)-2-méthylpyrrolo[3,4-c] carbazole-1,3(2*H*,6*H*)-dione**

Le produit attendu est obtenu selon le procédé de l'exemple 18 à partir du composé de l'exemple 39.
Point de fusion : 200°C.
IR(KBr) : $\nu_{C=O}$ = 1691 $cm^{-1}$; $\nu_{OH,NH}$ = 3399 $cm^{-1}$.
Spectre de masse : 375 $[M + 1]^+$.

**EXEMPLE 42 : 12-(Benzhydryloxy)-7-méthyl-6,8-dioxo-7,8-dihydro[1,4] benzodioxino[2,3-a]pyrrolo[3,4-c]carbazole-13(6*H*)-carboxylate de tert-butyle**

***Stade A: 7-(Benzhydryloxy)-3-[4-(1,4-benzodioxin-2-yl)-1-méthyl-2,5-dioxo-2,5-dihydro-1H-pyrrolo-3-yl]-1H-indole-1-carboxylate de tert-butyle***

Le produit attendu est obtenu selon le procédé décrit au stade A de l'exemple 5 à partir du composé de la préparation O.
Point de fusion : 138°C (décomposition).
IR(KBr) : $\nu_{C=O}$ = 1707, 1753 $cm^{-1}$.
Spectre de masse : 641,5 $[M + 1]^+$.

***Stade B: 12-(Benzhydryloxy)-7-méthyl-6,8-dioxo-7,8-dihydro[1,4]benzodioxino [2,3-a]pyrrolo[3,4-c]carbazole-13(6H)-carboxylate de tert-butyle***

Le produit attendu est obtenu selon le procédé décrit au stade B de l'exemple 17 à partir du composé du stade A précédent.

***ETUDE PHARMACOLOGIOUE DES COMPOSES DE L'INVENTION***

**EXEMPLE 43 : Activité in vitro**

✧ Leucémie murine L1210

La leucémie murine L1210 a été utilisée in vitro. Les cellules sont cultivées dans du milieu de culture RPMI 1640 complet contenant 10 % de sérum de veau foetal, 2 mM de glutamine, 50 U/ml de pénicilline, 50 μg/ml de streptomycine et 10 mM d'Hepes, pH : 7,4. Les cellules sont réparties dans des microplaques et exposées aux composés cytotoxiques pendant 4 temps de doublement, soit 48 heures. Le nombre de cellules viables est ensuite quantifié par un essai colorimétrique, le Microculture Tetrazolium Assay (J. Carmichael et al., Cancer Res.; 47, 936-942, (1987)). Les résultats sont exprimés en $IC_{50}$, concentration en cytotoxique qui inhibe à 50 % la prolifération des cellules traitées. Tous les produits de l'invention montrent une bonne cytotoxicité sur cette lignée cellulaire. A titre d'exemple, le composé de l'exemple 20 présente une $IC_{50}$ de 0,074 μM sur L1210.

✧ Lignées cellulaires humaines

Les composés de l'invention ont également été testés sur des lignées cellulaires humaines issues de tumeurs solides selon le même protocole expérimental que celui décrit sur la leucémie murine L1210 mais avec des temps d'incubation de 4 jours au lieux de 2 jours. A titre indicatif, le composé de l'exemple 20 présente un $IC_{50}$ de 190 nM sur le carcinome de la prostate DU145 et de l'ordre de 10 à 200 nM sur des lignées humaines issues de carcinome pulmonaire non à petites cellules A549, de carcinome du colon HT-29 et de carcinome epidermoïde KB-3-1.

**EXEMPLE 44: Action sur le cycle cellulaire**

Les cellules L1210 sont incubées pendant 21 heures à 37°C en présence de différentes concentrations en produit testés. Les cellules sont ensuite fixées par de l'éthanol à 70 % (v/v), lavées deux fois dans du PBS et incubées 30 minutes à 20°C dans du PBS contenant 100 μg/ml de RNAse et 50 μg/ml d'iodure de propidium. Les résultats sont exprimés en pourcentage des cellules accumulées en phase G2+M après 21 heures par rapport au témoin (témoin : 20 %). Les composés de l'invention sont particulièrement intéressants. Ainsi, ils induisent une accumulation d'au moins 70 % des cellules en phase G2 + M après 21 heures à une concentration inférieure à 2.5 μM.

**EXEMPLE 45: Activité in vivo**

***Activité antitumorale sur la leucémie P 388***

La lignée P388 (leucémie murine) a été fournie par le National Cancer Institute (Frederick, USA). Les cellules tumorales ($10^6$ cellules) ont été inoculées au jour 0 dans la cavité péritonéale de souris B6D2F1 femelles (Iffa Credo, France). Six souris de 18 à 20 g ont été utilisées par groupe expérimental. Les produits ont été administrés par voie intrapéritonéale au jour 1.

$$\text{T/C \% (souris)} = \frac{\text{Temps de survie médian des animaux traités}}{\text{Temps de survie médian des animaux contrôles}} \times 100$$

Les résultats obtenus montrent une excellente activité in vivo sur le modèle leucémique P 388 avec un T/C de 210 % pour une dose de 50 mg/kg, ainsi qu'une faible toxicité des composés témoin d'un excellent index thérapeutique.

**EXEMPLE 46 : Composition pharmaceutique : soluté injectable**

| | |
|---|---|
| Composé de l'exemple 20 | 10 mg |
| Eau distillée pour préparations injectables | 25 ml |

## Revendications

**1.** Composés de formule (I) :

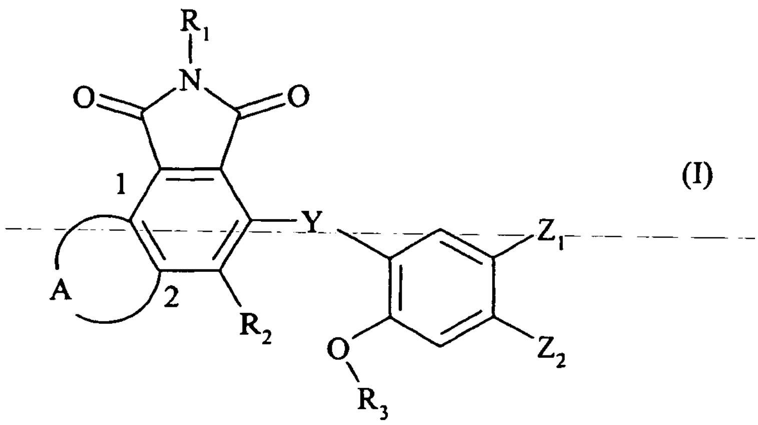

(I)

dans laquelle :

• A représente avec les atomes de carbone auxquel il est lié, un groupement de formule (a) ou (b) :

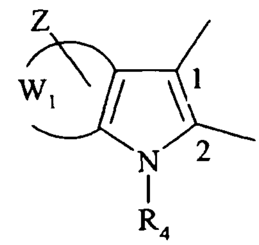

(a)

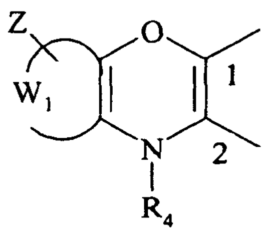

(b)

dans lesquels :

✧ $W_1$ représente avec les atomes de carbone auxquels il est lié, un groupement phényle ou un groupement pyridinyle,

➢ Z représente un groupement choisi parmi atome d'hydrogène, halogène, groupement alkyle ($C_1$-$C_6$) linéaire ou ramifié, nitro, cyano, hydroxy, alkoxy ($C_1$-$C_6$) linéaire ou ramifié, aryle, arylalkyle ($C_1$-$C_6$) linéaire ou ramifié, aryloxy, arylalkoxy ($C_1$-$C_6$) linéaire ou ramifié, $NR_5R_6$, dans lequel $R_5$ et $R_6$, identiques ou différents, indépendamment l'un de l'autre, représentent chacun un groupement choisi parmi atome d'hydrogène, groupement alkyle ($C_1$-$C_6$) linéaire ou ramifié,

✧ $R_4$ représente un groupement choisi parmi atome d'hydrogène, groupement alkyle ($C_1$-$C_6$) linéaire ou ramifié, aryle, arylalkyle ($C_1$-$C_6$) linéaire ou ramifié, ou un groupement -C(O)-OR'$_5$, dans lequel R'$_5$ représente un groupement choisi parmi groupement alkyle ($C_1$-$C_6$) linéaire ou ramifié, aryle, ou aryalkyle ($C_1$-$C_6$) linéaire ou ramifié,

• Y représente un groupement choisi parmi atome d'oxygène ou groupement méthylène,
• $R_2$ représente un atome d'hydrogène, et dans ce cas :

$R_3$ représente un groupement choisi parmi atome d'hydrogène, groupement alkyle ($C_1$-$C_6$) linéaire ou ramifié, aryle, arylalkyle ($C_1$-$C_6$) linéaire ou ramifié, ou $SO_2CF_3$,
• ou bien $R_2$ et $R_3$ forment une liaison,
• $R_1$ représente un groupement choisi parmi atome d'hydrogène, groupement alkyle ($C_1$-$C_6$) linéaire ou ramifié, aryle, arylalkyle ($C_1$-$C_6$) linéaire ou ramifié, ou une chaîne alkylène ($C_1$-$C_6$) linéaire ou ramifiée, substituée par un ou plusieurs groupements, identiques ou différents, choisis parmi -OR"$_5$, -NR"$_5$R"$_6$, dans lesquels R"$_5$ et R"$_6$ ont les mêmes définitions que $R_5$ et $R_6$ tels que définis précédemment,
• **$Z_1$** et **$Z_2$** représentent chacun un atome d'hydrogène ou,
**$Z_1$** et **$Z_2$** forment ensemble, avec les atomes de carbone qui les portent, un groupement phényle,

étant entendu que, lorsque Z représente un atome d'hydrogène alors $R_1$ est différent de atome d'hydrogène, leurs énantiomères, diastéréoisomères, N-oxyde, ainsi que leurs sels d'addition à un acide ou à une base pharmaceutiquement acceptable,
étant entendu que par aryle, on comprend un groupement phényle, naphtyle, dihydronaphtyle, tétrahydronaphtyle, indényle ou indanyle, chacun de ces groupements étant éventuellement substitué par un ou plusieurs groupements, identiques ou différents, choisis parmi halogène, alkyle ($C_1$-$C_6$) linéaire ou ramifié, triahalogénoalkyle ($C_1$-$C_6$) linéaire ou ramifié, hydroxy, alkoxy ($C_1$-$C_6$) linéaire ou ramifié, et amino éventuellement substitué par un ou deux groupements alkyle ($C_1$-$C_6$) linéaire ou ramifié.

**2.** Composés de formule (I) selon la revendication 1, **caractérisés en ce qu'**ils représentent des composés de formule (IA) :

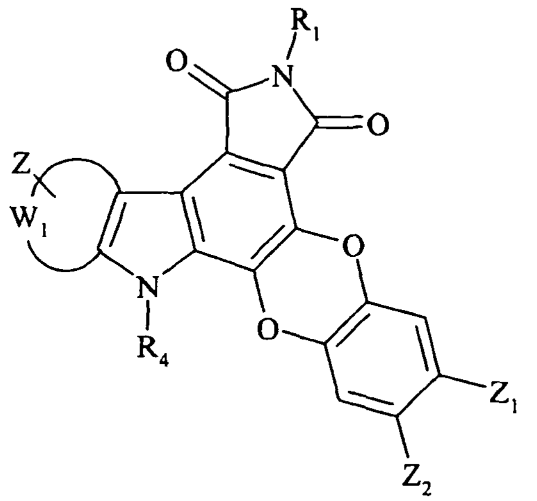

(IA)

dans laquelle $R_1$, $R_4$, $W_1$, Z, $Z_1$ et $Z_2$ sont tels que définis dans la formule (I), leurs énantiomères, diastéréoisomères, N-oxyde, ainsi que leurs sels d'addition à un acide ou à une base pharmaceutiquement acceptable.

**3.** Composés de formule (I) selon l'une quelconque des revendications 1 ou 2,
**caractérisés en ce qu'**ils représentent des composés de formule (IB) :

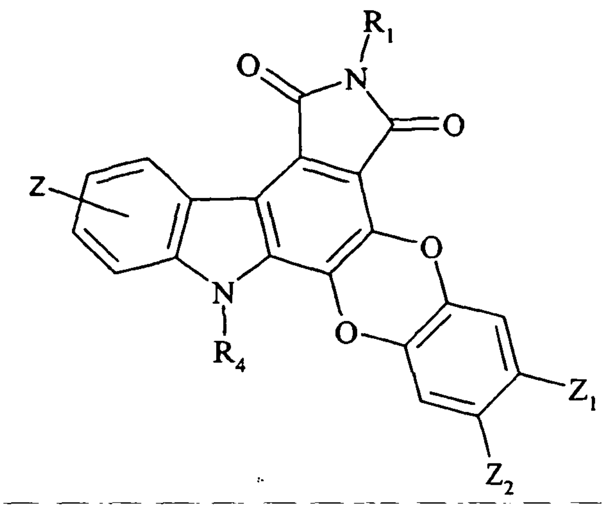

(IB)

dans laquelle $R_1$, $R_4$, Z, $Z_1$ et $Z_2$ sont tels que définis dans la formule (I), leurs énantiomères, diastéréoisomères, N-oxyde, ainsi que leurs sels d'addition à un acide ou à une base pharmaceutiquement acceptable.

4. Composés de formule (I) selon l'une quelconque des revendications 1 ou 2, **caractérisés en ce qu'**ils représentent des composés de formule (IC) :

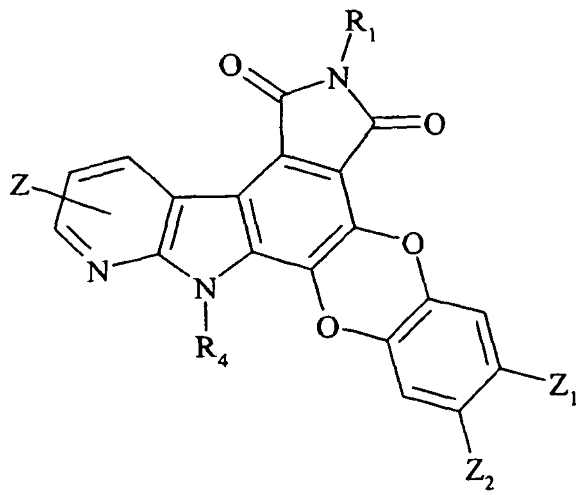

(IC)

dans laquelle $R_1$, $R_4$, Z, $Z_1$ et $Z_2$ sont tels que définis dans la formule (I), leurs énantiomères, diastéréoisomères, N-oxyde, ainsi que leurs sels d'addition à un acide ou à une base pharmaceutiquement acceptable.

5. Composés de formule (I) selon la revendication 1, **caractérisés en ce qu'**ils représentent des composés de formule (ID) :

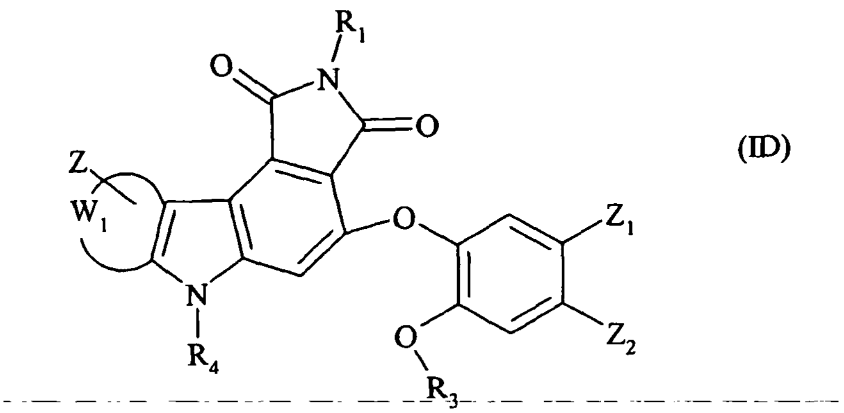

(ID)

dans laquelle $R_1$, $R_3$, $R_4$, $W_1$, Z, $Z_1$ et $Z_2$ sont tels que définis dans la formule (I), leurs énantiomères, diastéréoisomères, N-oxyde, ainsi que leurs sels d'addition à un acide ou à une base pharmaceutiquement acceptable.

6. Composés de formule (I) selon l'une quelconque des revendications 1 ou 5, **caractérisés en ce qu'**ils représentent des composés de formule (IE) :

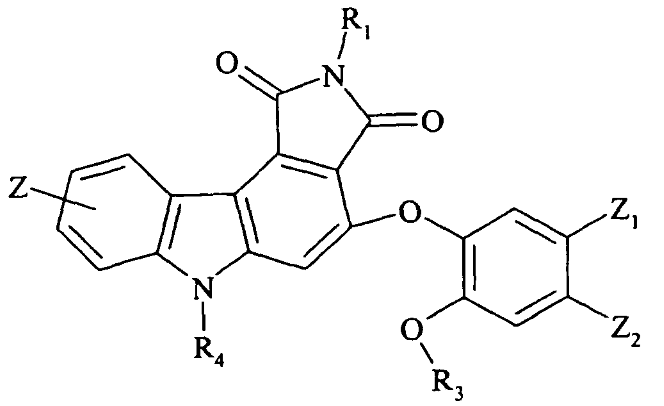

(IE)

dans laquelle $R_1$, $R_3$, $R_4$, Z, $Z_1$ et $Z_2$ sont tels que définis dans la formule (I), leurs énantiomères, diastéréoisomères, N-oxyde, ainsi que leurs sels d'addition à un acide ou à une base pharmaceutiquement acceptable.

7. Composés de formule (I) selon l'une quelconque des revendications 1 ou 5, **caractérisés en ce qu'**ils représentent des composés de formule (IF) :

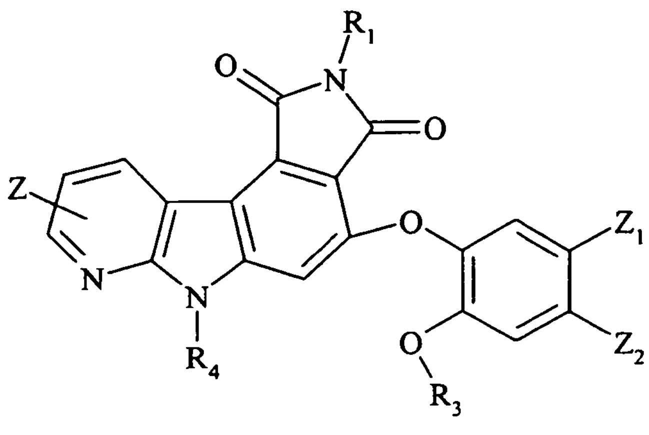

(IF)

dans laquelle $R_1$, $R_3$, $R_4$, Z, $Z_1$ et $Z_2$ sont tels que définis dans la formule (I), leurs énantiomères, diastéréoisomères, N-oxyde, ainsi que leurs sels d'addition à un acide ou à une base pharmaceutiquement acceptable.

**8.** Composés de formule (I) selon l'une quelconque des revendications 1 ou 5, **caractérisés en ce qu'**ils représentent des composés de formule (IG) :

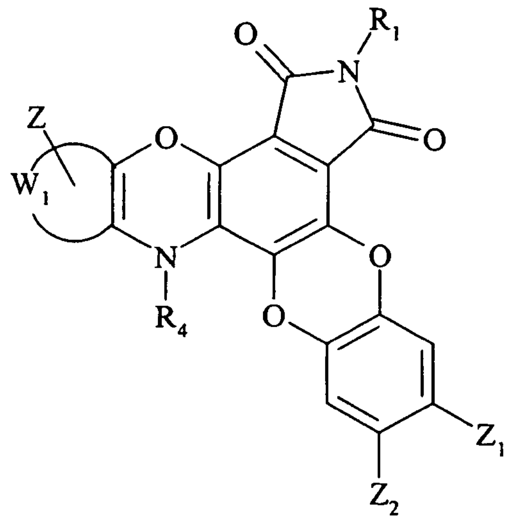

(IG)

dans laquelle $R_1$, $R_4$, $W_1$, Z, $Z_1$ et $Z_2$ sont tels que définis dans la formule (I), leurs énantiomères, diastéréoisomères, N-oxyde, ainsi que leurs sels d'addition à un acide ou à une base pharmaceutiquement acceptable.

**9.** Composés de formule (I) selon l'une quelconque des revendications 1 ou 8, **caractérisés en ce qu'**ils représentent des composés de formule (IH) :

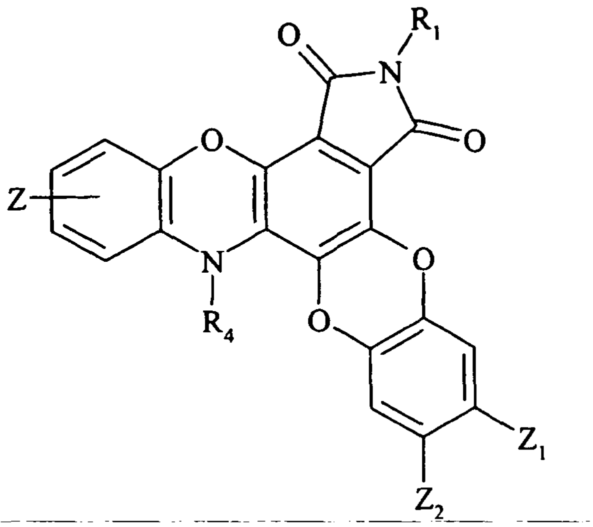

(IH)

dans laquelle $R_1$, $R_4$, Z, $Z_1$ et $Z_2$ sont tels que définis dans la formule (I), leurs énantiomères, diastéréoisomères, N-oxyde, ainsi que leurs sels d'addition à un acide ou à une base pharmaceutiquement acceptable.

**10.** Composés de formule (I) selon l'une quelconque des revendications 1 ou 8, **caractérisés en ce qu'**ils représentent des composés de formule (IJ) :

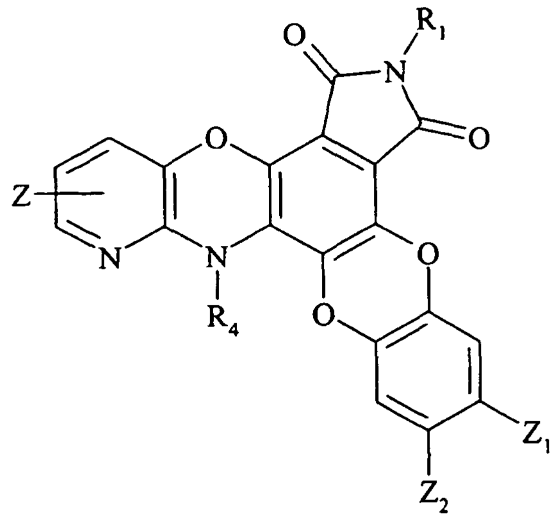

(IJ)

dans laquelle $R_1$, $R_4$, Z, $Z_1$ et $Z_2$ sont tels que définis dans la formule (I), leurs énantiomères, diastéréoisomères, N-oxyde, ainsi que leurs sels d'addition à un acide ou à une base pharmaceutiquement acceptable.

**11.** Composés de formule (I) selon l'une quelconque des revendications 1 à 10, **caractérisés en ce que** Z représente un atome d'hydrogène, halogène ou un groupement hydroxy, leurs énantiomères, diastéréoisomères, N-oxyde, ainsi que leurs sels d'addition à un acide ou à une base pharmaceutiquement acceptable.

**12.** Composés de formule (I) selon l'une quelconque des revendications 1 à 11, **caractérisés en ce que** A, avec les atomes de carbone auxquels ils sont liés, représentent les groupements de formules :

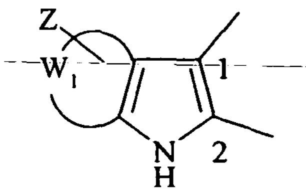

ou

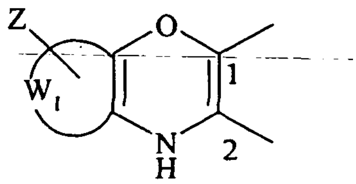

leurs énantiomères, diastéréoisomères, N-oxyde, ainsi que leurs sels d'addition à un acide ou à une base pharmaceutiquement acceptable.

**13.** Composés de formule (1) selon l'une quelconque des revendications 1 à 12, **caractérisés en ce** $R_3$ représente un atome d'hydrogène ou un groupement alkyle ($C_1$-$C_6$) linéaire ou ramifié, leurs énantiomères, diastéréoisomères, N-oxyde, ainsi que leurs sels d'addition à un acide ou à une base pharmaceutiquement acceptable.

**14.** Composés de formule (I) selon l'une quelconque des revendications 1 à 13, **caractérisés en ce** $R_1$ représente un atome d'hydrogène, un groupement alkyle ($C_1$-$C_6$) linéaire ou ramifié, ou une chaîne alkylène ($C_1$-$C_6$) linéaire ou ramifiée substituée par un ou plusieurs groupements, identiques ou différents, choisis parmi $-NR_5R_6$, dans lequel $R_5$ et $R_6$ sont tels que définis dans la formule (I), leurs énantiomères, diastéréoisomères, N-oxyde, ainsi que leurs sels d'addition à un acide ou à une base pharmaceutiquement acceptable.

**15.** Composés de formule (I) selon l'une quelconque des revendications 1 à 14, **caractérisés en ce que** $Z_1$ et $Z_2$ représentent l'atome d'hydrogène, leurs énantiomères, diastéréoisomères, N-oxyde, ainsi que leurs sels d'addition à un acide ou à une base pharmaceutiquement acceptable.

**16.** Composés de formule (I) selon la revendication 1 qui sont le :

- 7-méthyl[1,4]benzodioxino[2,3-a]pyrrolo[3,4-c]carbazole-6,8-dione,

- 10-fluoro-7-méthyl[1,4]benzodioxino[2,3-a]pyrrolo[3,4-c]carbazole-6,8-dione,
- 11-fluoro-7-méthyl[1,4]benzodioxino[2,3-a]pyrrolo[3,4-c]carbazole-6,8-dione,
- 7-[2-(diméthylamino)éthyl]-10-fluoro[1,4]benzodioxino[2,3-a]pyrrolo[3,4-c] carbazole-6,8-dione,
- 10-hydroxy[1,4]benzodioxino[2,3-a]pyrrolo[3,4-c]carbazole-6,8-dione,
- 11-hydroxy[1,4]benzodioxino[2,3-a]pyrrolo[3,4-c]carbazole-6,8-dione,
- 7-[2-(diméthylamino)éthyl][1,4]benzodioxino[2,3-a]pyrrolo[3,4-c]carbazole-6,8-dione,
- 7-[2-(diméthylamino)éthyl]-10-hydroxy[1,4]benzodioxino[2,3-a]pyrrolo[3,4-c] carbazole-6,8-dione,
- 7-[2-(diméthylamino)éthyl]-11-hydroxy[1,4]benzodioxino[2,3-a]pyrrolo[3,4-c] carbazole-6,8-dione,
- 7-[2-(diméthylamino)éthyl][1,4]benzodioxino[2,3-e]pyrido[2',3':5,6][1,4]oxazino [3,2-g]isoindole-6,8-dione.

leurs énantiomères, diastéréoisomères, N-oxyde, ainsi que leurs sels d'addition à un acide ou à une base pharmaceutiquement acceptable.

**17.** Procédé de préparation des composés de formule (I) selon la revendication 1, **caractérisé en ce que** l'on utilise comme produit de départ un composé de formule (II) :

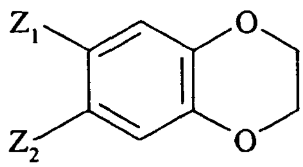

(II)

dans laquelle $Z_1$ et $Z_2$ sont tels que définis dans la formule (I), composé de formule (II) que l'on fait réagir avec du N-bromosuccinimide en présence de péroxyde de benzoyle pour conduire au composé de formule (III) :

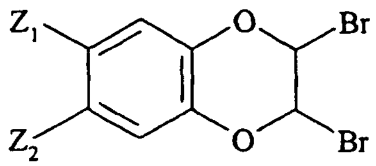

(III)

dans laquelle $Z_1$ et $Z_2$ sont tels que définis précédemment, composé de formule (III) qui est mis à réagir avec de l'iodure de sodium pour conduire au composé de formule (IV) :

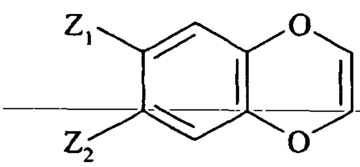

(IV)

dans laquelle $Z_1$ et $Z_2$ sont tels que définis précédemment, composé de formule (IV) qui est mis à réagir avec du n-butyllithium puis du chlorure de triméthylétain pour conduire au composé de formule (V) :

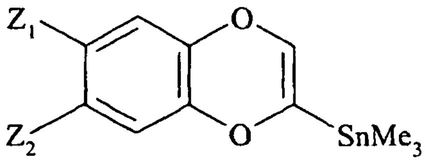

(V)

dans laquelle $Z_1$ et $Z_2$ sont tels que définis précédemment, composé de formule (V) qui est :

• soit traité, en présence de tétrakis(triphénylphosphine)palladium (0), par un composé de formule (VI) :

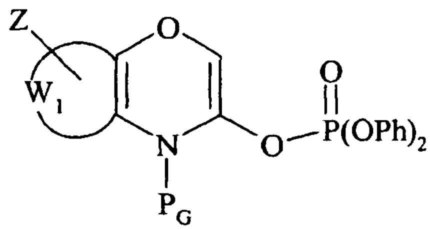

(VI)

dans laquelle $P_G$ représente un groupement protecteur des amines bien connu en synthèse organique et $W_1$ et Z sont tels que définis dans la formule (I) pour conduire au composé de formule (VII) :

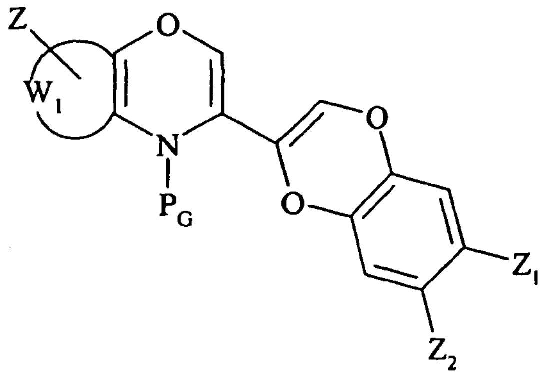

(VII)

dans laquelle $P_G$, Z, $Z_1$, $Z_2$ et $W_1$ sont tels que définis précédemment, composé de formule (VII) qui est traité par un composé de formule (VIII):

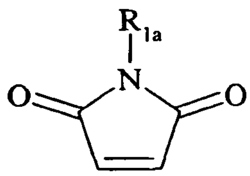

(VIII)

dans laquelle $R_{1a}$, représente un atome d'hydrogène ou un groupement méthyle, pour conduire au composé de formule (IX) :

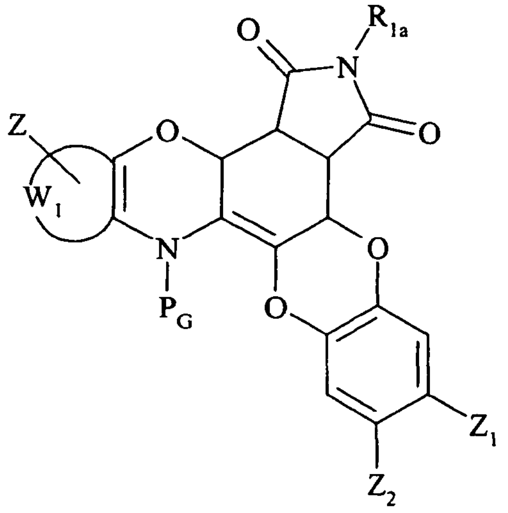

(IX)

dans laquelle $P_G$, $R_{1a}$, Z, $Z_1$, $Z_2$ et $W_1$ sont tels que définis précédemment, composé de formule (IX) qui est mis en présence de N-bromosuccinimide et de péroxyde de benzoyle, pour conduire au composé de formule (I/a), cas particulier des composés de formule (I) :

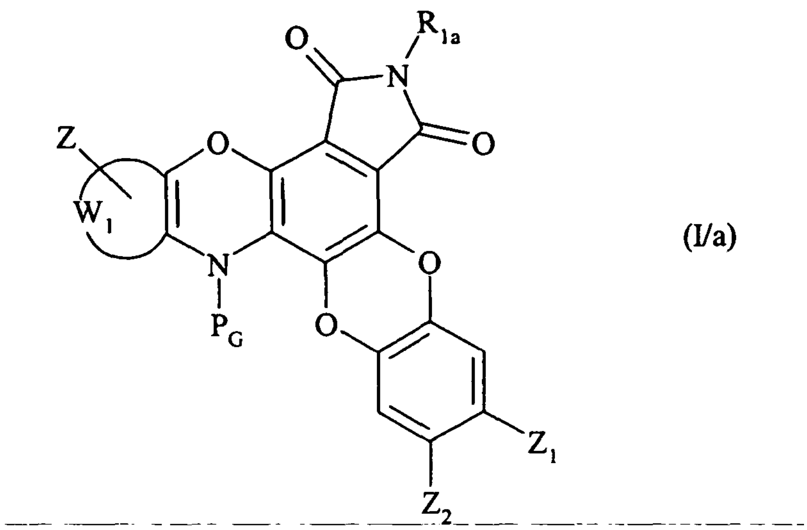

(I/a)

dans laquelle $P_G$, $R_{la}$, Z, $Z_1$, $Z_2$ et $W_1$ sont tels que définis précédemment, composé de formule (I/a) qui est éventuellement traité par un composé de formule (X) :

$R_{1b}$-$NH_2$ (X)

dans laquelle $R_{1b}$, différent de atome d'hydrogène et de groupement méthyle, a la même définition que $R_1$ dans la formule (I), pour conduire au composé de formule (I/b), cas particulier des composés de formule (I) :

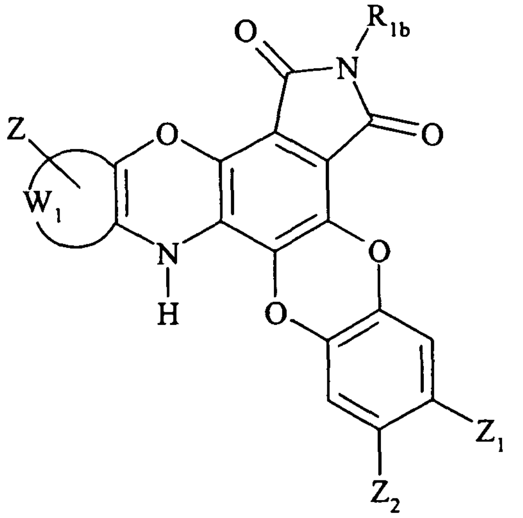

(I/b)

dans laquelle $R_{1b}$, Z, $Z_1$, $Z_2$ et $W_1$ sont tels que définis précédemment,
les composés des formules (I/a) et (I/b) forment les composés de formule (I/c) :

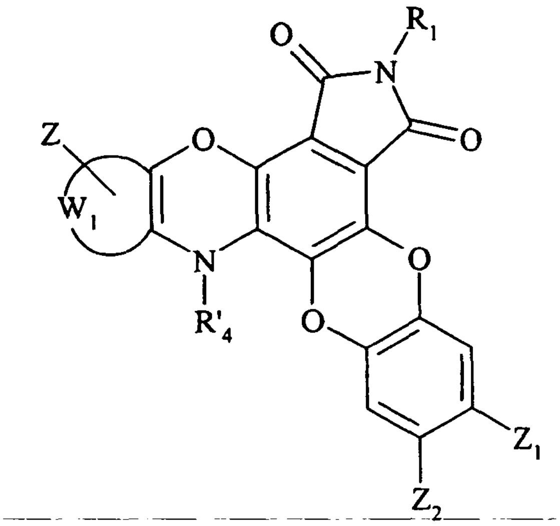

(I/c)

dans laquelle $R'_4$ représente un atome d'hydrogène ou un groupement $P_G$ et $R_1$, Z, $Z_1$, $Z_2$ et $W_1$ sont tels que définis précédemment,

• soit traité, en présence de chlorure de bistriphénylphosphinepalladium (II), par un composé de formule (XI) :

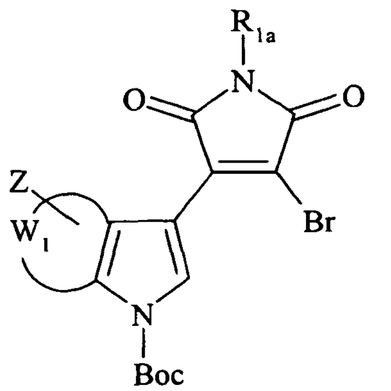

(XI)

dans laquelle Boc représente un groupement tert-butoxycarbonyle et $R_{1a}$, $W_1$ et Z sont tels que définis précédemment, pour conduire au composé de formule (XII) :

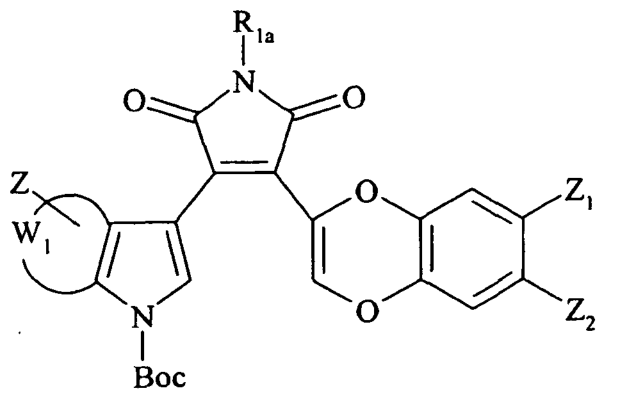

(XII)

dans laquelle Boc, $R_{1a}$, Z, $Z_1$, $Z_2$ et $W_1$ sont tels que définis précédemment, composé de formule (XII) qui est :

♦ soit irradié par une lampe UV, en présence d'iode, dans un solvant apolaire et aprotique, pour conduire aux composés de formules (I/d) et (I/e), cas particulier des composés de formule (I) :

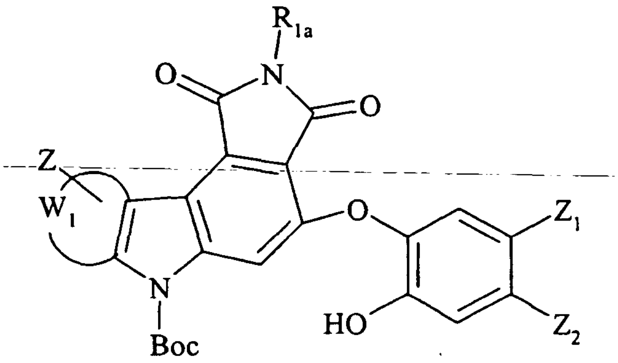

(I/d)

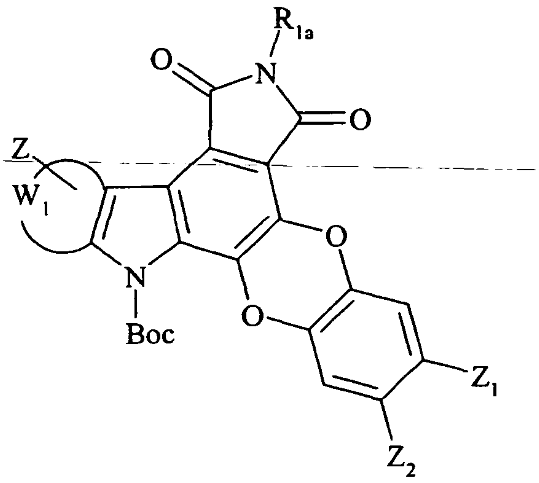

(I/e)

dans lesquelles Boc, $R_{1a}$, Z, $Z_1$, $Z_2$ et $W_1$ sont tels que définis précédemment, composés de formule (I/d) :

✧ dont on déprotège éventuellement la fonction amine selon des méthodes classiques de la synthèse organique pour conduire au composé de formule (I/f), cas particulier des composés de formule (I) :

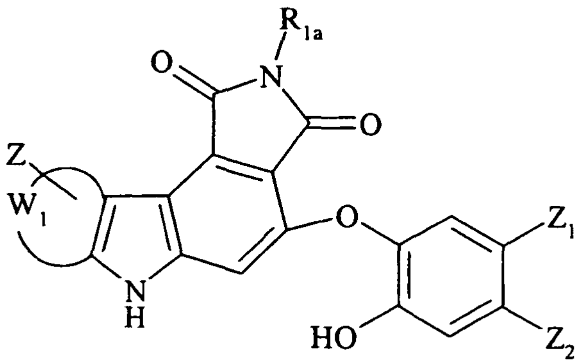

(I/f)

dans laquelle $R_{1a}$, Z, $Z_1$, $Z_2$ et $W_1$ sont tels que définis précédemment,

✧ soit soumis éventuellement à l'action d'un composé de formule (XIII) :

$R_{3a}$-G (XIII)

dans laquelle $R_{3a}$, différent de atome d'hydrogène, à la même définition que $R_3$ dans la formule (I) et G est tel que défini précédemment, pour conduire au composé de formule (I/g), cas particulier des composés de formule (I) :

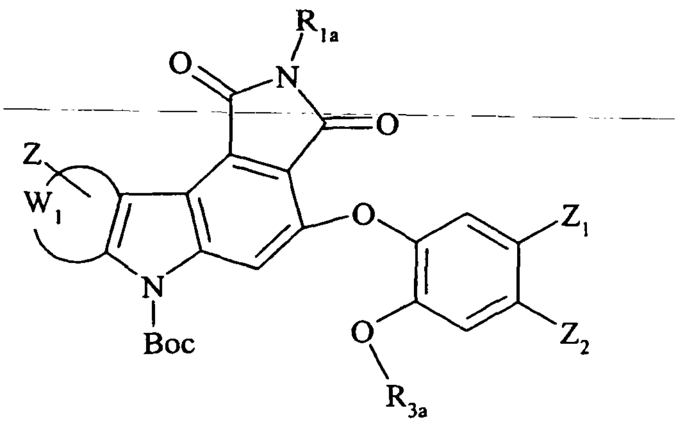

(I/g)

dans laquelle Boc, $R_{1a}$, $R_{3a}$, Z, $Z_1$, $Z_2$ et $W_1$ sont tels que définis précédemment, les composés de formule (I/d), (I/e) et (I/g) forment le composé de formule (I/h) :

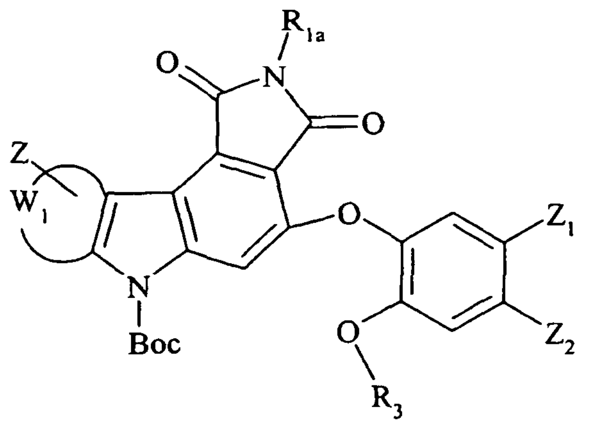

(I/h)

dans laquelle Boc, $R_{1a}$, $R_3$, Z, $Z_1$, $Z_2$ et $W_1$ sont tels que définis précédemment, composés de formule (I/h) qui est éventuellement soumis aux mêmes conditions de réaction que le composé de formule (I/a), pour conduire au composé de formule (I/i), cas particulier des composés de formule (I) :

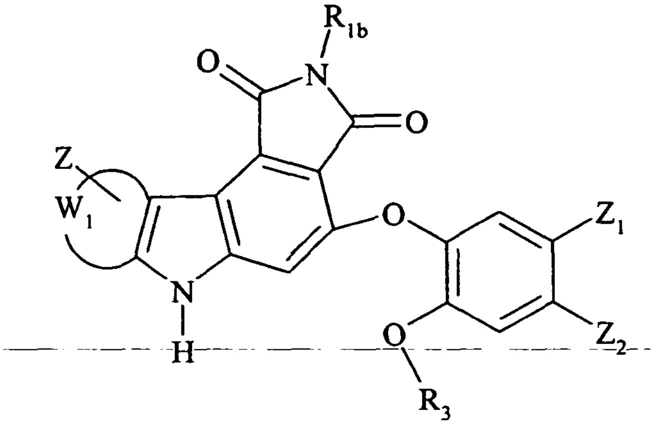

(I/i)

dans laquelle $R_{1b}$, $R_3$, Z, $Z_1$, $Z_2$ et $W_1$ sont tels que définis précédemment,

♦ soit soumis à l'action d'acide chlorhydrique pour conduire au composé de formule (XIV):

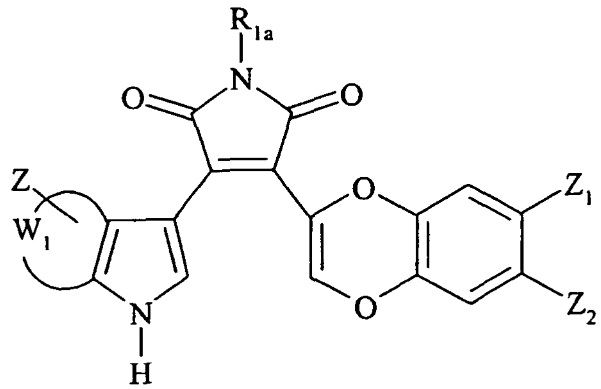

(XIV)

dans laquelle $R_{1a}$, Z, $Z_1$, $Z_2$ et $W_1$ sont tels que définis précédemment, composé de formule (XIV) qui est soumis à l'action d'un composé de formule (XV) :

$R_{4a}$-G (XV)

dans laquelle G représente un groupement partant et $R_{4a}$, différent de atome d'hydrogène, a la même définition que $R_4$ dans la formule (I), pour conduire au composé de formule (XVI) :

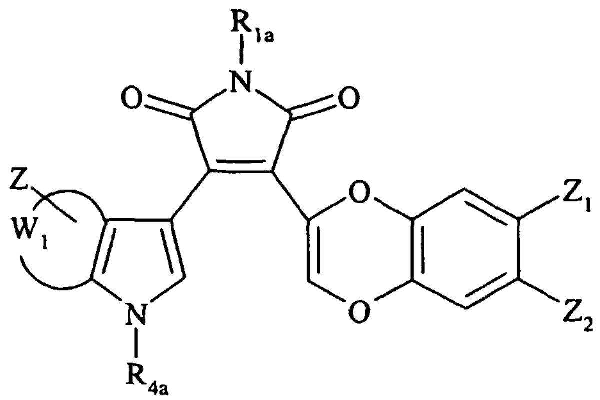

(XVI)

dans laquelle $R_{1a}$, $R_{4a}$, Z, $Z_1$, $Z_2$ et $W_1$ sont tels que définis précédemment,
composé de formule (XVI) qui est soumis aux mêmes conditions de réaction que le composé de formule (XII) pour conduire aux composés de formules (I/j) et (I/k) cas particulier des composés de formule (I) :

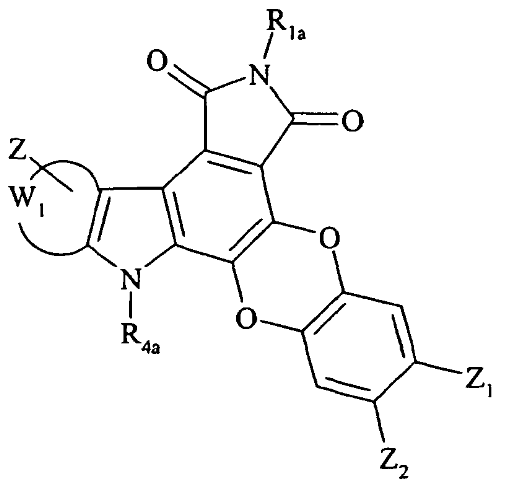

(I/j)

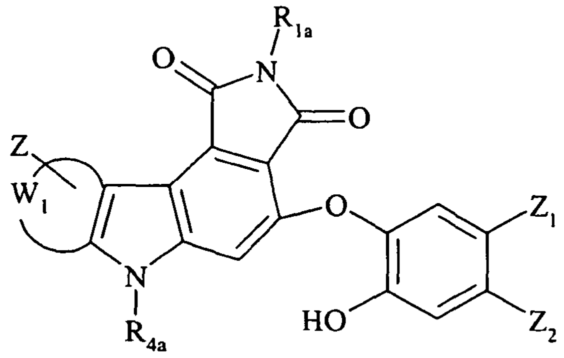

(I/k)

dans lesquelles $R_{1a}$, $R_{4a}$, Z, $Z_1$, $Z_2$ et $W_1$ sont tels que définis précédemment, composé de formule (I/k) qui est éventuellement soumis à l'action d'un composé de formule (XIII) tel que défini précédemment pour conduire au composé de formule (I/1) :

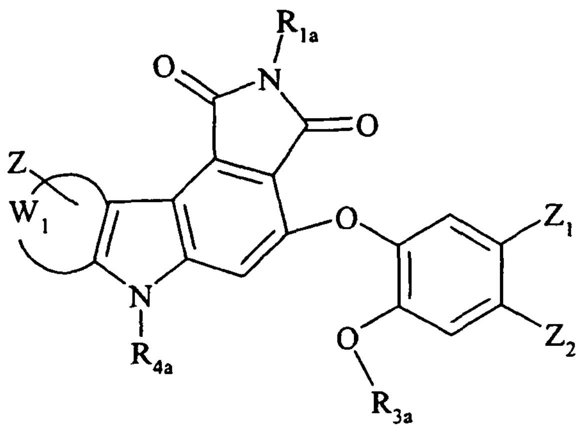

(I/l)

dans laquelle $R_{1a}$, $R_{3a}$, $R_{4a}$, Z, $Z_1$, $Z_2$ et $W_1$ sont tels que définis précédemment, les composés de formule (I/j), (I/k) et (I/1) forment les composés de formule (I/m) :

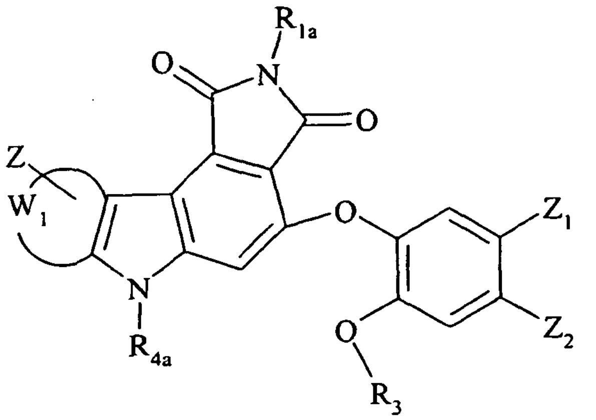

(I/m)

dans laquelle $R_{1a}$, $R_3$, $R_{4a}$, Z, $Z_1$, $Z_2$ et $W_1$ sont tels que définis précédemment, composé de formule (I/m) qui est éventuellement soumis aux mêmes conditions de réaction que le composé de formule (I/h) pour conduire au composé de formule (I/n) :

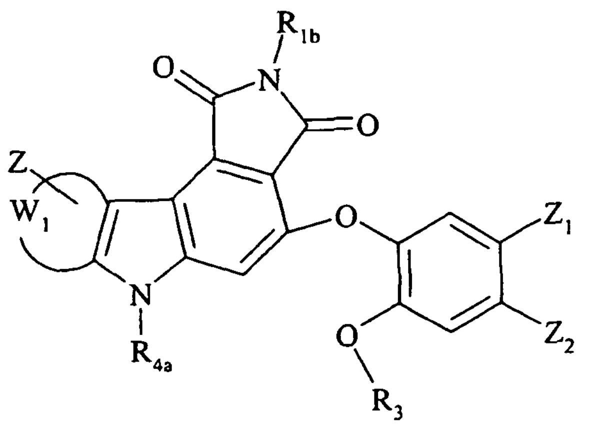

(I/n)

dans laquelle $R_{1b}$, $R_3$, $R_{4a}$, Z, $Z_1$, $Z_2$ et $W_1$ sont tels que définis précédemment,
les composés de formule (I/e), (I/h) et (I/i), (I/m) et (I/n) forment les composés de formule (I/o) :

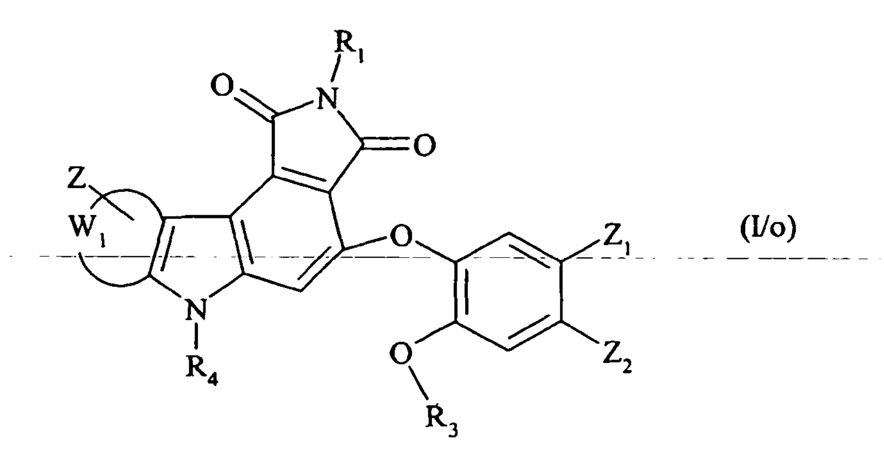

(I/o)

dans laquelle $R_1$, $R_3$, $R_4$, Z, $Z_1$, $Z_2$ et $W_1$ sont tels que définis précédemment,
les composés de formule (I/a) à (I/o) forment l'ensemble des composés de formule (I), que l'on purifie, le cas échéant, selon des techniques classiques de purification, qui peuvent, si on le désire, être séparés en leurs différents isomères, selon une technique classique de séparation, et que l'on transforme, si on le souhaite, en leurs N-oxydes et, le cas échéant, en leurs sels d'addition à un acide ou à une base pharmaceutiquement acceptable.

**18.** Compositions pharmaceutiques contenant comme principe actif au moins un composé de formule (I), selon l'une quelconque des revendications 1 à 16, seul ou en combinaison avec un ou plusieurs excipients ou véhicules inertes, non toxiques, pharmaceutiquement acceptables.

**19.** Compositions pharmaceutiques selon le revendication 18, utiles en tant que médicament, dans le traitement des cancers.

## Claims

**1.** Compounds of formula (I) :

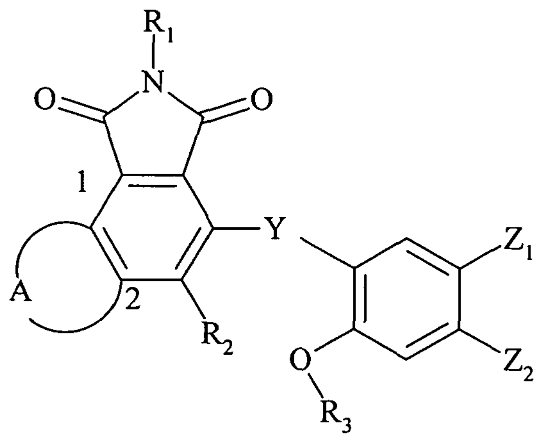

(I),

wherein :

• A, together with the carbon atoms to which it is bonded, represents a group of formula (a) or (b) :

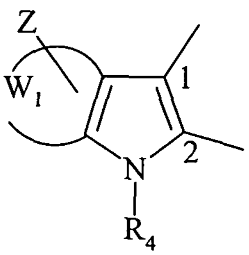

(a)

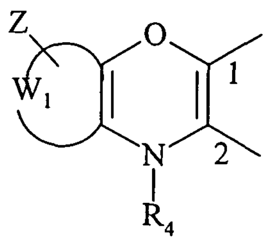

(b),

wherein :

- $W_1$, together with the carbon atoms to which it is bonded, represents a phenyl group or a pyridyl group,

- Z represents a group selected from hydrogen and halogen atoms and the groups linear or branched ($C_1$-$C_6$)alkyl, nitro, cyano, hydroxy, linear or branched ($C_1$-$C_6$)alkoxy, aryl, aryl-($C_1$-$C_6$)alkyl (in which the alkyl moiety is linear or branched), aryloxy and aryl-($C_1$-$C_6$)alkoxy (in which the alkoxy moiety is linear or branched) and $NR_5R_6$ wherein $R_5$ and $R_6$, which are identical or different, each independently of the other represents a group selected from a hydrogen atom and a linear or branched ($C_1$-$C_6$)alkyl group,

- $R_4$ represents a group selected from a hydrogen atom and the groups linear or branched ($C_1$-$C_6$)alkyl, aryl and aryl-($C_1$-$C_6$)alkyl (in which the alkyl moiety is linear or branched) or a group -C(O)-OR'$_5$ wherein R'$_5$ represents a group selected from the groups linear or branched ($C_1$-$C_6$)alkyl, aryl and aryl-($C_1$-$C_6$)alkyl (in which the alkyl moiety is linear or branched),

• Y represents a group selected from an oxygen atom and a methylene group,
• $R_2$ represents a hydrogen atom and, in that case:
$R_3$ represents a group selected from a hydrogen atom and the groups linear or branched ($C_1$-$C_6$)alkyl, aryl, aryl-($C_1$-$C_6$)alkyl (in which the alkyl moiety is linear or branched) and $SO_2CF_3$,
• or $R_2$ and $R_3$ form a bond,
• $R_1$ represents a group selected from a hydrogen atom and the groups linear or branched ($C_1$-$C_6$)alkyl, aryl and aryl-($C_1$-$C_6$)alkyl (in which the alkyl moiety is linear or branched) or a linear or branched ($C_1$-$C_6$)alkylene chain substituted by one or more identical or different groups selected from -OR"$_5$ and -NR"$_5$R"$_6$ wherein R"$_5$ and R"$_6$ are as defined for $R_5$ and $R_6$ defined hereinbefore,
• **$Z_1$** and **$Z_2$** each represent a hydrogen atom or
**$Z_1$** and **$Z_2$**, together with the carbon atoms carrying them, form a phenyl group,
with the proviso that, when Z represents a hydrogen atom, $R_1$ is other than a hydrogen atom,
their enantiomers, diastereoisomers, N-oxide, and addition salts thereof with a pharmaceutically acceptable acid or base,

wherein "aryl" is to be understood as meaning a phenyl, naphthyl, dihydronaphthyl, tetrahydronaphthyl, indenyl or indanyl group, each of those groups being optionally substituted by one or more identical or different groups selected from halogen, linear or branched ($C_1$-$C_6$)alkyl, linear or branched ($C_1$-$C_6$)trihaloalkyl, hydroxy, linear or branched ($C_1$-$C_6$)alkoxy, and amino optionally substituted by one or two linear or branched ($C_1$-$C_6$)alkyl groups.

**2.** Compounds of formula (I) according to claim 1, **characterised in that** they represent compounds of formula (IA) :

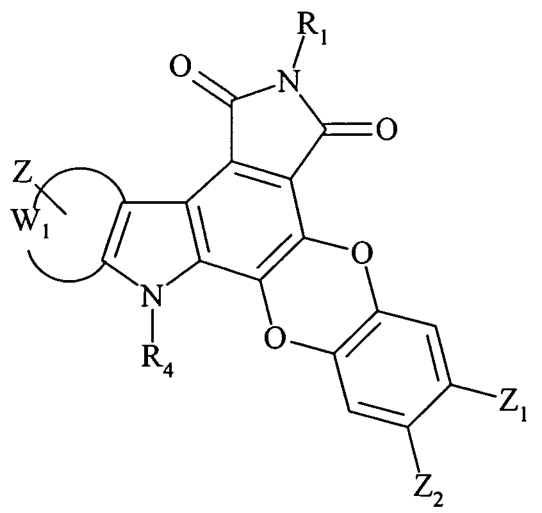

(IA),

wherein $R_1$, $R_4$, $W_1$, Z, $Z_1$ and $Z_2$ are as defined for formula (I), their enantiomers, diastereoisomers, N-oxide, and

addition salts thereof with a pharmaceutically acceptable acid or base.

**3.** Compounds of formula (I) according to either claim 1 or claim 2, **characterised in that** they represent compounds of formula (IB) :

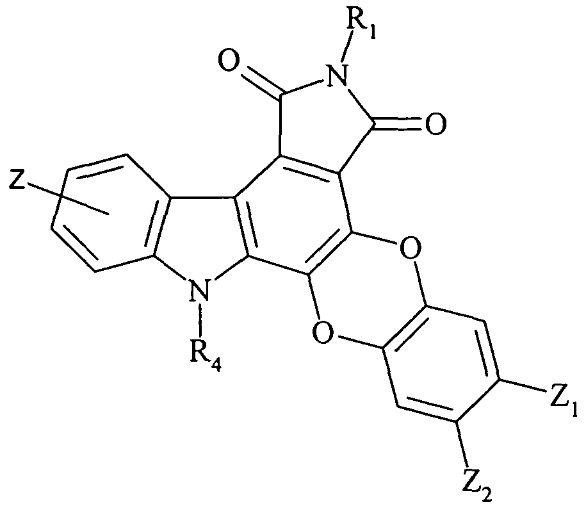

(IB),

wherein $R_1$, $R_4$, Z, $Z_1$ and $Z_2$ are as defined for formula (I), their enantiomers, diastereoisomers, N-oxide, and addition salts thereof with a pharmaceutically acceptable acid or base.

**4.** Compounds of formula (I) according to either claim 1 or claim 2, **characterised in that** they represent compounds of formula (IC) :

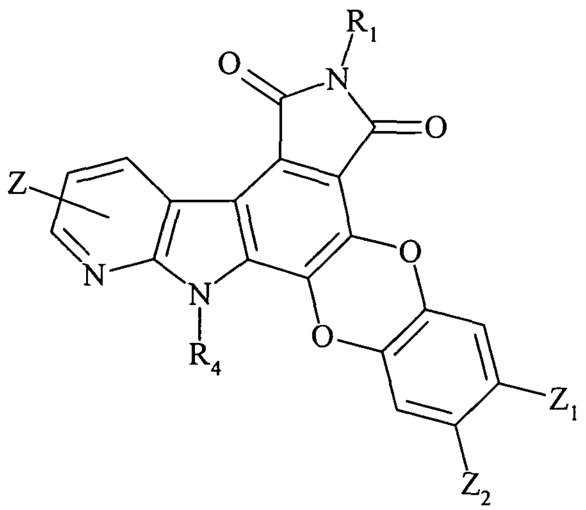

(IC),

wherein $R_1$, $R_4$, Z, $Z_1$ and $Z_2$ are as defined for formula (I), their enantiomers, diastereoisomers, N-oxide, and addition salts thereof with a pharmaceutically acceptable acid or base.

**5.** Compounds of formula (I) according to claim 1, **characterised in that** they represent compounds of formula (ID) :

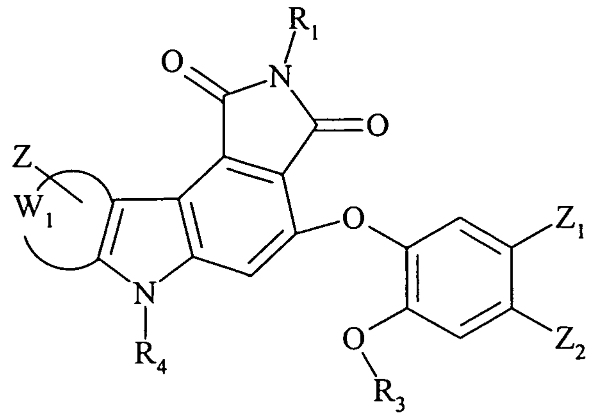

(ID),

wherein $R_1$, $R_3$, $R_4$, $W_1$, Z, $Z_1$ and $Z_2$ are as defined for formula (I), their enantiomers, diastereoisomers, N-oxide, and addition salts thereof with a pharmaceutically acceptable acid or base.

6. Compounds of formula (I) according to either claim 1 or claim 5, **characterised in that** they represent compounds of formula (IE) :

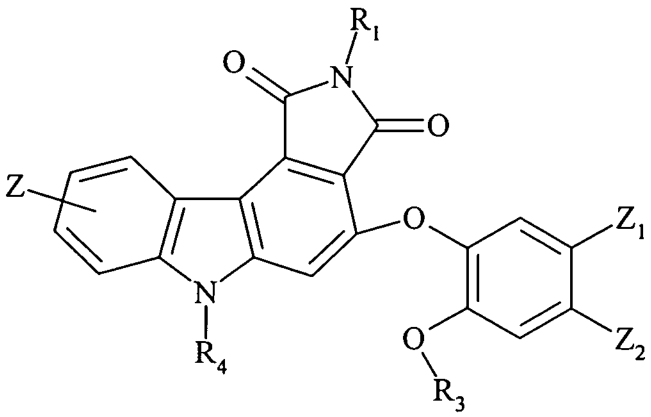

(IE),

wherein $R_1$, $R_3$, $R_4$, Z, $Z_1$ and $Z_2$ are as defined for formula (I), their enantiomers, diastereoisomers, N-oxide, and addition salts thereof with a pharmaceutically acceptable acid or base.

7. Compounds of formula (I) according to either claim 1 or claim 5, **characterised in that** they represent compounds of formula (IF) :

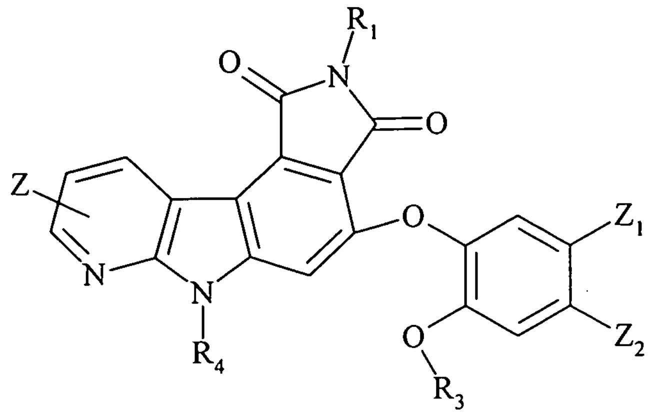

(IF),

wherein $R_1$, $R_3$, $R_4$, Z, $Z_1$ and $Z_2$ are as defined for formula (I), their enantiomers, diastereoisomers, N-oxide, and addition salts thereof with a pharmaceutically acceptable acid or base.

**8.** Compounds of formula (I) according to either claim 1 or claim 5, **characterised in that** they represent compounds of formula (IG) :

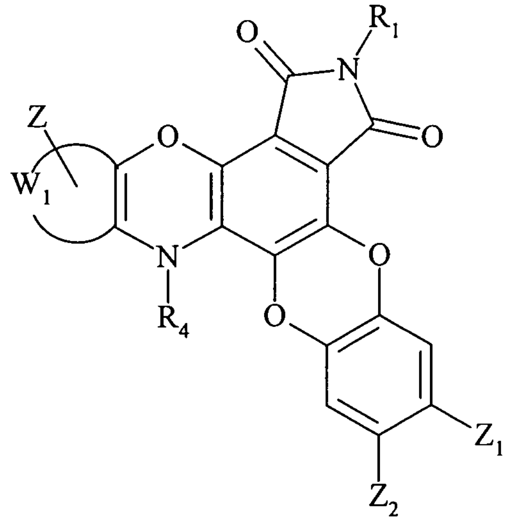

(IG),

wherein $R_1$, $R_4$, $W_1$, Z, $Z_1$ and $Z_2$ are as defined for formula (I), their enantiomers, diastereoisomers, N-oxide, and addition salts thereof with a pharmaceutically acceptable acid or base.

**9.** Compounds of formula (I) according to either claim 1 or claim 8, **characterised in that** they represent compounds of formula (IH) :

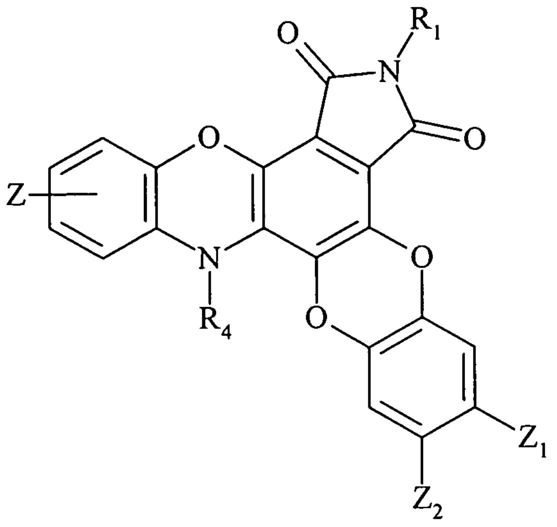

(IH),

wherein $R_1$, $R_4$, Z, $Z_1$ and $Z_2$ are as defined for formula (I), their enantiomers, diastereoisomers, N-oxide, and addition salts thereof with a pharmaceutically acceptable acid or base.

**10.** Compounds of formula (I) according to either claim 1 or claim 8, **characterised in that** they represent compounds of formula (IJ) :

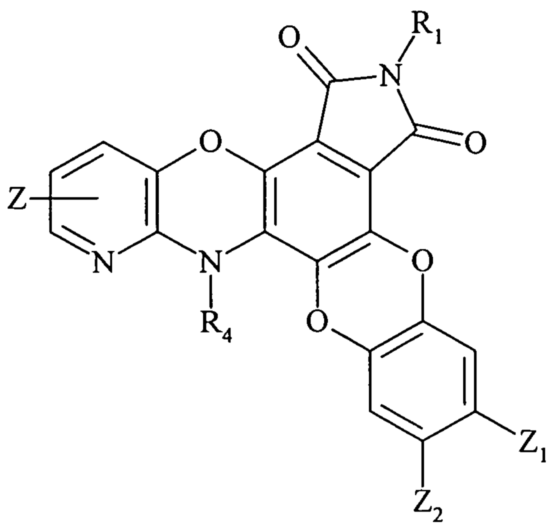

(IJ),

wherein $R_1$, $R_4$, Z, $Z_1$ and $Z_2$ are as defined for formula (I), their enantiomers, diastereoisomers, N-oxide, and addition salts thereof with a pharmaceutically acceptable acid or base.

**11.** Compounds of formula (I) according to any one of claims 1 to 10, **characterised in that** Z represents a hydrogen atom, halogen atom or hydroxy group, their enantiomers, diastereoisomers, N-oxide, and addition salts thereof with a pharmaceutically acceptable acid or base.

**12.** Compounds of formula (I) according to any one of claims 1 to 11, **characterised in that** A, together with the carbon atoms to which it is bonded, represents a group of formula :

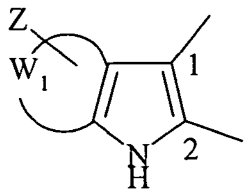 or 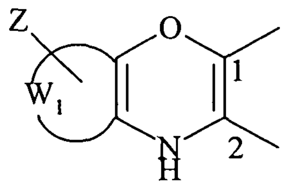 ,

their enantiomers, diastereoisomers, N-oxide, and addition salts thereof with a pharmaceutically acceptable acid or base.

**13.** Compounds of formula (I) according to any one of claims 1 to 12, **characterised in that** $R_3$ represents a hydrogen atom or a linear or branched ($C_1$-$C_6$)alkyl group, their enantiomers, diastereoisomers, N-oxide, and addition salts thereof with a pharmaceutically acceptable acid or base.

**14.** Compounds of formula (I) according to any one of claims 1 to 13, **characterised in that** $R_1$ represents a hydrogen atom, a linear or branched ($C_1$-$C_6$)alkyl group or a linear or branched ($C_1$-$C_6$)alkylene chain substituted by one or more identical or different groups selected from $-NR_5R_6$ wherein $R_5$ and $R_6$ are as defined for formula (I), their enantiomers, diastereoisomers, N-oxide, and addition salts thereof with a pharmaceutically acceptable acid or base.

**15.** Compounds of formula (I) according to any one of claims 1 to 14, **characterised in that** $Z_1$ and $Z_2$ represent hydrogen atoms, their enantiomers, diastereoisomers, N-oxide, and addition salts thereof with a pharmaceutically acceptable acid or base.

**16.** Compounds of formula (I) according to claim 1, which are :

- 7-methyl [1,4]benzodioxino[2,3-a]pyrrolo[3,4-c]carbazole-6,8-dione,
- 10-fluoro-7-methyl[1,4]benzodioxino [2,3 -a]pyrrolo [3,4-c]carbazole-6,8-dione,
- 11-fluoro-7-methyl[1,4]benzodioxino[2,3-a]pyrrolo[3,4-c]carbazole-6,8-dione,
- 7-[2-(dimethylamino)ethyl]-10-fluoro[1,4]benzodioxino[2,3-a]pyrrolo[3,4-c]-carbazole-6,8-dione,
- 10-hydroxy[1,4]benzodioxino[2,3-a]pyrrolo[3,4-c]carbazole-6,8-dione,
- 11-hydroxy[1,4]benzodioxino[2,3-a]pyrrolo[3,4-c]carbazole-6, 8-dione,
- 7-[2-(dimethylamino)ethyl][1,4]benzodioxino[2,3-a]pyrrolo[3,4-c]carbazole-6,8-dione,
- 7-[2-(dimethylamino)ethyl]-10-hydroxy[1,4]benzodioxino[2,3-a]pyrrolo[3,4-c]-carbazole-6,8-dione,
- 7-[2-(dimethylamino)ethyl]-11-hydroxy[1,4]benzodioxino[2,3-a]pyrrolo[3,4-c]-carbazole-6,8-dione,
- 7-[2-(dimethylamino)ethyl][1,4]benzodioxino[2,3-e]pyrido[2',3':5,6][1,4]oxazino-[3,2-g]isoindole-6,8-dione,

their enantiomers, diastereoisomers, N-oxide, and addition salts thereof with a pharmaceutically acceptable acid or base.

**17.** Process for the preparation of compounds of formula (I) according to claim 1, **characterised in that** there is used as starting material a compound of formula (II) :

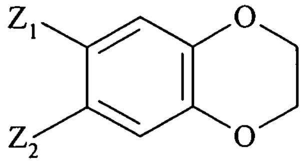 (II),

wherein $Z_1$ and $Z_2$ are as defined for formula (I),
which compound of formula (II) is reacted with N-bromosuccinimide in the presence of benzoyl peroxide to yield the compound of formula (III) :

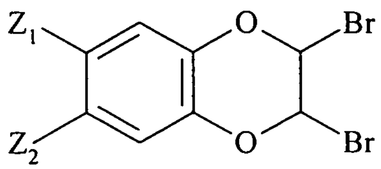

(III),

wherein $Z_1$ and $Z_2$ are as defined hereinbefore,
which compound of formula (III) is reacted with sodium iodide to yield the compound of formula (IV) :

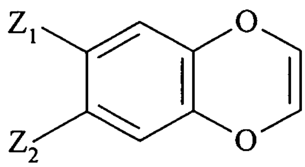

(IV),

wherein $Z_1$ and $Z_2$ are as defined hereinbefore,
which compound of formula (IV) is reacted with n-butyllithium and then with trimethyltin chloride to yield the compound of formula (V) :

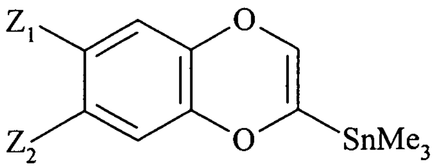

(V),

wherein $Z_1$ and $Z_2$ are as defined hereinbefore,
which compound of formula (V) is :

- either treated, in the presence of tetrakis(triphenylphosphine)palladium(0), with a compound of formula (VI) :

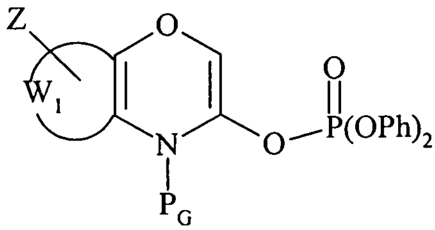

(VI),

wherein $P_G$ represents an amine-protecting group well-known in organic synthesis and $W_1$ and Z are as defined for formula (I), to yield the compound of formula (VII) :

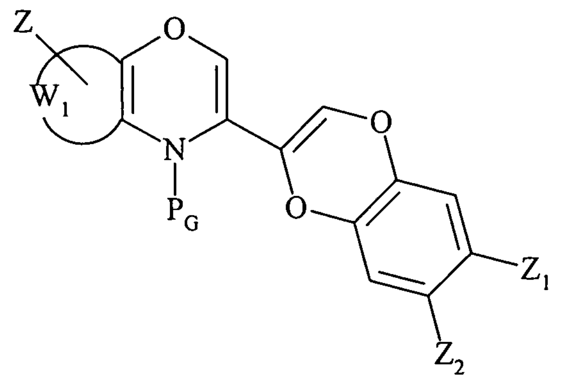

(VII),

wherein $P_G$, Z, $Z_1$, $Z_2$ and $W_1$ are as defined hereinbefore, which compound of formula (VII) is treated with a compound of formula (VIII) :

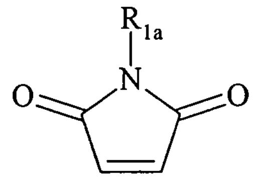

(VIII),

wherein $R_{1a}$ represents a hydrogen atom or a methyl group, to yield the compound of formula (IX) :

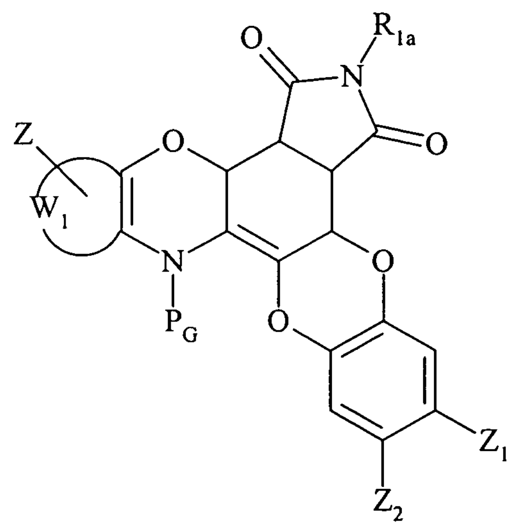

(IX),

wherein $P_G$, $R_{1a}$, Z, $Z_1$, $Z_2$ and $W_1$ are as defined hereinbefore, which compound of formula (IX) is treated with N-bromosuccinimide and benzoyl peroxide to yield the compound of formula (I/a), a particular case of the compounds of formula (I) :

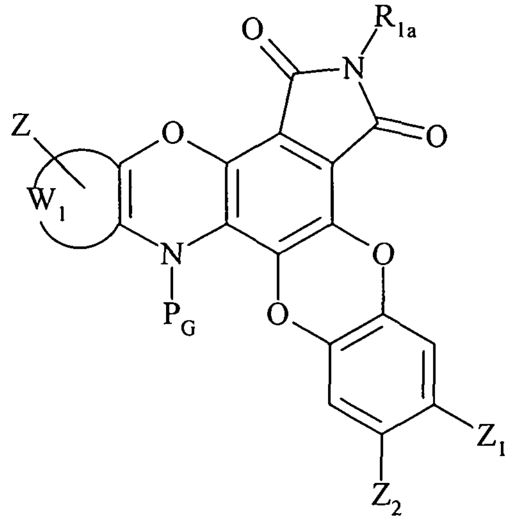

(I/a),

wherein $P_G$, $R_{1a}$, Z, $Z_1$, $Z_2$ and $W_1$ are as defined hereinbefore, which compound of formula (I/a) is optionally treated with a compound of formula (X) :

$R_{1b}$-$NH_2$ (X),

wherein $R_{1b}$ has the same definition as $R_1$ in formula (I) but is other than a hydrogen atom or a methyl group, to yield the compound of formula (I/b), a particular case of the compounds of formula (I) :

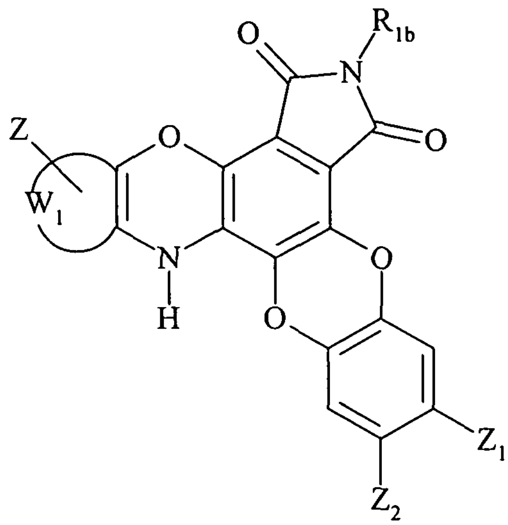

(I/b),

wherein $R_{1b}$, Z, $Z_1$, $Z_2$ and $W_1$ are as defined hereinbefore, which compounds of formulae (I/a) and (I/b) constitute the compounds of formula (I/c) :

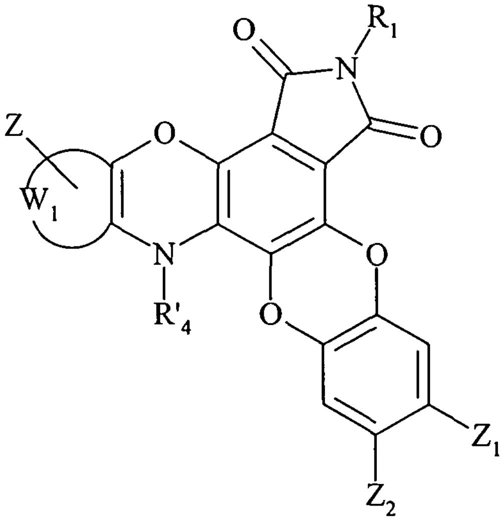

(I/c),

wherein $R'_4$ represents a hydrogen atom or a group $P_G$, and $R_1$, Z, $Z_1$, $Z_2$ and $W_1$ are as defined hereinbefore,
• or treated in the presence of bis(triphenylphosphine)palladium(II) chloride with a compound of formula (XI) :

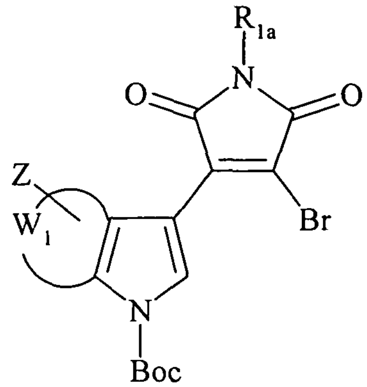

(XI),

wherein Boc represents a tert-butoxycarbonyl group and $R_{1a}$, $W_1$ and Z are as defined hereinbefore, to yield the compound of formula (XII) :

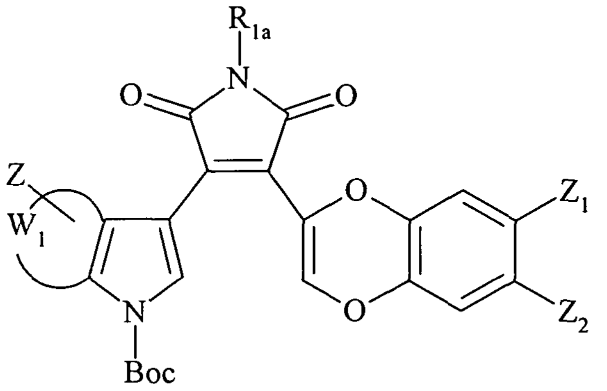

(XII),

wherein Boc, $R_{1a}$, Z, $Z_1$, $Z_2$ and $W_1$ are as defined hereinbefore, which compound of formula (XII) is :

♦ either irradiated with a UV lamp, in the presence of iodine, in a non-polar and aprotic solvent, to yield the compounds of formulae (I/d) and (I/e), particular cases of the compounds of formula (I) :

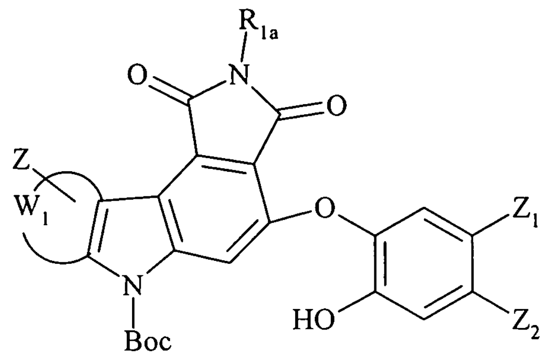

(I/d)

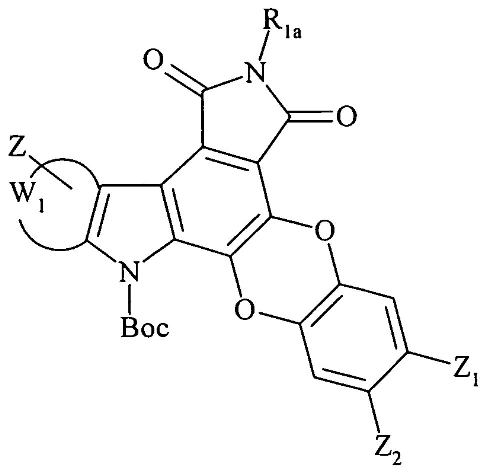

(I/e),

wherein Boc, $R_{1a}$, Z, $Z_1$, $Z_2$ and $W_1$ are as defined hereinbefore, which compounds of formula (I/d) :

✧ optionally are subjected to deprotection of the amine function according to conventional methods of organic synthesis to yield the compound of formula (I/f), a particular case of the compounds of formula (I) :

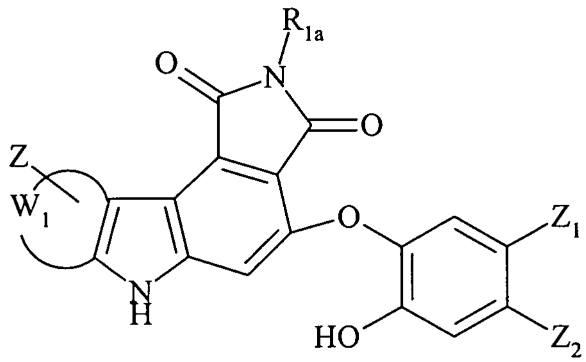

(I/f),

wherein $R_{1a}$, Z, $Z_1$, $Z_2$ and $W_1$ are as defined hereinbefore,

✧ or optionally are subjected to the action of a compound of formula (XIII) :

$R_{3a}$-G (XIII),

wherein $R_{3a}$, has the same definition as $R_3$ in formula (I) but is other than a hydrogen atom and G is as defined hereinbefore, to yield the compound of formula (I/g), a particular case of the compounds of formula (I) :

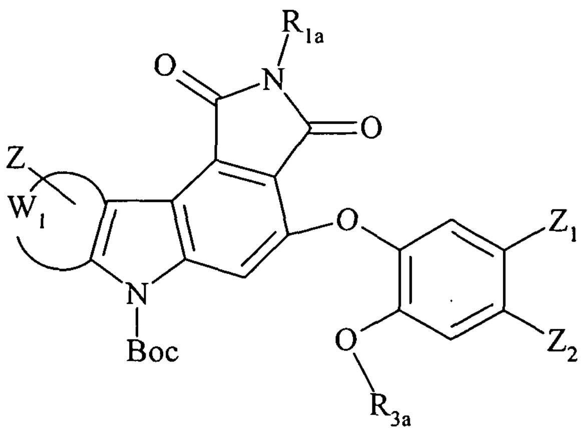

(I/g),

wherein Boc, $R_{1a}$, $R_{3a}$, Z, $Z_1$, $Z_2$ and $W_1$ are as defined hereinbefore,
which compounds of formulae (I/d), (I/e) and (I/g) constitute the compound of formula (I/h) :

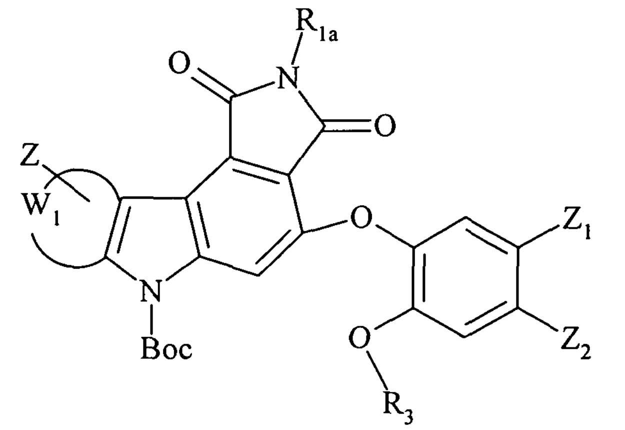

(I/h),

wherein Boc, $R_{1a}$, $R_3$, Z, $Z_1$, $Z_2$ and $W_1$ are as defined hereinbefore,
which compound of formula (I/h) optionally is subjected to the same reaction conditions as the compound of formula (I/a) to yield the compound of formula (I/i), a particular case of the compounds of formula (I) :

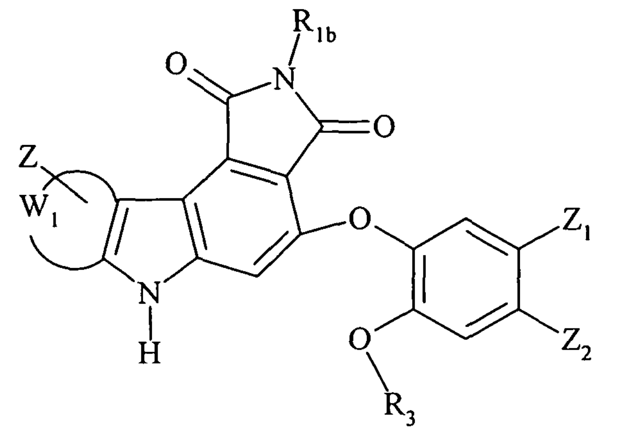

(I/i),

wherein $R_{1b}$, $R_3$, Z, $Z_1$, $Z_2$ and $W_1$ are as defined hereinbefore,

♦ or subjected to the action of hydrochloric acid to yield the compound of formula (XIV) :

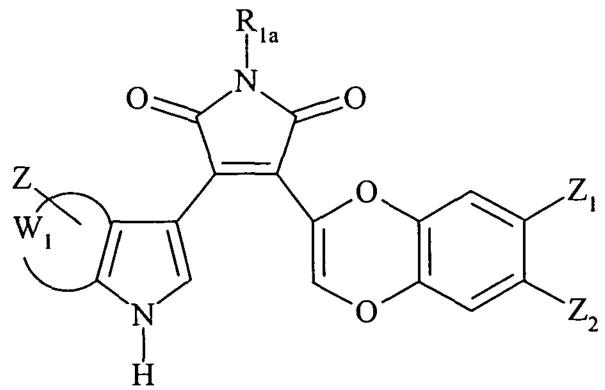

(XIV),

wherein $R_{1a}$, Z, $Z_1$, $Z_2$ and $W_1$ are as defined hereinbefore,

which compound of formula (XIV) is subjected to the action of a compound of formula (XV) :

$R_{4a}$-G (XV),

wherein G represents a leaving group and $R_{4a}$ has the same definition as $R_4$ in formula (I) but is other than a hydrogen atom, to yield the compound of formula (XVI) :

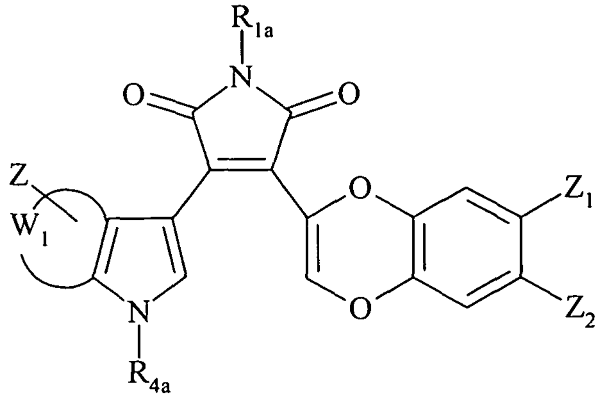

(XVI),

wherein $R_{1a}$, $R_{4a}$, Z, $Z_1$, $Z_2$ and $W_1$ are as defined hereinbefore,
which compound of formula (XVI) is subjected to the same reaction conditions as the compound of formula (XII) to yield the compounds of formulae (I/j) and (I/k), particular cases of the compounds of formula (I) :

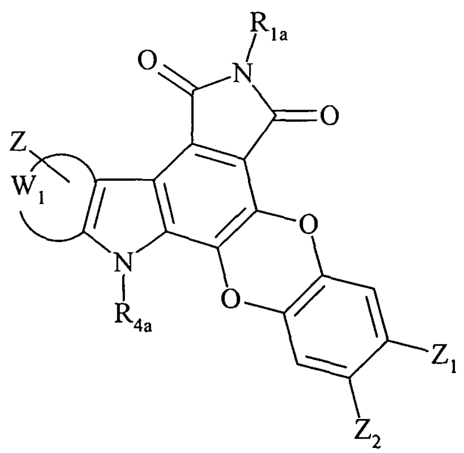

(I/j)

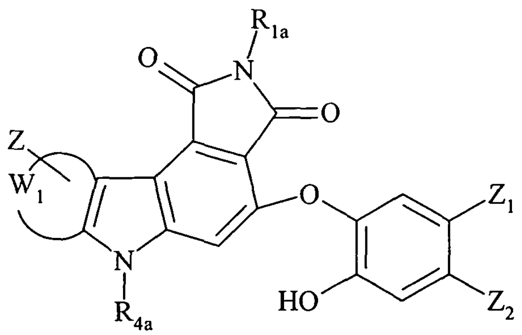

(I/k),

wherein $R_{1a}$, $R_{4a}$, Z, $Z_1$, $Z_2$ and $W_1$ are as defined hereinbefore,
which compound of formula (I/k) optionally is subjected to the action of a compound of formula (XIII) as defined hereinbefore to yield the compound of formula (I/1) :

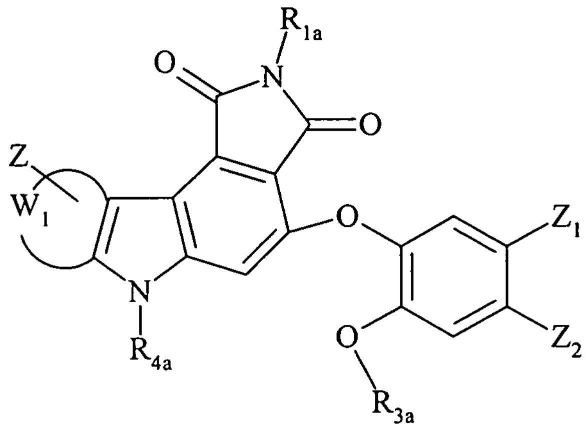

(I/l),

wherein $R_{1a}$, $R_{3a}$, $R_{4a}$, Z, $Z_1$, $Z_2$ and $W_1$ are as defined hereinbefore,
which compounds of formulae (I/j), (I/k) and (I/1) constitute the compounds of formula (I/m) :

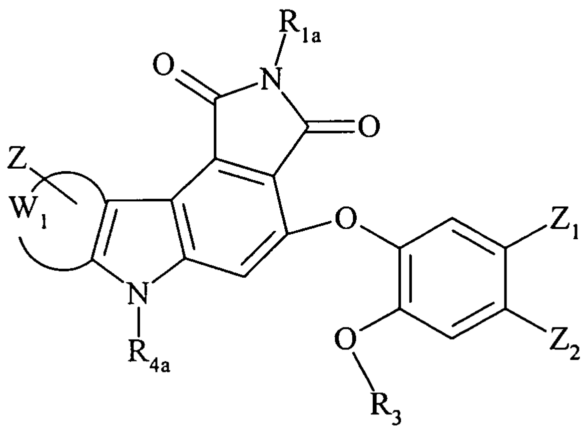

(I/m),

wherein $R_{1a}$, $R_3$, $R_{4a}$, Z, $Z_1$, $Z_2$ and $W_1$ are as defined hereinbefore,
which compound of formula (I/m) optionally is subjected to the same reaction conditions as the compound of formula (I/h) to yield the compound of formula (I/n) :

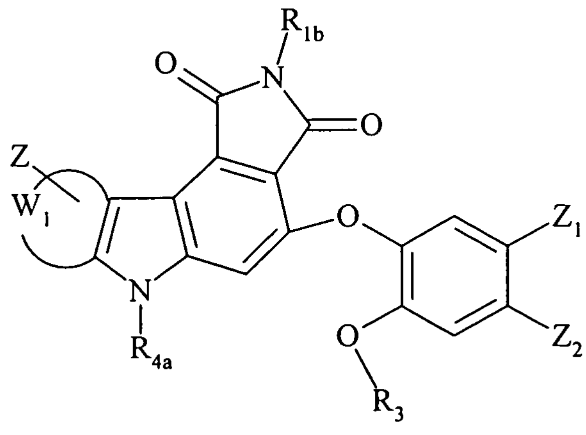

(I/n),

wherein $R_{1b}$, $R_3$, $R_{4a}$, Z, $Z_1$, $Z_2$ and $W_1$ are as defined hereinbefore,

which compounds of formulae (I/e), (I/h) and (I/i), (I/m) and (I/n) constitute the compounds of formula (I/o) :

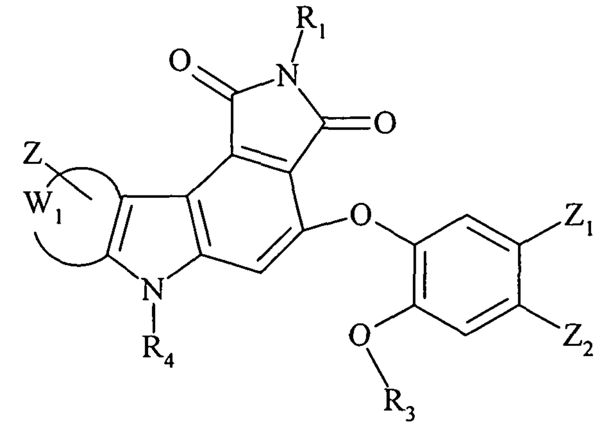

(I/o),

wherein $R_1$, $R_3$, $R_4$, Z, $Z_1$, $Z_2$ and $W_1$ are as defined hereinbefore, which compounds of formulae (I/a) to (I/o) constitute the totality of the compounds of formula (I), which are purified, where necessary, according to conventional purification techniques, which may be separated, if desired, into their different isomers according to a conventional separation technique, and which are converted, if desired, into their N-oxides and, where appropriate, their addition salts with a pharmaceutically acceptable acid or base.

**18.** Pharmaceutical compositions comprising, as active ingredient, at least one compound of formula (I) according to any one of claims 1 to 16, alone or in combination with one or more pharmaceutically acceptable, inert, non-toxic excipients or carriers.

**19.** Pharmaceutical compositions according to claim 18 for use as medicaments, in the treatment of cancers.

**Patentansprüche**

**1.** Verbindungen der Formel (I):

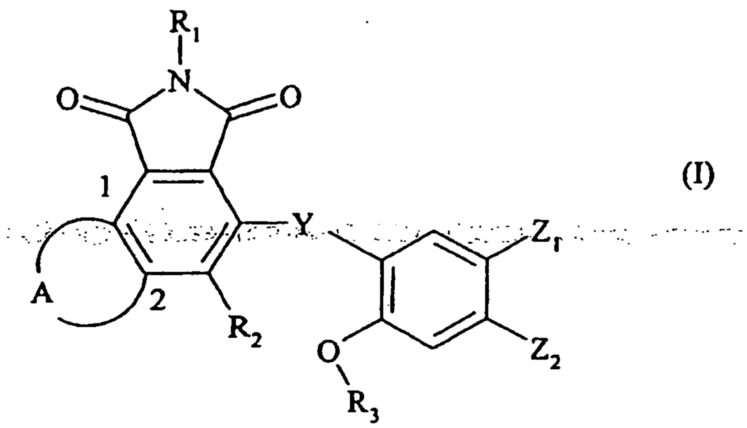

(I)

in der:

• A zusammen mit den Kohlenstoffatomen, an die es gebunden ist, eine Gruppe der Formel (a) oder (b) bedeutet:

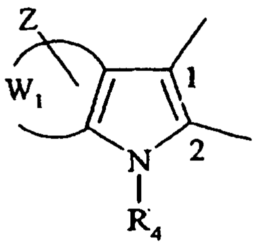

(a)

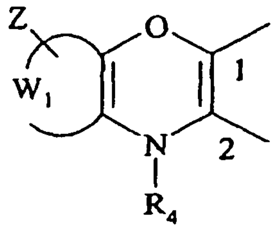

(b)

in denen:

✧ $W_1$ zusammen mit den Kohlenstoffatomen, an die es gebunden ist, eine Phenylgruppe oder eine Pyridinylgruppe darstellt,

➭ Z eine Gruppe bedeutet ausgewählt aus Wasserstoffatomen, Halogenatomen, geradkettigen oder verzweigten ($C_1$-$C_6$)-Alkylgruppen, Nitrogruppen, Cyanogruppen, Hydroxygruppen, geradkettigen oder verzweigten ($C_1$-$C_6$)-Alkoxygruppen, Arylgruppen, geradkettigen oder verzweigten Aryl-($C_1$-$C_6$)-alkylgruppen, Aryloxygruppen, geradkettigen oder verzweigten Aryl-($C_1$-$C_6$)-alkoxygruppen und Gruppen $NR_5R_6$, worin $R_5$ und $R_6$, die gleichartig oder verschieden sind, unabhängig voneinander jeweils eine Gruppe bedeuten ausgewählt aus dem Wasserstoffatom und geradkettigen oder verzweigten ($C_{1\text{-}}C_6$)-Alkylgruppen,

✧ $R_4$ eine Gruppe bedeutet ausgewählt aus dem Wasserstoffatom, geradkettigen oder verzweigten ($C_1$-$C_6$)-Alkylgruppen, Arylgruppen, geradkettigen oder verzweigten Aryl-($C_1$-$C_6$)-alkylgruppen oder Gruppen -C(O)-OR'$_5$, worin R'$_5$ eine Gruppe darstellt ausgewählt aus geradkettigen oder verzweigten ($C_1$-$C_6$)-Alkylgruppen, Arylgruppen oder geradkettigen oder verzweigten Aryl-($C_1$-$C_6$)-alkylgruppen,

• Y eine Gruppe bedeutet ausgewählt aus dem Sauerstoffatom oder der Methylengruppe,
• $R_2$ ein Wasserstoffatom bedeutet, wobei in diesem Fall:
$R_3$ eine Gruppe bedeutet ausgewählt aus dem Wasserstoffatom, geradkettigen oder verzweigten ($C_1$-$C_6$)-Alkylgruppen, geradkettigen oder verzweigten Aryl-($C_{1\text{-}}C_6$)-alkylgruppen oder $SO_2CF_3$, Arylgruppen,
• oder $R_2$ und $R_3$ gemeinsam eine Bindung bilden,
• $R_1$ eine Gruppe bedeutet ausgewählt aus dem Wasserstoffatom, geradkettigen oder verzweigten ($C_1$-$C_6$)-Alkylgruppen, Arylgruppen, geradkettigen oder verzweigten Aryl-($C_1$-$C_6$)-alkylgruppen oder geradkettigen oder verzweigten ($C_1$-$C_6$)-Alkylenketten, die durch eine oder mehrere gleichartige oder verschiedenartige Gruppen substituiert sind, ausgewählt aus -OR"$_5$, -NR"$_5$R"$_6$, worin R"$_5$ und R"$_6$ die gleichen Bedeutungen besitzen, wie sie oben für $R_5$ und $R_6$ definiert worden sind,
• $\mathbf{Z_1}$ und $\mathbf{Z_2}$ jeweils ein Wasserstoffatom bedeuten oder
$\mathbf{Z_1}$ und $\mathbf{Z_2}$ gemeinsam mit den sie tragenden Kohlenstoffatomen eine Phenylgruppe bilden,

mit der Maßgabe, daß, wenn Z ein Wasserstoffatom darstellt, $R_1$ von dem Wasserstoffatom verschieden ist,
deren Enantiomere, Diastereoisomere und N-Oxide sowie deren Additionssalze mit einer pharmazeutisch annehmbaren Säure oder Base,
wobei man unter einer Arylgruppe eine Phenyl-, Naphthyl-, Dihydronaphthyl-, Tetrahydronaphthyl-, Indenyl- oder Indanyl-gruppe versteht, wobei jede dieser Gruppen gegebenenfalls durch eine oder mehrere gleichartige oder verschiedenartige Gruppen substituiert ist ausgewählt aus Halogen, geradkettigem oder verzweigtem ($C_1$-$C_6$)-Alkyl, geradkettigem oder verzweigtem ($C_1$-$C_6$)-Trihalogenalkyl, Hydroxy, geradkettigem oder verzweigtem ($C_1$-$C_6$)-Alkoxy und gegebenenfalls durch eine oder zwei geradkettige oder verzweigte ($C_1$-$C_6$)-Alkylgruppen substituiertem Amino.

2. Verbindungen der Formel (I) nach Anspruch 1, **dadurch gekennzeichnet, daß** sie der Formel (IA) entsprechen:

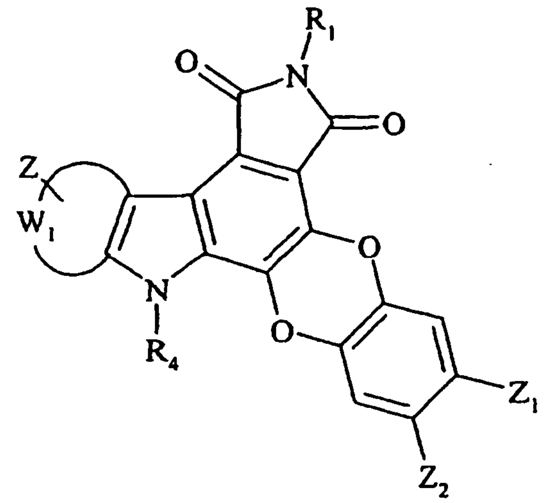

(IA)

in der $R_1$, $R_4$, $W_1$, Z, $Z_1$ und $Z_2$ die bezüglich der Formel (I) angegebenen Bedeutungen besitzen, deren Enantiomere, Diastereoisomere und N-Oxide sowie deren Additionssalze mit einer pharmazeutisch annehmbaren Säure oder Base.

3. Verbindungen der Formel (I) nach einem der Ansprüche 1 oder 2, **dadurch gekennzeichnet, daß** sie der Formel (IB) entsprechen:

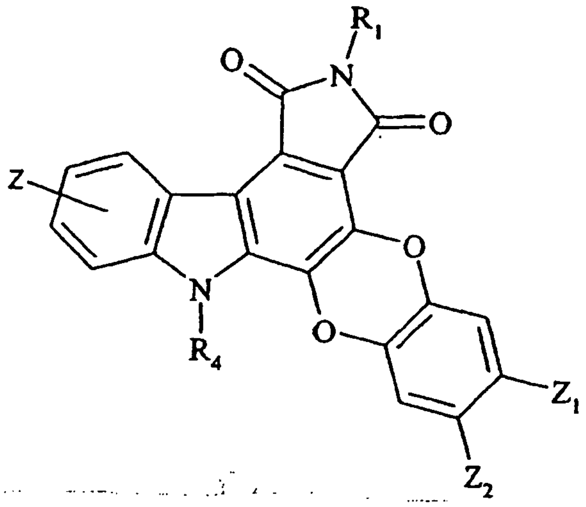

(IB)

in der $R_1$, $R_4$, Z, $Z_1$ und $Z_2$ die bezüglich der Formel (I) angegebenen Bedeutungen besitzen, deren Enantiomere, Diastereoisomere, N-Oxide sowie deren Additionssalze mit einer pharmazeutisch annehmbaren Säure oder Base.

4. Verbindungen der Formel (I) nach einem der Ansprüche 1 oder 2, **dadurch gekennzeichnet, daß** sie der Formel (IC) entsprechen:

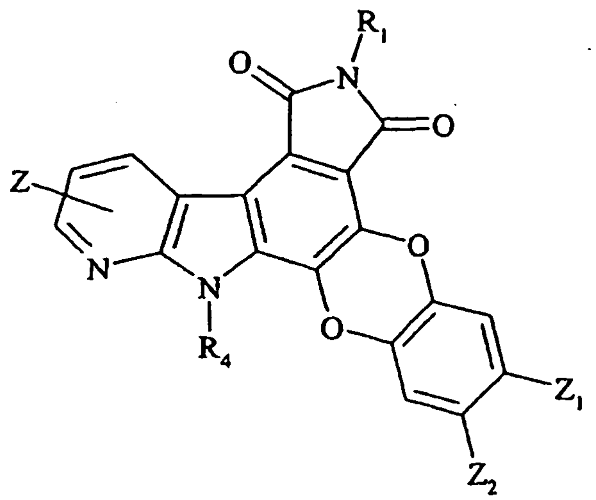

(IC)

in der $R_1$, $R_4$, Z, $Z_1$ und $Z_2$ die bezüglich der Formel (I) angegebenen Bedeutungen besitzen, deren Enantiomere, Diastereoisomere, N-Oxide sowie deren Additionssalze mit einer pharmazeutisch annehmbaren Säure oder Base.

**5.** Verbindungen der Formel (I) nach Anspruch 1, **dadurch gekennzeichnet, daß** sie der Formel (ID) entsprechen:

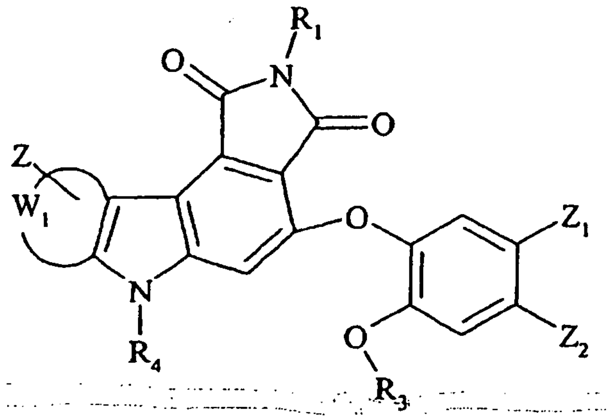

(ID)

in der $R_1$, $R_3$, $R_4$, $W_1$, Z, $Z_1$ und $Z_2$ die bezüglich der Formel (I) angegebenen Bedeutungen besitzen, deren Enantiomere, Diastereoisomere, N-Oxide sowie deren Additionssalze mit einer pharmazeutisch annehmbaren Säure oder Base.

**6.** Verbindungen der Formel (I) nach einem der Ansprüche 1 oder 5, **dadurch gekennzeichnet, daß** sie der Formel (IE) entsprechen:

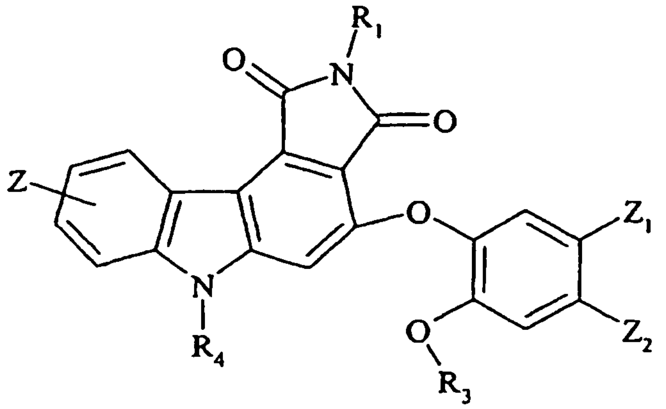

(IE)

in der $R_1$, $R_3$, $R_4$, Z, $Z_1$ und $Z_2$ die bezüglich der Formel (I) angegebenen Bedeutungen besitzen, deren Enantiomere, Diastereoisomere, N-Oxide sowie deren Additionssalze mit einer pharmazeutisch annehmbaren Säure oder Base.

**7.** Verbindungen der Formel (I) nach einem der Ansprüche 1 oder 5, **dadurch gekennzeichnet, daß** sie der Formel (IF) entsprechen:

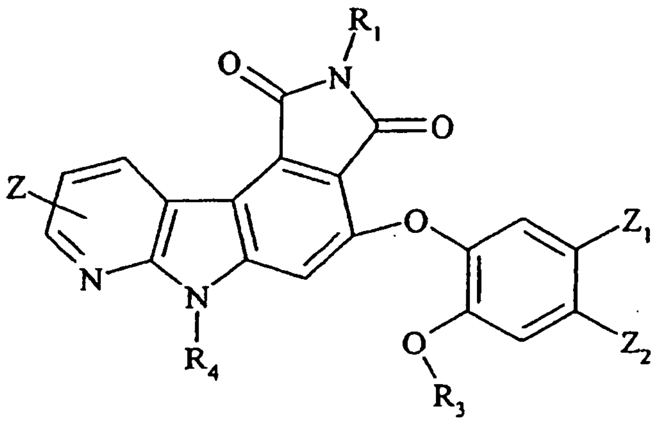

(IF)

in der $R_1$, $R_3$, $R_4$, Z, $Z_1$ und $Z_2$ die bezüglich der Formel (I) angegebenen Bedeutungen besitzen, deren Enantiomere, Diastereoisomere, N-Oxide sowie deren Additionssalze mit einer pharmazeutisch annehmbaren Säure oder Base.

**8.** Verbindungen der Formel (I) nach einem der Ansprüche 1 oder 5, **dadurch gekennzeichnet, daß** sie der Formel (IG) entsprechen:

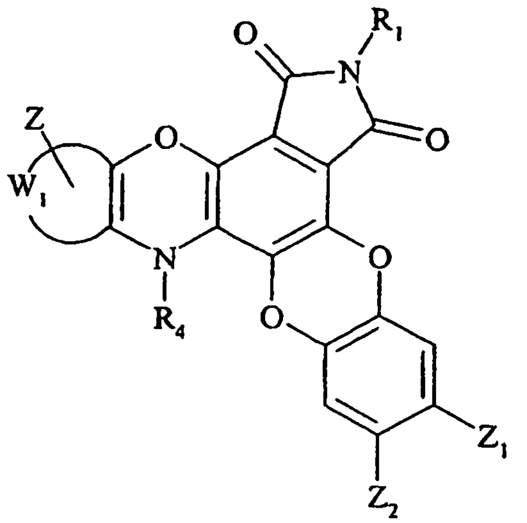

(IG)

in der $R_1$, $R_4$, $W_1$, Z, $Z_1$ und $Z_2$ die bezüglich der Formel (I) angegebenen Bedeutungen besitzen, deren Enantiomere, Diastereoisomere, N-Oxide sowie deren Additionssalze mit einer pharmazeutisch annehmbaren Säure oder Base.

**9.** Verbindungen der Formel (I) nach einem der Ansprüche 1 oder 8, **dadurch gekennzeichnet, daß** sie der Formel (IH) entsprechen:

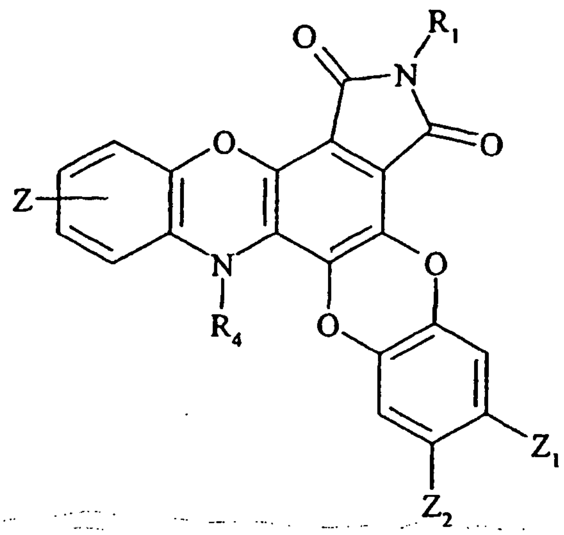

(IH)

in der $R_1$, $R_4$, Z, $Z_1$ und $Z_2$ die bezüglich der Formel (I) angegebenen Bedeutungen besitzen, deren Enantiomere, Diastereoisomere. N-Oxide sowie deren Additionssalze mit einer pharmazeutisch annehmbaren Säure oder Base.

**10.** Verbindungen der Formel (I) nach einem der Ansprüche 1 oder 8, **dadurch gekennzeichnet, daß** sie der Formel (IJ) entsprechen:

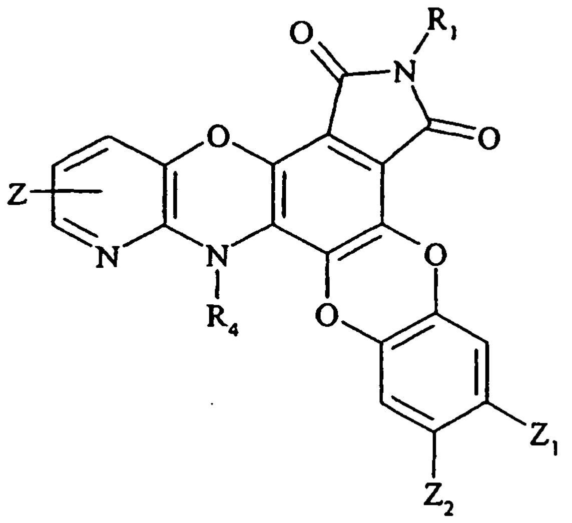

(IJ)

in der $R_1$, $R_4$, Z, $Z_1$ und $Z_2$ die bezüglich der Formel (I) angegebenen Bedeutungen besitzen, deren Enantiomere, Diastereoisomere, N-Oxide sowie deren Additionssalze mit einer pharmazeutisch annehmbaren Säure oder Base.

**11.** Verbindungen der Formel (I) nach einem der Ansprüche 1 bis 10, **dadurch gekennzeichnet, daß** Z ein Wasserstoffatom, ein Halogenatom oder eine Hydroxygruppe bedeutet, deren Enantiomere, Diastereoisomere, N-Oxide sowie deren Additionssalze mit einer pharmazeutisch annehmbaren Säure oder Base.

**12.** Verbindungen der Formel (I) nach einem der Ansprüche 1 bis 11, **dadurch gekennzeichnet, daß** A zusammen mit den Kohlenstoffatomen, an die es gebunden ist, Gruppen der Formeln bedeutet:

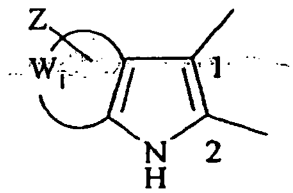

oder

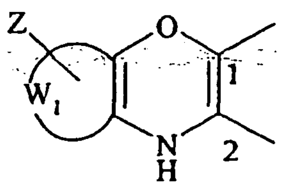

deren Enantiomere, Diastereoisomere, N-Oxide sowie deren Additionssalze mit einer pharmazeutisch annehmbaren Säure oder Base.

**13.** Verbindungen der Formel (I) nach einem der Ansprüche 1 bis 12, **dadurch gekennzeichnet, daß** $R_3$ ein Wasserstoffatom oder eine geradkettige oder verzweigte ($C_1$-$C_6$)-Alkylgruppe bedeutet, deren Enantiomere, Diastereoisomere, N-Oxide sowie deren Additionssalze mit einer pharmazeutisch annehmbaren Säure oder Base.

**14.** Verbindungen der Formel (I) nach einem der Ansprüche 1 bis 13, **dadurch gekennzeichnet, daß** $R_1$ ein Wasserstoffatom, eine geradkettige oder verzweigte ($C_1$.$C_6$)-Alkylgruppe oder eine geradkettige oder verzweigte ($C_1$-$C_6$)-Alkylenkette, die durch eine oder mehrere gleichartige oder verschiedenartige Gruppen substituiert ist, ausgewählt aus -$NR_5R_6$, worin $R_5$ und $R_6$ die bezüglich der Formel (I) angegebenen Bedeutungen besitzen, deren Enantiomere, Diastereoisomere, N-Oxide sowie deren Additionssalze mit einer pharmazeutisch annehmbaren Säure oder Base.

**15.** Verbindungen der Formel (I) nach einem der Ansprüche 1 bis 14, **dadurch gekennzeichnet, daß** $Z_1$ und $Z_2$ Wasserstoffatome bedeuten, deren Enantiomere, Diastereoisomere, N-Oxide sowie deren Additionssalze mit einer pharmazeutisch annehmbaren Säure oder Base.

**16.** Verbindungen der Formel (I) nach Anspruch 1, nämlich:

- N-Methyl[1,4]benzodioxino[2,3-a]pyrrolo[3,4-c]carbazol-6,8-dion,
- 10-Fluor-7-methyl[1 ,4]benzodioxino[2,3-a]pyrrolo[3,4-c]carbazol-6,8-dion,
- 11-Fluor-7-methyl[1,4]benzodioxino[2,3-a]pyrrolo[3,4-c]carbazol-6,8-dion,
- 7-[2-(Dimethylamino)-ethyl]-10-fluor[1,4]benzodioxino[2,3-a]pyrrolo[3,4-c]carbazol-6,8-dion,
- 10-Hydroxy[1,4]benzodioxino[2,3-a]pyrrolo[3,4-c]carbazol-6,8-dion.
- 11-Hydroxy[1,4]benzodioxino[2,3-a]pyrrolo[3,4-c]carbazol-6,8-dion,
- 7-[2-(Dimethylamino)-ethyl[1,4]benzodioxino[2,3-a]pyrrolo[3,4-c]carbazol-6,8-dion,
- 7-[2-(Dimethylamino)-ethyl]-10-hydroxy[1,4]benzodioxino[2,3-a]pyrrolo[3,4-c]-carbazol-6, 8-dion,
- 7-[2-(Dimethylamino)-ethyl]-11hydroxy[1,4]benzodioxino[2,3-a]pyrrolo[3,4-c]-carbazol-6,8-dion,
- 7-[2-(Dimethylamino)-ethyl][1,4]benzodioxino[2,3-e]pyrido[2',3':5.6][1,4]oxazino-[3,2-g]isoindol-6,8-dion,

deren Enantiomere, Diastereoisomere, N-Oxide sowie deren Additionssalze mit einer pharmazeutisch annehmbaren Säure oder Base.

**17.** Verfahren zur Herstellung der Verbindungen der Formel (I) nach Anspruch 1, **dadurch gekennzeichnet, daß** man als Ausgangsprodukt eine Verbindung der Formel (II) verwendet:

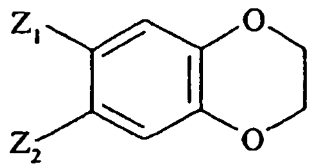

(II)

in der $Z_1$ und $Z_2$ die bezüglich der Formel (I) angegebenen Bedeutungen besitzen, welche Verbindung der Formel (II) man mit N-Bromsuccinimid in Gegenwart von Benzoylperoxid umsetzt zur Bildung der Verbindung der Formel (III):

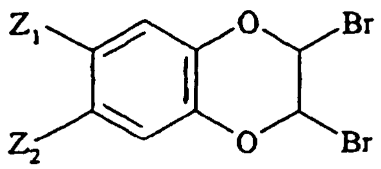

(III)

in der $Z_1$ und $Z_2$ die oben angegebenen Bedeutungen besitzen, welche Verbindung der Formel (III) mit Natriumiodid umgesetzt wird zur Bildung der Verbindung der Formel (IV):

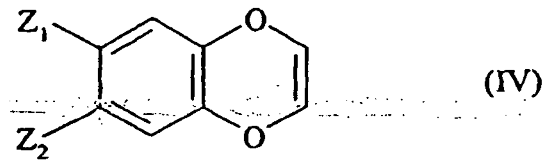

(IV)

in der $Z_1$ und $Z_2$ die oben angegebenen Bedeutungen besitzen, welche Verbindung der Formel (IV) mit n-Butyllithium und dann Trimethylzinnchlorid umgesetzt wird zur Bildung der Verbindung der Formel (V):

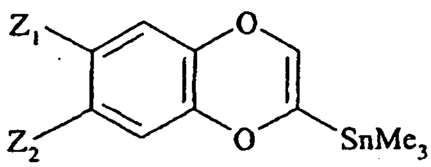

(V)

in der $Z_1$ und $Z_2$ die oben angegebenen Bedeutungen besitzen, welche Verbindung der Formel (V):

• entweder in Gegenwart von Tetrakis(triphenylphosphin)palladium(0) mit einer Verbindung der Formel (VI):

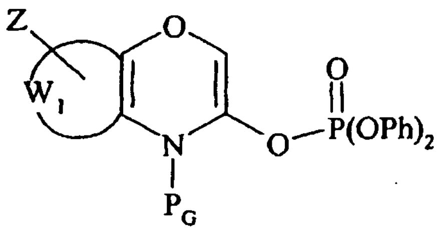

(VI)

in der $P_G$ eine in der organischen Synthese gut bekannte Schutzgruppe für Amine darstellt und $W_1$ und Z die bezüglich der Formel (I) angegebenen Bedeutungen besitzen, behandelt wird, zur Bildung der Verbindung der Formel (VII):

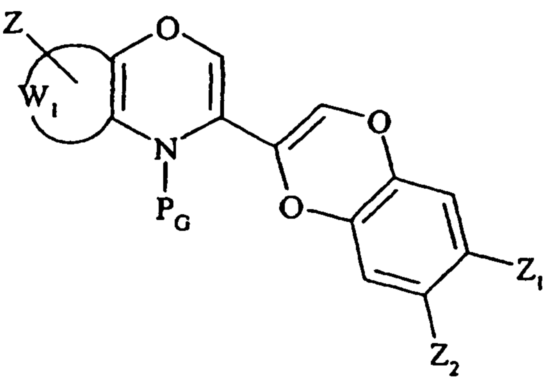

(VII)

in der $P_G$, Z, $Z_1$, $Z_2$ und $W_1$ die oben angegebenen Bedeutungen besitzen, welche Verbindung der Formel (VII) mit einer Verbindung der Formel (VIII) behandelt wird:

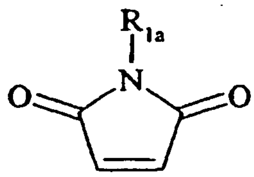

(VIII)

in der $R_{1a}$ ein Wasserstoffatom oder eine Methylgruppe bedeutet, zur Bildung der Verbindung der Formel (IX):

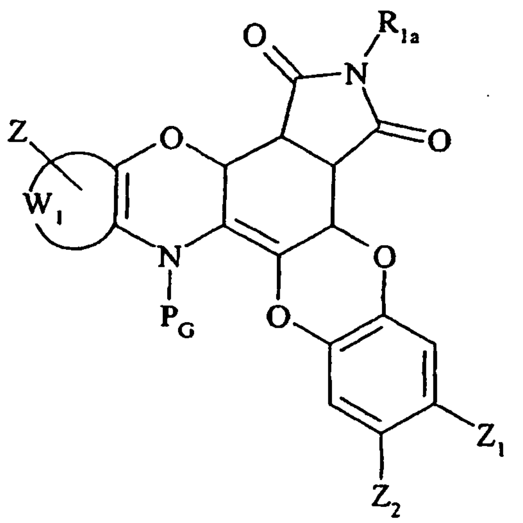

(IX)

in der $P_G$, $R_{1a}$, Z, $Z_1$, $Z_2$ und $W_1$ die oben angegebenen Bedeutungen besitzen, welche Verbindung der Formel (IX) in Gegenwart von N-Bromsuccinimid und Benzoylperoxid umgesetzt wird zur Bildung der Verbindung der Formel (I/a), einem Sonderfall der Verbindungen der Formel (I):

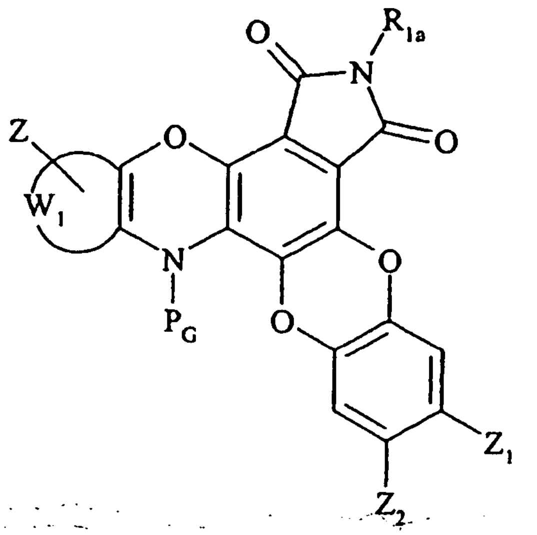

(I/a)

in der $P_G$, $R_{1a}$, Z, $Z_1$, $Z_2$ und $W_1$ die oben angegebenen Bedeutungen besitzen, welche Verbindung der Formel (I/a) gegebenenfalls mit einer Verbindung der Formel (X):

$R_{1b}$-$NH_2$ (X)

in der $R_{1b}$ von Wasserstoff und der Methylgruppe verschieden ist und die gleichen Bedeutungen besitzt wie $R_1$ bezüglich der Formel (I), behandelt wird zur Bildung der Verbindung der Formel (I/b), einem Sonderfall der Verbindungen der Formel (I):

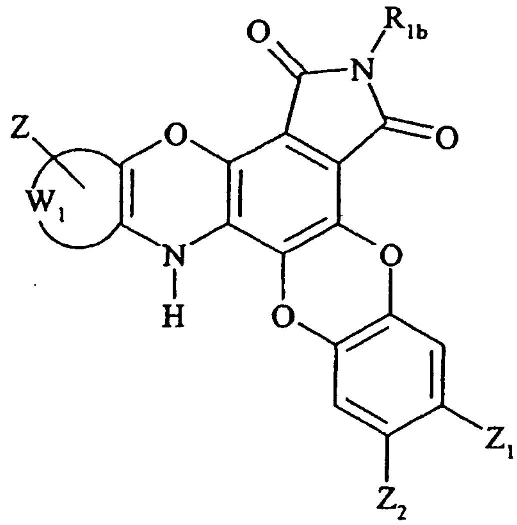

(I/b)

in der $R_{1b}$, Z, $Z_1$, $Z_2$ und $W_1$ die oben angegebenen Bedeutungen besitzen, welche Verbindungen der Formeln (I/a) und (I/b), die die Verbindungen der Formel (I/c) bilden:

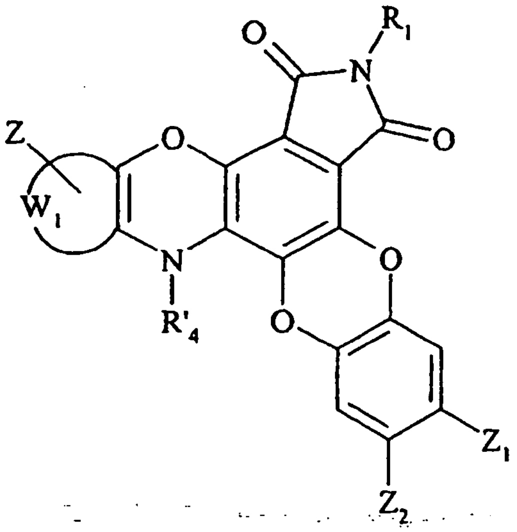

(I/c)

in der $R'_4$ ein Wasserstoffatom oder eine Gruppe $P_G$ bedeutet und $R_1$, Z, $Z_1$, $Z_2$ und W, die oben angegebenen Bedeutungen besitzen,

• oder in Gegenwart von Bistriphenylphosphinpalladium(II)-chlorid mit einer Verbindung der Formel (XI):

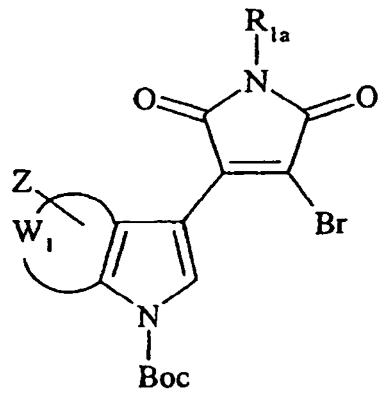

(XI)

in der Boc eine tert.-Butoxycarbonylgruppe darstellt und $R_{1a}$, $W_1$ und Z die oben angegebenen Bedeutungen besitzen, behandelt wird zur Bildung der Verbindung der Formel (XII):

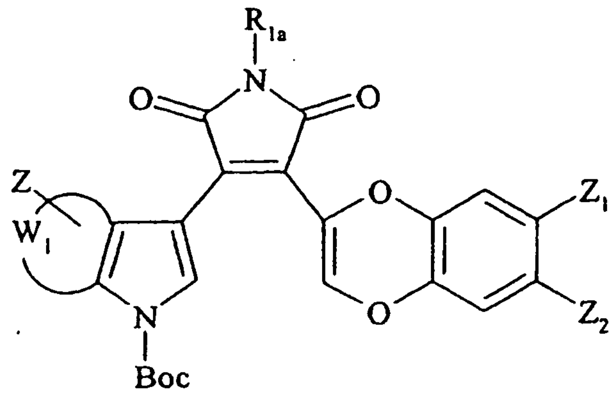

(XII)

in der Boc, $R_{1a}$, Z, $Z_1$, $Z_2$ und $W_1$ die oben angegebenen Bedeutungen besitzen, welche Verbindung der Formel (XII):

- ♦ entweder mit einer UV-Lampe in Gegenwart von Iod in einem apolaren und aprotischen Lösungsmittel bestrahlt wird zur Bildung der Verbindungen der Formeln (I/d) und (I/e), einem Sonderfall der Verbindungen der Formel (I):

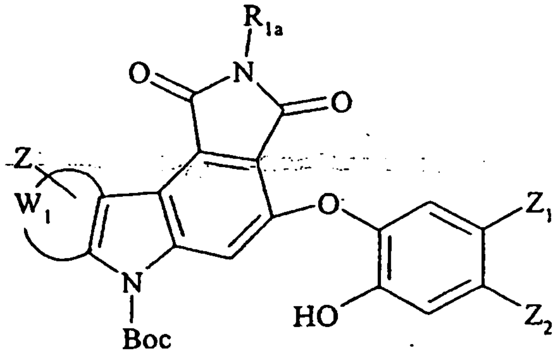

(I/d)

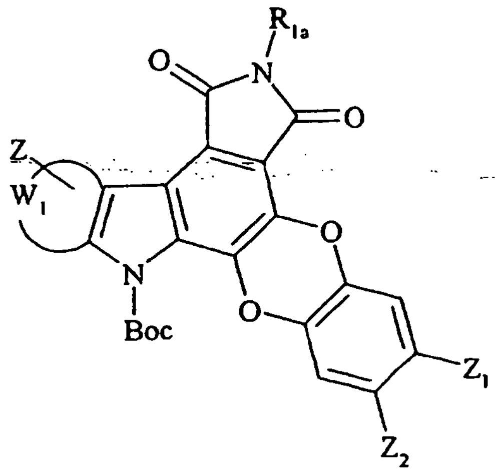

(I/e)

worin Boc, $R_{1a}$, Z, $Z_1$, $Z_2$ und $W_1$ die oben angegebenen Bedeutungen besitzen, von welchen Verbindungen der Formel (I/d) man:

✧ gegebenenfalls die Schutzgruppe der Aminfunktion mit Hilfe klassischer Methoden der organischen Synthese abspaltet zur Bildung der Verbindung der Formel (I/f), einem Sonderfall der Verbindungen der Formel (I):

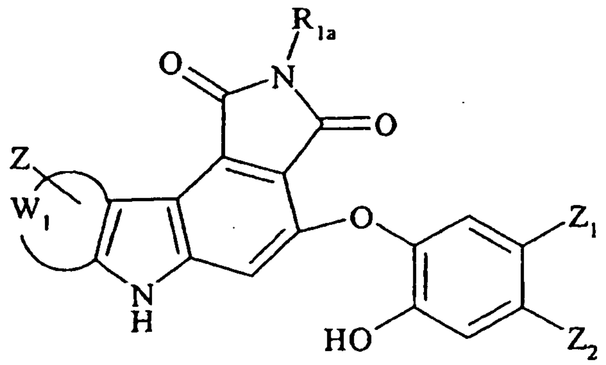

(I/f)

in der $R_{1a}$, Z, $Z_1$, $Z_2$ und $W_1$ die oben angegebenen Bedeutungen besitzen,

✧ oder gegebenenfalls der Einwirkung einer Verbindung der Formel (XIII):

$R_{3a}$-G (XIII)

in der $R_{3a}$, welches von einem Wasserstoffatom verschieden ist, die gleichen Bedeutungen besitzt wie $R_3$ bezüglich der Formel (I) und G die oben angegebenen Bedeutungen besitzt, unterwirft zur Bildung der Verbindung der Formel (I/g), einem Sonderfall der Verbindungen der Formel (I):

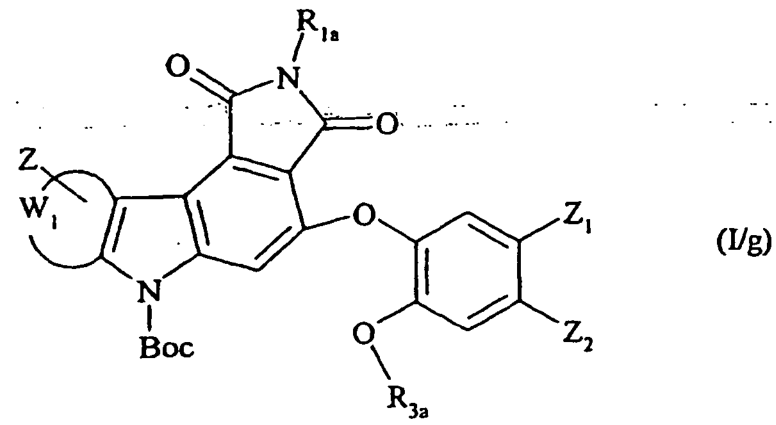

(I/g)

in der Boc, $R_{1a}$, $R_{3a}$, Z, $Z_1$, $Z_2$ und $W_1$ die oben angegebenen Bedeutungen besitzen, welche Verbindungen der Formeln (I/d), (I/e) und (I/g) die Verbindungen der Formel (I/h) bilden:

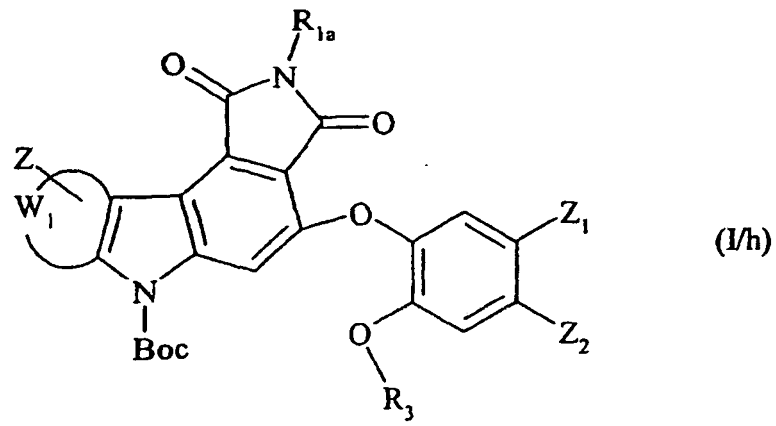

(I/h)

in der Boc, $R_{1a}$, $R_3$, $Z_1$, $Z_2$ und $W_2$ die oben angegebenen Bedeutungen besitzen, welche Verbindungen der Formel (I/h) gegebenenfalls den gleichen Reaktionsbedingungen wie die Verbindungen der Formel (I/a) unterworfen werden zur Bildung der Verbindungen der Formel (I/i), einem Sonderfall der Verbindungen der Formel (I):

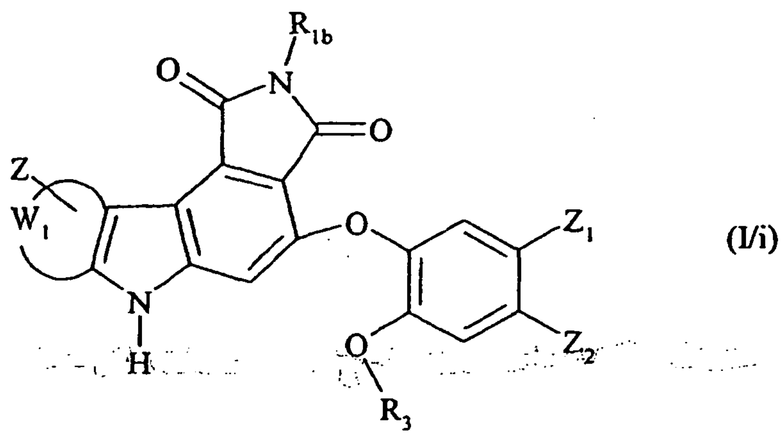

(I/i)

in der $R_{1b}$, $R_3$, Z, $Z_1$, $Z_2$ und $W_1$ die oben angegebenen Bedeutungen besitzen,

♦ oder der Einwirkung von Chlorwasserstoffsäure unterzogen werden zur Bildung der Verbindungen der Formel (XIV):

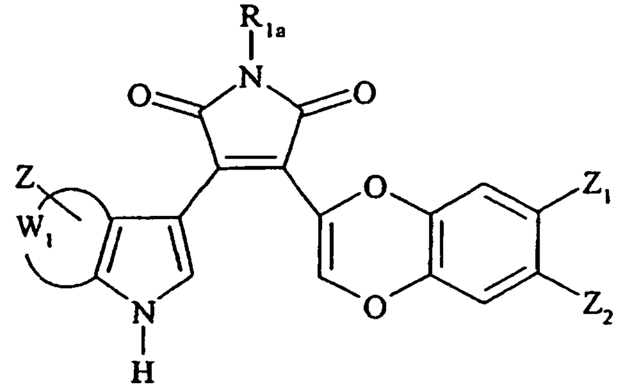

(XIV)

in der $R_{1a}$, Z, $Z_1$, $Z_2$ und $W_1$ die oben angegebenen Bedeutungen besitzen, welche Verbindung der Formel (XIV) der Einwirkung einer Verbindung der Formel (XV):

$R_{4a}$-G (XV)

in der G eine austretende Gruppe darstellt und $R_{4a}$, welches von einem Wasserstoffatom verschieden ist, die gleichen Bedeutungen besitzt wie $R_4$ bezüglich der Formel (I), unterworfen wird zur Bildung der Verbindung der Formel (XVI):

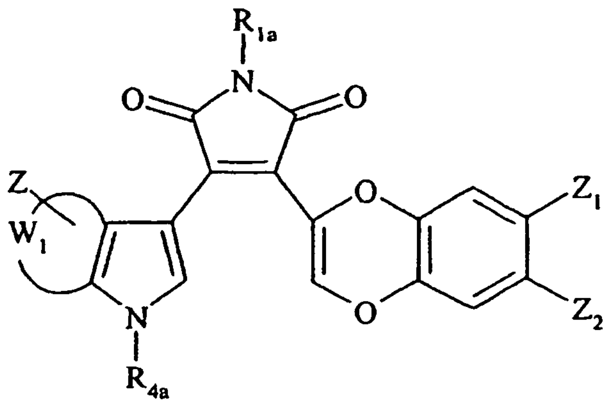

(XVI)

in der $R_{1a}$, $R_{4a}$, Z, $Z_1$, $Z_2$ und $W_1$ die oben angegebenen Bedeutungen besitzen, welche Verbindung der Formel (XVI) den gleichen Reaktionsbedingungen unterworfen wird wie die Verbindung der Formel (XII) zur Bildung der Verbindungen der Formeln (I/j) und (I/k), einem Sonderfall der Verbindungen der Formel (I):

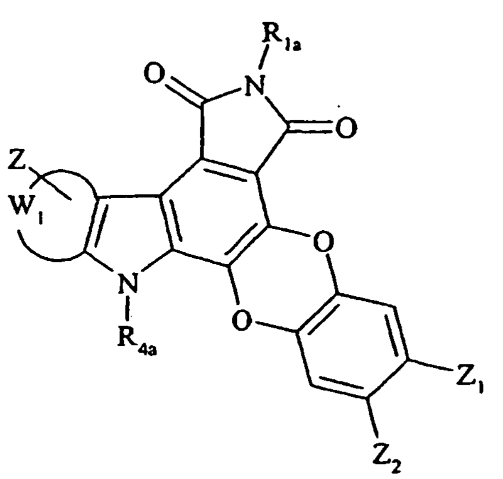

(I/j)

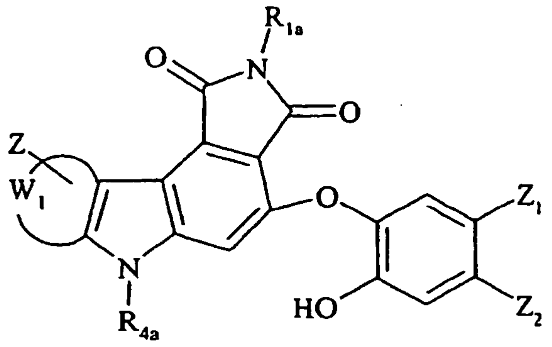

(I/k)

in denen $R_{1a}$, $R_{4a}$, Z, $Z_1$, $Z_2$ und $W_1$ die oben angegebenen Bedeutungen besitzen, welche Verbindung der Formel (I/k) gegebenenfalls der Einwirkung einer Verbindung der Formel (XIII), wie sie oben definiert worden ist, unterworfen wird zur Bildung der Verbindung der Formel (I/1):

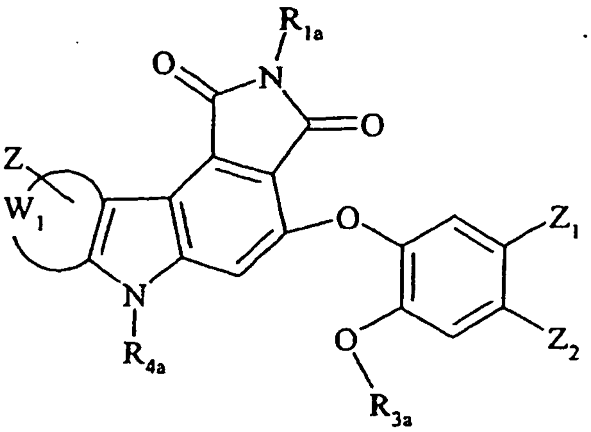

(I/l)

in der $R_{1a}$, $R_{3a}$, $R_{4a}$, Z, $Z_1$, $Z_2$ und $W_1$ die oben angegebenen Bedeutungen besitzen,
welche Verbindungen der Formeln (I/j), (I/k) und (I/l) die Verbindungen der Formel (I/m) bilden:

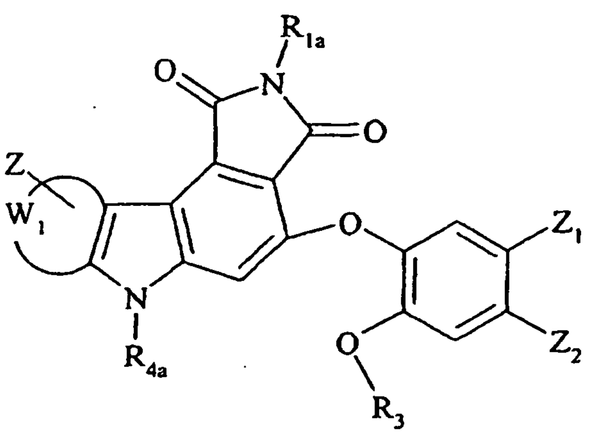

(I/m)

in der $R_{1a}$, $R_3$, $R_{4a}$, Z, $Z_1$, $Z_2$ und $W_1$ die oben angegebenen Bedeutungen besitzen, welche Verbindung der Formel (I/m) gegebenenfalls den gleichen Reaktionsbedingungen unterworfen wird wie die Verbindung der Formel (I/h) zur Bildung der Verbindung der Formel (I/n):

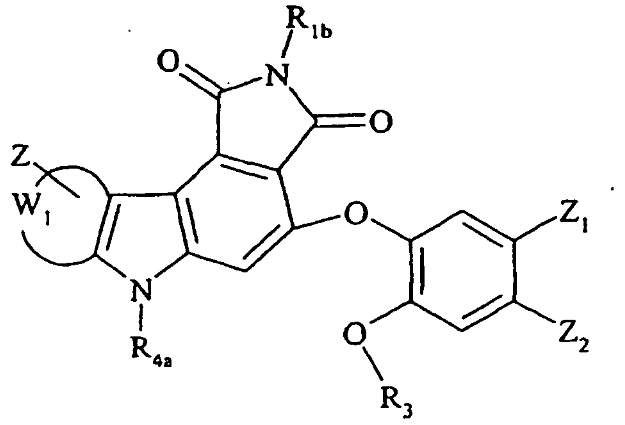

(I/n)

in der $R_{1b}$, $R_3$, $R_4$a, Z, $Z_1$, $Z_2$ und $W_1$ die oben angegebenen Bedeutungen besitzen, welche Verbindungen der Formeln (I/e), (I/h). (I/i), (I/m) und (I/n) die Verbindungen der Formel (I/o) bilden:

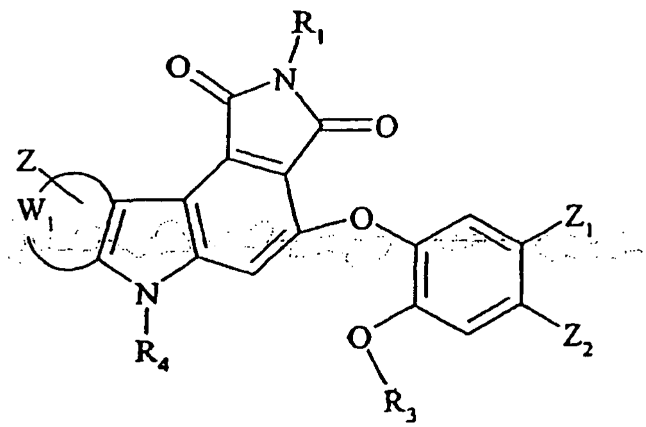

(I/o)

in der $R_1$, $R_3$, $R_4$, Z, $Z_1$, $Z_2$ und $W_1$ die oben angegebenen Bedeutungen besitzen, welche Verbindungen der Formeln (I/a) bis (I/o), welche die Gesamtheit der Verbindungen der Formel (I) bilden, man gegebenenfalls mit Hilfe klassischer Reinigungsmethoden reinigt, gewünschtenfalls mit Hilfe einer klassischen Trennmethode in ihre verschiedenen Isomeren auftrennt und gewünschtenfalls in ihre N-Oxide umwandelt und gegebenenfalls in ihre Additionssalze mit einer pharmazeutisch annehmbaren Säure oder Base umwandelt.

**18.** Pharmazeutische Zubereitungen, enthaltend als Wirkstoff mindestens eine Verbindung der Formel (I) nach einem der Ansprüche 1 bis 16 allein oder in Kombination mit einem oder mehreren inerten, nichttoxischen, pharmazeutisch annehmbaren Trägermaterialien oder Hilfsstoffen.

**19.** Pharmazeutische Zubereitungen nach Anspruch 18, nützlich als Arzneimittel bei der Behandlung von Krebs.